# EUROPEAN PATENT APPLICATION

(11) **EP 4 497 828 A1**
(43) Date of publication of application: **29.01.2025**
(21) Application number: 23382794.8
(22) Date of filing: 28.07.2023
(51) Int. Cl.: C12N 15/82

(54) **MINIMAL MIRNA PRECURSORS**

(71) Applicant: CONSEJO SUPERIOR DE INVESTIGACIONES CIENTIFICAS (CSIC), 28006 Madrid (ES); Universitat Politècnica de València, 46022 Valencia (ES)
(72) Inventor: CARBONELL OLIVARES, Alberto Tomás, 46022 VALÈNCIA (ES); DARÓS ARNAU, José Antonio, 46022 VALÈNCIA (ES); GONZÁLEZ CISNEROS, Adriana Estela, 46022 VALÈNCIA (ES); MARTÍN GARCÍA, Tamara, 46022 VALÈNCIA (ES)
(74) Representative: ZBM Patents - Zea, Barlocci & Markvardsen

(57) **Abstract**

The invention provides a viral vector comprising a heterologous nucleotide sequence that encodes, or corresponds to the RNA sequence S1 that is of a length of 56-400 ribonucleotides and that comprises regions R1, R2, R3, R4 and R5 directly linked to each other as indicated in formula (i):

(i) 5'-R1-R2-R3-R4-R5-3',

wherein:
- R1 consists of the RNA sequence of a strand of a basal stem (BS) structure,
- R2 consists of the RNA sequence of a miRNA,
- R3 comprises an RNA sequence that has a length of at least 8 nucleotides,
- R4 consists of an RNA sequence that base pairs with the RNA sequence of R2 to form a miRNA/miRNA* duplex,
- R5 consists of the RNA sequence complementary to R1 in the BS comprising R1,
and wherein the RNA sequence S1 forms a stem-loop structure where the stem comprises regions R1, R2, R4 and R5 wherein region R1 base pairs with region R5 and region R2 base pairs with region R4, and the loop comprises a sequence of at least 4 contiguous nucleotides of region R3. The invention also provides a virus encoded by or comprising the viral vector, a plasmid comprising or encoding the viral vector, a bacterium comprising the plasmid, a plant comprising the viral vector, the virus or the bacterium, a plant extract from the plant, and a method for obtaining the plant.

## Description

### Technical Field

The present invention relates to the field of biotechnology. More particularly it relates to minimal miRNA precursors that are suitable to be expressed from viral vectors, and in plants in a transgene-free manner.

### Background Art

MiRNAs are regulatory RNAs that are expressed in a wide range of eukaryotes that affect the translation or stability of target mRNAs. Mature miRNAs are single stranded RNAs of 20-24 nucleotides (nt) in length that base-pair with their target mRNA to negatively regulate its expression.

The expression of miRNAs in plants is used as a biotechnological tool for both functional genomics studies and for crop improvements. Indeed, the downregulation of a specific gene's expression in plants has been shown to be effective for controlling insects and other pests, pathogens, and abiotic stresses like drought, salinity, and extreme temperatures.

Artificial miRNAs (amiRNAs) are miRNAs designed for redirecting the endogenous miRNA biogenesis and silencing machineries of plants so that the expression of specific target RNAs is suppressed with high efficacy and specificity (with no predicted off-targets). However, a current limitation of the amiRNA technology is the relatively long length of the precursors that allow the production of the amiRNAs *in vivo.* Said precursors are mostly based on full-length primary miRNA (pri-miRNA) sequences. Reducing the size of amiRNA precursors should have several biotechnological advantages such as a more cost-effective production of amiRNA constructs or a more efficient *in vitro* or in bacteria synthesis of amiRNA precursors for topical application to plants. Another limitation of the current techniques is the need to genetically modify plant genomes with amiRNA transgenes. The use of plant DNA viruses to successfully express authentic amiRNAs in *Nicotiana benthamiana* (Tang,Y., et al. (2010), Virus-based microRNA expression for gene functional analysis in plants. Plant Physiol, 153, 632-41) is promising; however, agroinfiltration of plant tissues with plasmids including the amiRNA-expressing viral vector implies the modification of the plant's genome, and it is limited to a narrow list of host species.

Therefore, there is a need in the field of new strategies for the efficient expression of amiRNAs in plants, and especially in a transgene-free and cost-effective manner.

### Summary of Invention

The present inventors have developed chimeric minimal precursors of any miRNA of interest, including artificial miRNAs (amiRNAs). Said minimal precursors comprise the sequences that are sufficient and necessary for the expression of the corresponding mature miRNA in plants.

As shown in the examples below, the minimal miRNA precursors developed by the inventors provide various advantages over other known miRNA precursors, such as the full-length primary miRNA precursor of *Arabidopsis thaliana MIR390a (pri-AtMIR390a).* For example, amiRNA production and silencing of the corresponding target gene can be achieved to the same extent with the minimal precursors developed by the inventors, as with the full length *pri-AtMIR390a* comprising said amiRNA. However, the length of the minimal precursors is almost 6 times lower than that of the *pri-AtMIR390a.* Moreover, the minimal precursors developed can be efficiently expressed from a viral vector, contrary to the long *pri-AMIR390a,* which appears to be eliminated from the viral vector during replication. Importantly, gene silencing can also be achieved by mechanical inoculation with a crude extract from a plant comprising the viral vector from which the minimal precursors are expressed. Thus, the minimal precursors of the invention permit obtaining plants with a gene knock-down that are, however, non-transgenic. Additionally, since the miRNA comprised in the minimal precursors can be artificially designed against any sequence of interest, such as against an endogenous nucleic acid sequence of a plant or a sequence in the genome of a plant pathogen, non-transgenic plants resistant to a specific pathogen or with an improved agricultural trait can be produced, by means of the minimal miRNA precursors of the invention.

All these unique characteristics make the minimal miRNA precursors of the invention important molecular tools for any application that involves gene silencing in plants, especially for obtaining non-transgenic plants, such as non-transgenic plants resistant to a particular plant pathogen or with an improved agricultural trait.

Thus, in a first aspect, the invention provides a viral vector comprising a heterologous nucleotide sequence that encodes, or corresponds to the RNA sequence S1 that is of a length of 56--400 ribonucleotides and that comprises regions R1, R2, R3, R4 and R5 directly linked to each other as indicated in formula (i):

(I) 5'-R1-R2-R3-R4-R5-3',

wherein:
- R1 consists of the RNA sequence of a strand of a basal stem (BS) structure,
- R2 consists of the RNA sequence of a miRNA,
- R3 comprises an RNA sequence that has a length of at least 8 nucleotides,
- R4 consists of an RNA sequence that base pairs with the RNA sequence of R2 to form a miRNA/miRNA* duplex,
- R5 consists of the RNA sequence complementary to R1 in the BS comprising R1,
and wherein the RNA sequence S1 forms a stem-loop structure where the stem comprises regions R1, R2, R4 and R5 wherein region R1 base pairs with region R5 and region R2 base pairs with region R4, and the loop comprises a sequence of at least 4 contiguous nucleotides of region R3.

In a second aspect, the invention provides a viral vector comprising a sequence that is the reverse complement of the sequence of the viral vector as defined in the first aspect of the invention.

In a third aspect, the invention provides a virus encoded by or comprising the viral vector according to the first or the second aspect of the invention.

In a fourth aspect, the invention provides an RNA polynucleotide comprising the RNA sequence S1 as defined in the first aspect, provided that the RNA polynucleotide does not comprise:
(i) in the region upstream of region R1, a sequence comprised in SEQ ID NO. 25 of at least 10 nucleotides, and/or
(ii) in the region downstream of region R5, a sequence comprised in SEQ ID NO. 26 of at least 10 nucleotides.

In a fifth aspect, the invention provides a DNA polynucleotide encoding the polynucleotide as defined in the fourth aspect of the invention.

In a sixth aspect, the invention provides a plasmid comprising a sequence that encodes or corresponds to the sequence of the viral vector as defined in the first or second aspect of the invention, or the sequence encoding the polynucleotide as defined in the fourth aspect of the invention.

In a seventh aspect, the invention provides a bacterium that comprises the plasmid as defined in the sixth aspect of the invention.

In an eighth aspect, the invention provides a plant that comprises the viral vector as defined in the first or second aspect of the invention, the virus as defined in the third aspect of the invention, the polynucleotide as defined in the fourth or fifth aspect, the plasmid as defined in the sixth aspect and/or the bacterium as defined in the seventh aspect.

In a ninth aspect, the invention provides a plant extract from the plant as defined in the eighth aspect.

In a tenth aspect, the invention provides a method for obtaining the plant as defined in the eighth aspect, the method comprising the step of:
(i) transfecting a plant with the plasmid as defined in the sixth aspect, particularly by agroinfiltration with the bacterium as defined in the seventh aspect, or alternatively,
(ii) contacting the surface of at least one organ of a plant with a with the virus as defined in the third aspect.

In an eleventh aspect, the invention provides a method for obtaining the plant extract according to the ninth aspect that comprises the steps of:
(i) Grinding to powder at least one organ or a section thereof of the plant as defined in the eighth aspect, and
(ii) Homogenizing the powder obtained in step (i) in a buffer.

In a twelfth aspect, the invention provides a kit of parts comprising the viral vector as defined in the first or second aspect, the virus as defined in the third aspect, the polynucleotide as defined the fourth or fifth aspect, and/or the plasmid as defined in the sixth aspect.

In a thirteenth aspect, the invention provides a device comprising the virus as defined in the third aspect, the bacterium as defined in the seventh aspect, or the plant extract as defined in the ninth aspect.

In a fourteenth aspect, the invention provides a method for obtaining the viral vector as defined in the first or second aspect that comprises:
i) Transfecting an organ of a plant with the plasmid as defined in sixth aspect, particularly by agroinfiltration with the bacterium as defined in the seventh aspect,
ii) Allowing the plant to grow for at least 1 week, and
iii) Isolating from the plant obtained from step (ii) the viral vector comprised in the organ transfected in step (i),.

In a fifteenth aspect, the invention provides a method for obtaining the virus as defined in the third aspect that comprises:
i) Transfecting an organ of a plant with the plasmid as defined in sixth aspect, particularly by agroinfiltration with the bacterium as defined in the seventh aspect,
ii) Allowing the plant to grow for at least 1 week, and
iii) Isolating from the plant obtained from step (ii) the viral vector comprised in the organ transfected in step (i).

In a sixteenth aspect, the invention provides a method for protecting a plant from one or more plant pathogens, the method comprising:
(i) transfecting a plant with the plasmid as defined in the sixth aspect, particularly by agroinfiltration with the bacterium as defined in the seventh aspect, or alternatively,
(ii) contacting the surface of at least one organ of a plant with a the virus as defined in the third aspect.

In a seventeenth aspect, the invention provides a method for the treatment of a plant pathogen disease in a plant, the method comprising:
(i) transfecting a plant with the plasmid as defined in the sixth aspect, particularly by agroinfiltration with the bacterium as defined in the seventh aspect, or alternatively,
(ii) contacting the surface of at least one organ of a plant with a the virus of the third aspect of the invention.

In an eighteenth aspect, the invention provides a method for improving a trait of a plant, the method comprising:
(i) transfecting a plant with the plasmid as defined in the sixth aspect, particularly by agroinfiltration with the bacterium as defined in the seventh aspect, or alternatively,
(ii) contacting the surface of at least one organ of a plant with the virus of the third aspect of the invention.

### Brief description of the drawings

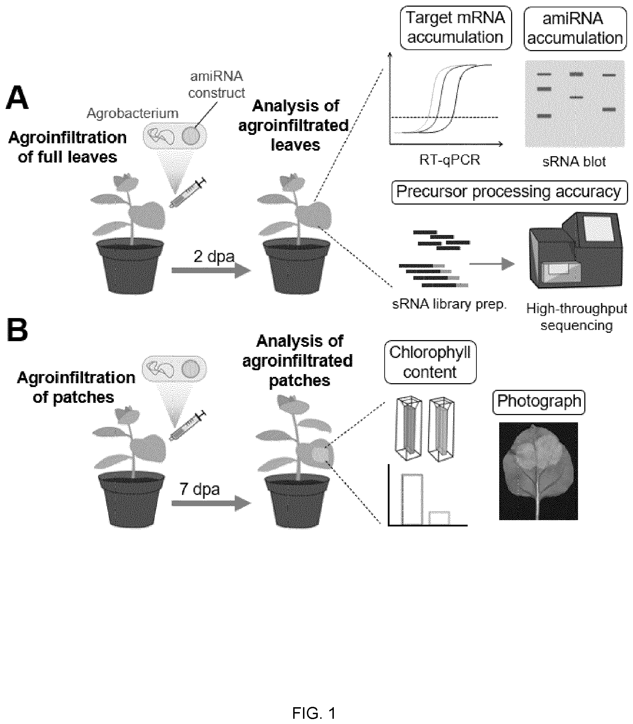

**Figure 1****.** Methodology for easily visualizing and quantifying the silencing activity of amiRNAs derived from AtMIR390a-based precursors. A and B, Steps for the analysis of fully agroinfiltrated leaves or patches, respectively.

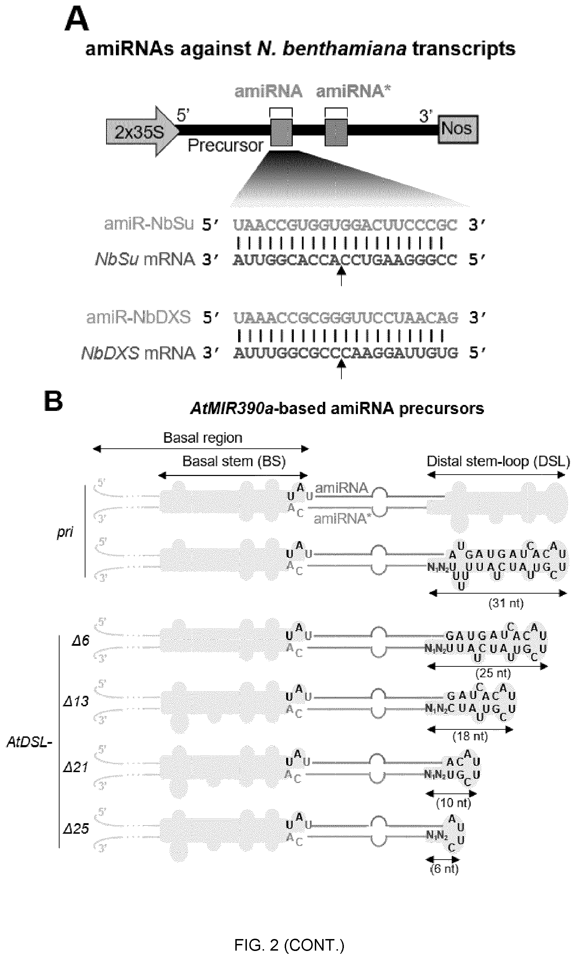

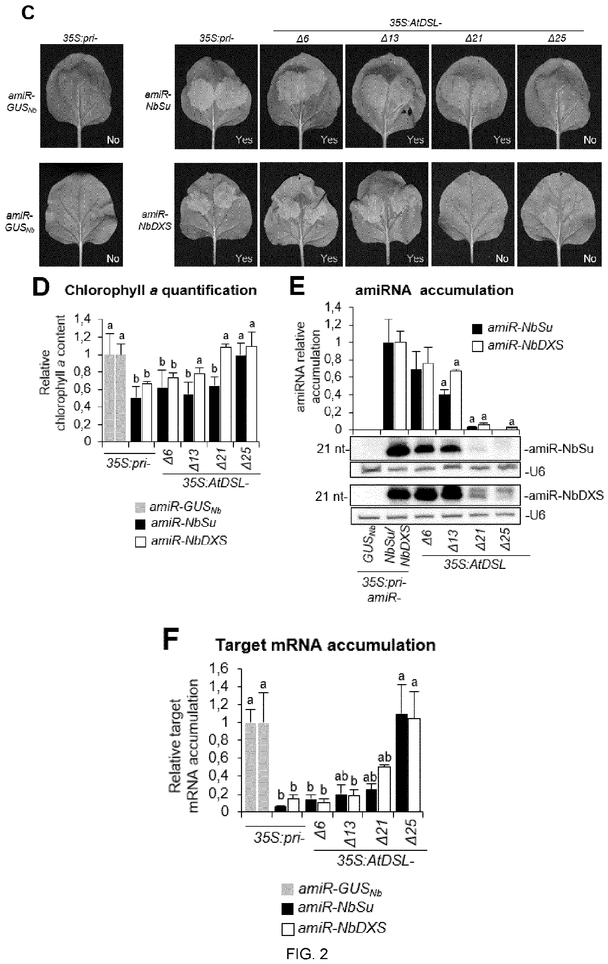

**Figure 2****.** Functional analysis of constructs expressing amiR-NbSu or amiR-NbDXS against *N. benthamiana SULPHUR (NbSu)* or *DXS (NbDXS),* respectively, from *AtMIR390a*-based precursors with shortened distal stem-loop (DSL) region. A, Diagram of *AtMIR390a*-based amiRNA constructs including the base-pairing of amiRNAs and target mRNAs. Nucleotides corresponding to the guide strand of the amiRNA against *NbSu* and *NbDXS* are depicted in the upper strand ("amiR-NbSu" and "amiR-NbDXS"), while nucleotides of target mRNAs are depicted in the lower strand ("NbSu mRNA" and "NbDXS mRNA"). The arrows indicate the amiRNA-predicted cleavage site. B, Diagrams of the amiRNA precursors with nucleotides corresponding to the amiRNA guide and amiRNA* strands are in grey, while those from *AtMIR390a* are in black. The two nucleotides specific to each amiRNA construct that are modified for preserving authentic *pri* foldback secondary structure are depicted as "N₁N₂". The shapes corresponding to *pri* basal stem (BS) or DSL regions are also highlighted with a ligth grey background. The size in nucleotides of the DSL region of each precursor is given. C, Photos at 7 days post-agroinfiltration (dpa) of leaves agroinfiltrated with different constructs. "Yes" and "No" labels indicate the presence or absence of bleached patches, respectively. D, Bar graph showing the relative content of chlorophyll a in patches agroinfiltrated with different constructs (*pri-amiR-GUS_{Nb} =* 1.0). Bars with the letter "b" or "a" are significantly different from that of the corresponding control samples *pri-amiR-GUS_{Nb}* or *pri-amiR-NbSulpri-amiR-NbDXS,* respectively (P < 0.05 in pairwise Student's t-test comparisons). E, Northern blot detection of amiR-NbSu and amiR-NbDXS in RNA preparations from agroinfiltrated leaves at 2 dpa. The graph at top shows the mean (n = 3) + standard deviation amiRNA relative accumulation (*pri-amiR-NbSu =* 1.0; *pri-amiR-NbDXS* = 1.0). Bars with a letter "a" are significantly different from that of sample *pri-amiR-NbSu* or *pri-amiR-NbDXS.* One blot from three biological replicates is shown. F, Target mRNA accumulation in agroinfiltrated leaves. Mean relative level (n = 3) + standard error of *NbSu* or *NbDXS* mRNAs after normalization to *PROTEIN PHOSPHATASE 2A* (*PP2A*)*,* as determined by quantitative RT-PCR (qPCR) (*pri-amiR-GUS_{Nb}* = 1.0 in all comparisons). Other details are as in D.

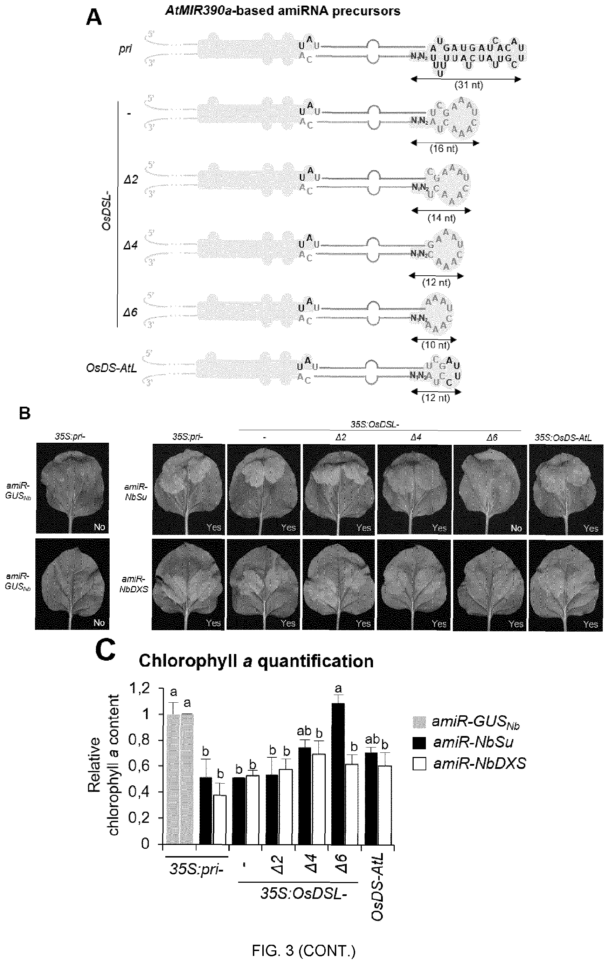

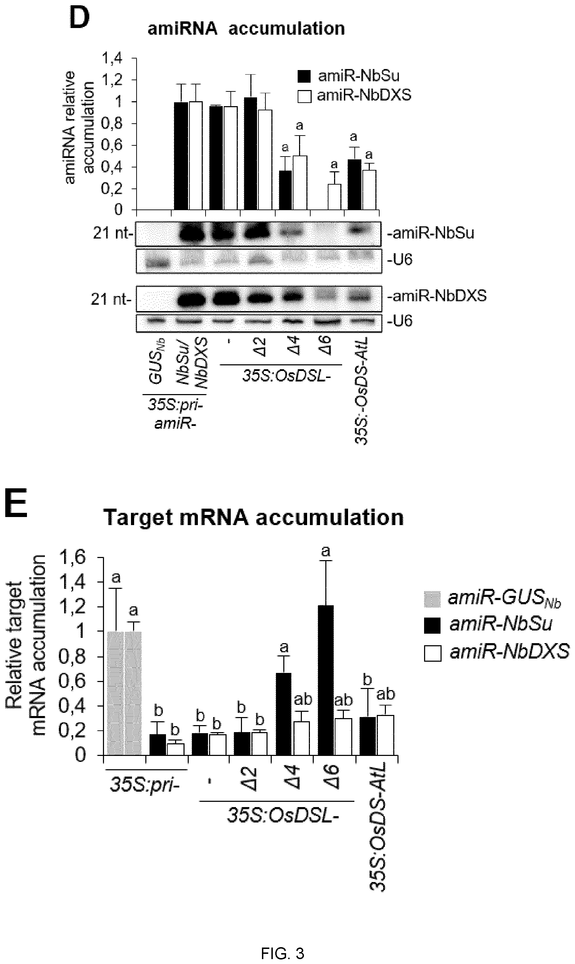

**Figure 3****.** Functional analysis of constructs expressing amiR-NbSu or amiR-NbDXS against *N. benthamiana SULPHUR (NbSu)* or *DXS (NbDXS)* from chimeric precursors including intact *AtMIR390a* basal region and shortened *OsMIR390* distal stem-loop (DSL) region. A, Diagrams of the amiRNA precursors. Nucleotides from *OsMIR390* are in grey with a light grey background. The shapes corresponding to *OsMIR390a* distal stem-loop regions are highlighted with a light grey background. Other details are as in Figure 2B. B, Photos at 7 days post-agroinfiltration (dpa) of leaves agroinfiltrated with different constructs. C, Bar graph showing the relative content of chlorophyll *a* in patches agroinfiltrated with different constructs (*pri-amiR-GUS_{Nb} =* 1.0). Other details are as in Figure 2D. D, Northern blot detection of amiR-NbSu and amiR-NbDXS amiRNAs in RNA preparations from agroinfiltrated leaves at 2 dpa. Other details are as in Figures 2D and 2E. E, Target mRNA accumulation in agroinfiltrated leaves. Other details are as in Figures 2D and 2F.

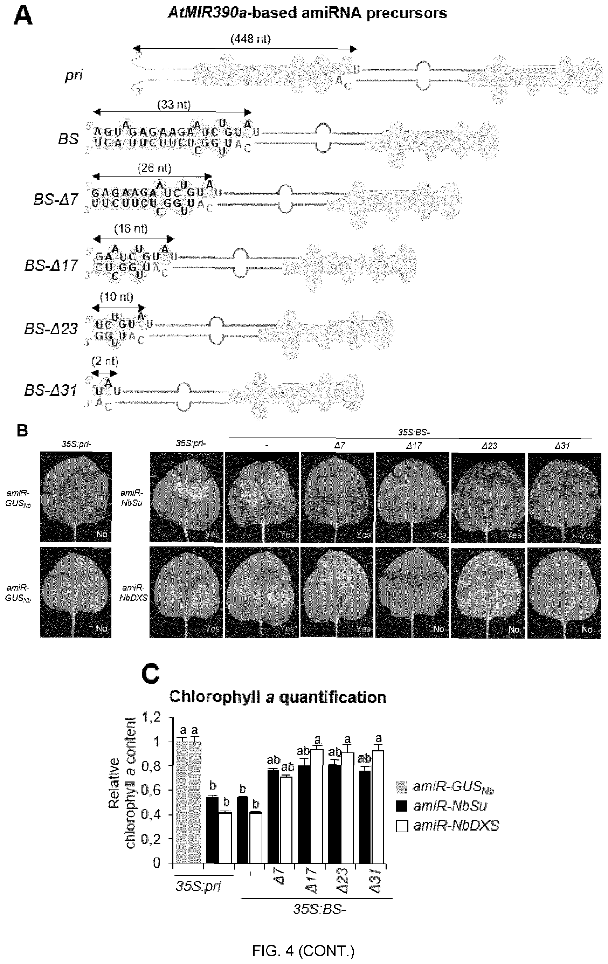

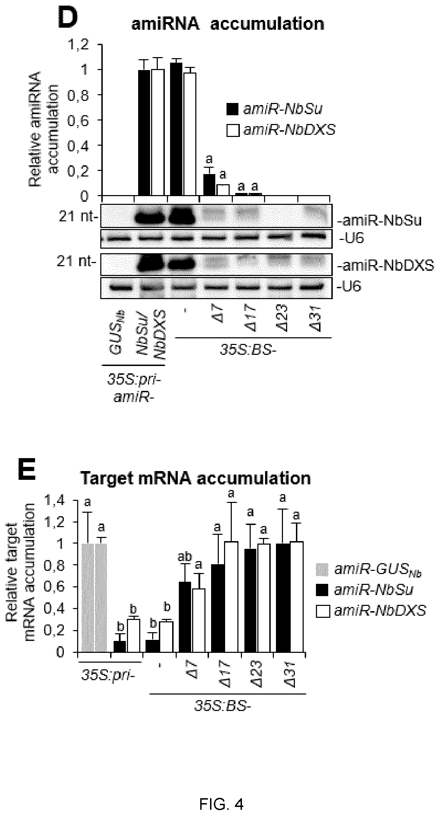

**Figure 4****.** Functional analysis of constructs expressing the amiR-NbSu amiRNA against *N. benthamiana SULPHUR (NbSu)* or *DXS (NbDXS)* from AtMIR390a precursors with shortened basal stem (BS) region. A, Diagrams of the amiRNA precursors. The size in nucleotides of the BS region of each precursor is given. Other details are as in Figure 2B. B, Photos at 7 days post-agroinfiltration (dpa) of leaves agroinfiltrated with different constructs. C, Bar graph showing the relative content of chlorophyll a in patches agroinfiltrated with different constructs (*pri-amiR-GUS_{Nb} =* 1.0). Other details are as in Figure 2D. D, Northern blot detection of amiR-NbSu and amiR-NbDXS amiRNAs in RNA preparations from agroinfiltrated leaves at 2 dpa. Other details are as in Figures 2D and 2E. E, Target mRNA accumulation in agroinfiltrated leaves. Other details are as in Figures 2D and 2F.

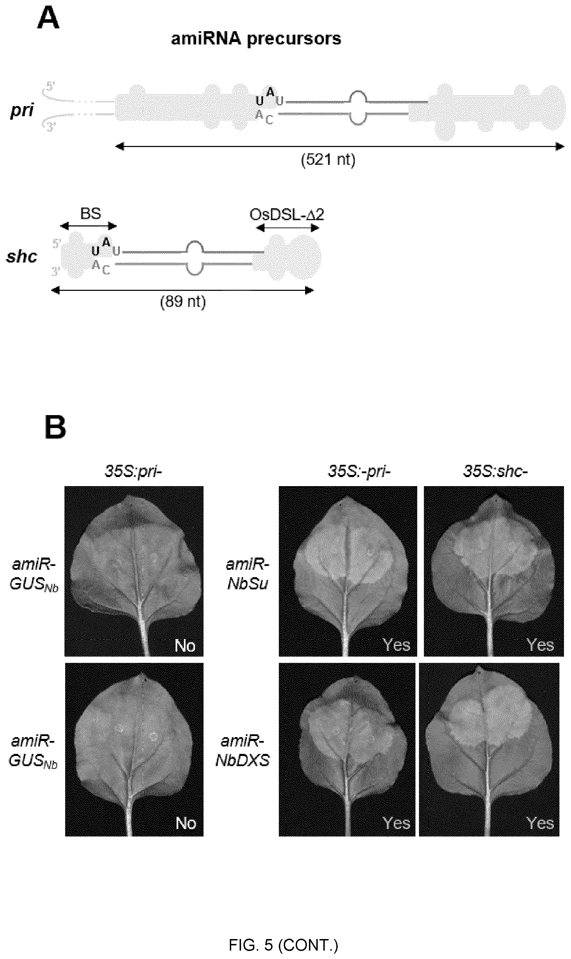

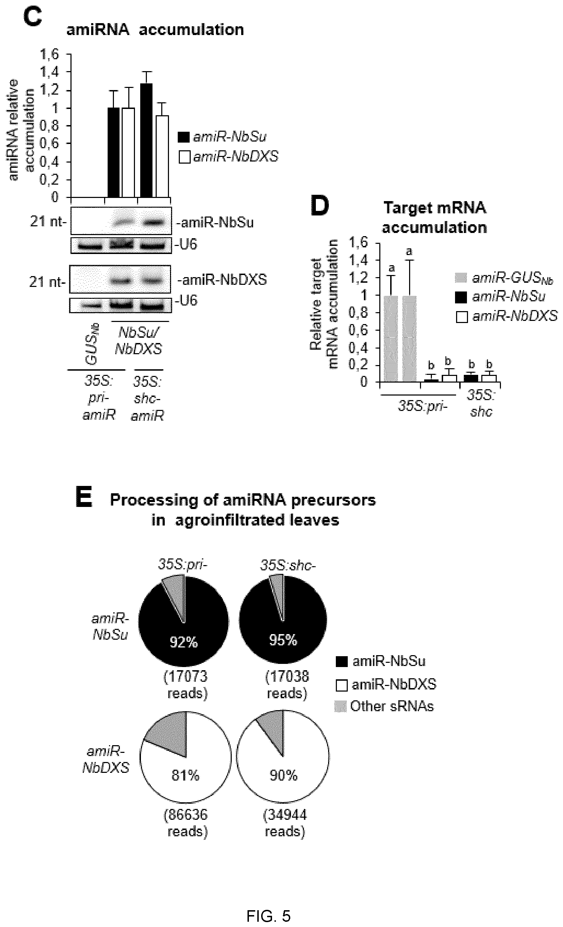

**Figure 5****.** Functional analysis of constructs expressing the amiR-NbSu amiRNA against *N. benthamiana SULPHUR (NbSu)* or *DXS (NbDXS)* from *pri* and shc precursors. A, Diagrams of the *pri* and *shc* amiRNA precursors. The total length of each precursor is indicated. Other details are as in Figure 2B. B, Photos at 7 days post-agroinfiltration (dpa) of leaves agroinfiltrated with the two constructs. C, Northern blot detection of amiR-NbSu and amiR-NbDXS amiRNAs in RNA preparations from agroinfiltrated leaves at 2 dpa. Other details are as in Figures 2D and 2E. D, Target mRNA accumulation in agroinfiltrated leaves. Other details are as in Figures 2D and 2F. E, amiRNA processing from *AtMIR390a*-based precursors. Pie charts show percentages of reads corresponding to expected, accurately processed 21-nucleotide mature amiR-NbSu and amiR-NbDXS or to other 19-24-nucleotide sRNAs.

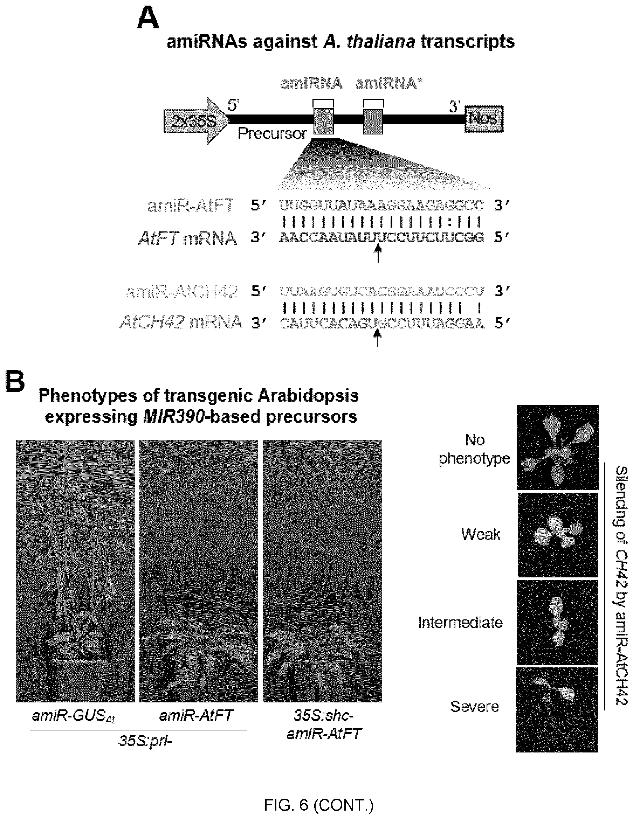

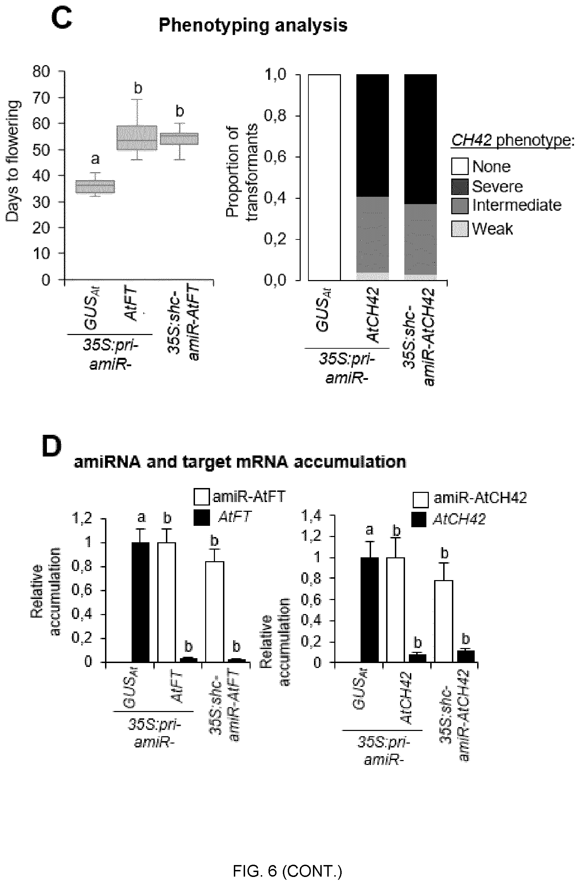

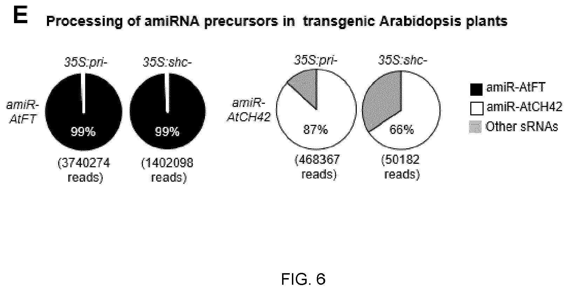

**Figure 6****.** Functional analysis of constructs expressing the amiR-AtFT and amiR-AtCH42 amiRNAs against A. *thaliana FLOWERING LOCUS T* (*AtFT*) or *CHLORINE 42* (*AtCH42*) from *pri* and *shc* precursors. A, Nucleotides corresponding to the guide strand of the amiRNA against *AtFT* and *AtCH42* are are depicted in the upper strand ("amiR-AtFT" and "amiR-AtCh42") while nucleotides of *AtFT* and *AtCH42* target mRNAs are depicted in the lower strand ("AtFT mRNA" and "AtCH42 mRNA"). Other details are as in Figure 2A.B, Representative images of Arabidopsis T1 transgenic plants expressing amiRNAs from different precursors. Left, 45-day-old adult plants expressing amiR-GUS_{At} or amiR-AtFT. Right, 10-day-old T1 seedlings expressing amiR-AtCH42 and showing bleaching phenotypes of diverse degrees. C, Phenotyping analysis. Left, box plot representing the mean flowering time of Arabidopsis T1 transgenic plants expressing amiR-GUS_{At} or amiR-AtFT from different precursors. Pairwise Student's t-test comparisons are represented with the letter 'a' if significantly different (P < 0.05) and the letter 'b' if not (P > 0.05). Right, bar graph representing, for each line, the proportion of seedlings displaying a severe (black areas), intermediate (dark gray areas), or weak (light gray areas) bleaching phenotype, or with wild-type appearance (white areas). D, amiRNA and target RNA accumulation. Left, bar graph showing the relative accumulation of amiR-AtFT [mean relative level (n = 3) + standard deviation amiRNA relative accumulation, *pri-amiR-AtFT* = 1.0] and of *AtFT* mRNA [mean relative level (n = 3) + standard error of *AtFT mRNAs* after normalization to *ACTIN 2* (*AtACT2*)*,* as determined by quantitative RT-qPCR, *pri-amiR-GUS_{At} =* 1]. Right, bar graph showing the relative accumulation of amiR-AtCH42 [mean relative level (n = 3) + standard deviation amiRNA relative accumulation, *pri-amiR-AtCH42* = 1.0] and of *AtCH42* mRNA [mean relative level (n = 3) + standard error of *AtCH42* mRNAs after normalization to *ACTIN 2* (*AtACT2*)*,* as determined by quantitative RT-qPCR, *pri-amiR-GUS_{At} =* 1]. E, amiRNA processing from *pri* or shc precursors. Pie charts show percentages of reads corresponding to expected, accurately processed 21-nucleotide mature amiR-AtFT and amiR-AtCH42 or to other 19-24-nucleotide sRNAs.

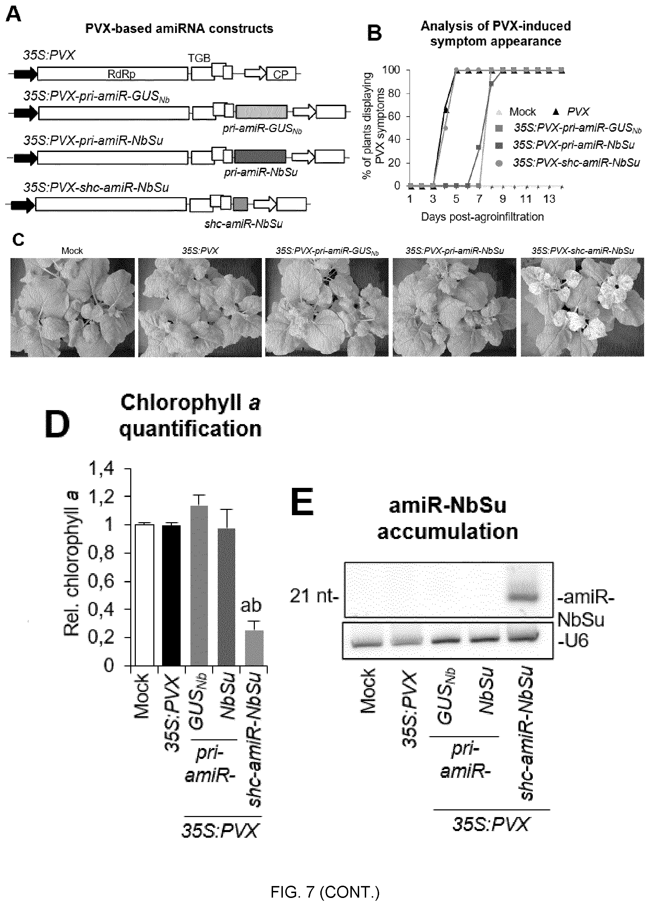

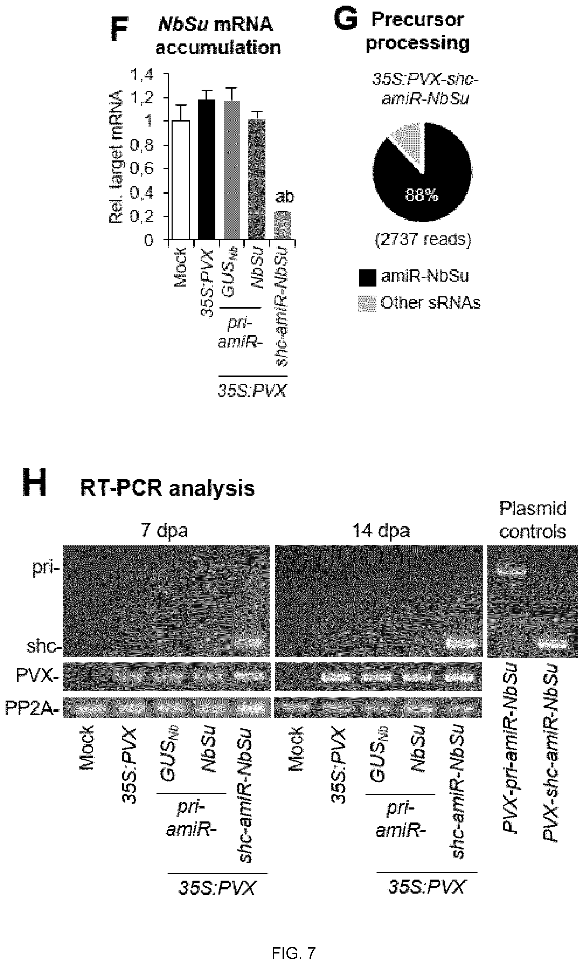

**Figure** 7. Functional analysis of *Potato virus* X (PVX) constructs expressing amiRNAs from *pri* and *shc* precursors. A, Diagram of PVX-based constructs. *pri-amiR-GUS_{Nb}, pri-amiR-NbSu* and *shc-amiR-NbSu* cassettes are shown in grey boxes with different greys. PVX genes RdRp, TGB and CP are represented in white boxes, and CP promoter from *Bamboo mosaic virus* (BaMV) with a black arrow. B, Two-dimensional line graph showing, for each of the three-plant sets listed, the percentage of symptomatic plants per day during 14 days. C, Photos at 14 days post-agroinfiltration (dpa) of sets of three plants agroinfiltrated with the different constructs. D, Bar graph showing the relative content of chlorophyll a in apical leaves from plants agroinfiltrated with different constructs (Mock = 1.0). Bar with the letter "a" are significantly different from that of the corresponding mock control samples (P < 0.05 in pairwise Student's t-test comparison). E, Northern blot detection of amiR-NbSu in RNA preparations from apical leaves collected at 14 dpa and pooled from three independent plants. F, Target *NbSu* mRNA accumulation in RNA preparations from apical leaves collected at 14 dpa and analyzed individually. Mean relative level (n = 3) + standard error of *NbSu* mRNAs after normalization to *PROTEIN PHOSPHATASE 2A* (*PP2A*)*,* as determined by RT-qPCR (mock = 1.0 in all comparisons). Other details are as in D. G, amiR-NbSu processing from the shc precursor included in PVX *(35S:PVX-shc-amiR-NbSu* construct). Pie chart show percentages of reads corresponding to expected, accurately processed 21-nucleotide mature amiR-NbSu or to other 19-24-nucleotide sRNAs. H, RT-PCR detection of PVX, *pri* and shc precursors in apical leaves at 7 and 14 dpa. RT-PCR products corresponding to the control *NbPP2A* are also shown (bottom), as well as control amplifications of *pri* and shc fragments from plasmids.

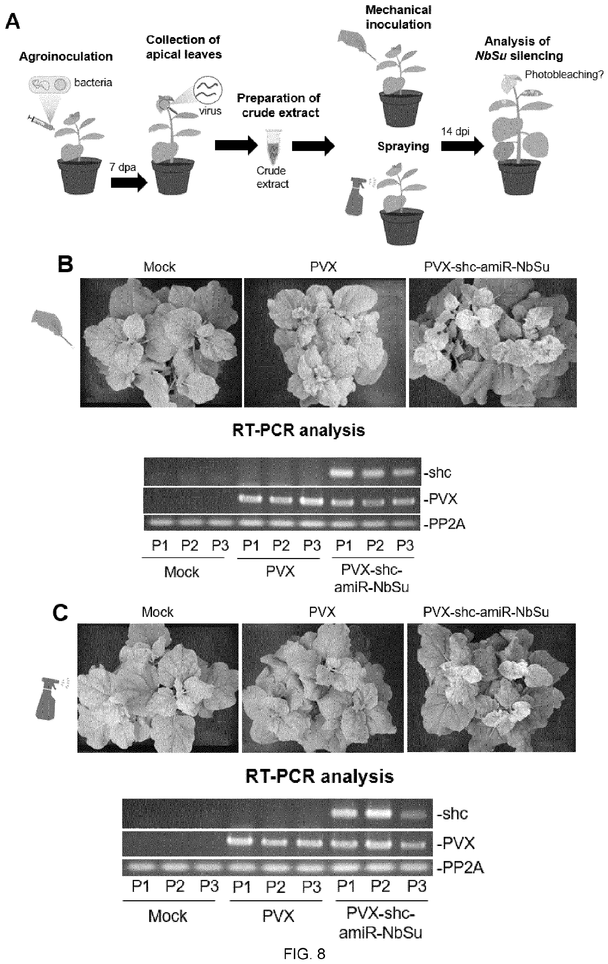

**Figure 8****.** Non-transgenic, DNA-free widespread gene silencing through amiR-VIGS. A, Experimental set up to test gene silencing triggered by *PVX-shc-amiR-NbSu.* B, Widespread *NbSu* silencing induced by mechanically inoculated crude extracts. Left, photos at 14 days post-agroinfiltration (dpa) of sets of three plants mechanically inoculated with different crude extracts obtained from agroinoculated plants. Right, RT-PCR detection of PVX and *shc* precursors in apical leaves at 14 dpa. RT-PCR products corresponding to the control *NbPP2A* are also shown (bottom). C, Widespread *NbSu* silencing induced by sprayed crude extracts. Left, photos at 14 days post-agroinfiltration (dpa) of sets of three plants sprayed with different crude extracts obtained from agroinoculated plants. Right, RT-PCR detection of PVX and shc precursors in apical leaves at 14 dpa. Other details are as in B.

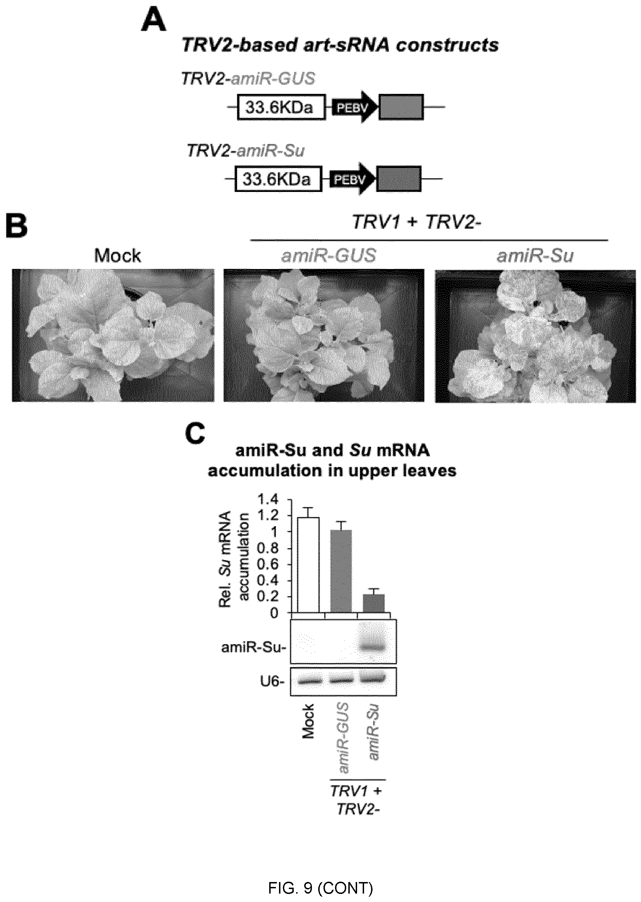

**Figure 9****.** TRV-based amiR-VIGS in *N. benthamiana.* (A) TRV-based art-sRNA constructs. (B) Pictures at 10 dpa. (C) amiR-Su and Su mRNA accumulation in upper leaves at 10 dpa as per northern blot and RT-qPCR analysis, respectively.

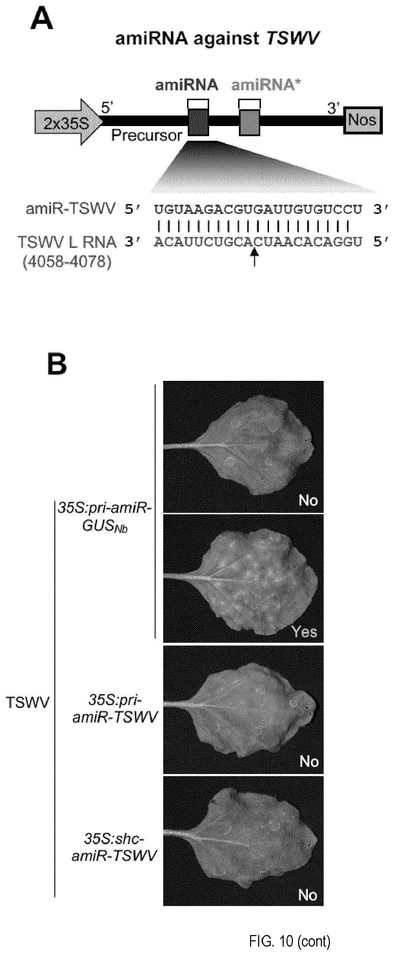

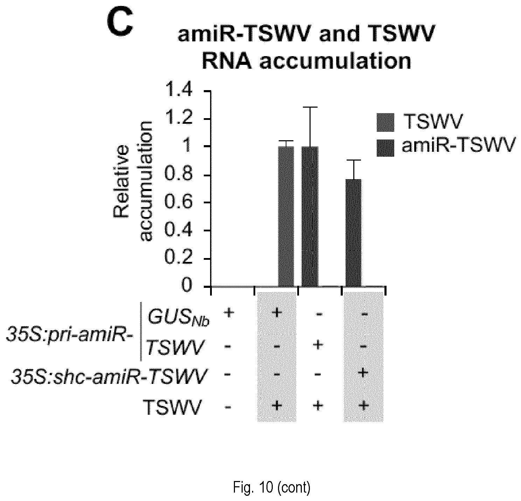

**Figure 10****.** Antiviral effects of constructs expressing amiR-TSWV, an amiRNA against *Tomato spotted wilt virus* (TSWV), from pri and shc precursors. (A) Diagram of amiR-TSWV constructs expressing amiR-TSWV directed against TSWV segment L, with amiRNA and star strand positions in the precursor indicated with grey boxes. Base pairing between amiR-TSWV and its target site is shown, with the predicted cleavage position indicated by an arrow. (B) Photos at 7 days post-agroinfiltration (dpa) of leaves agroinfiltrated with the different constructs, some of which were further inoculated with TSWV. C, Bar graph showing the relative accumulation of amiR-TSWV in agroinfiltrated leaves at 2 dpa [mean relative level (n = 3) + standard deviation amiRNA relative accumulation, pri-amiR-TSWV + TSWV= 1.0] and of TSWV RNA in apical leaves at 21 dpa [mean relative level (n = 3) + standard error of TSWV RNAs after normalization
   to PROTEIN PHOSPHATASE 2A (PP2A), as determined by quantitative RT-qPCR, pri-amiR-GUSNb + TSWV= 1].

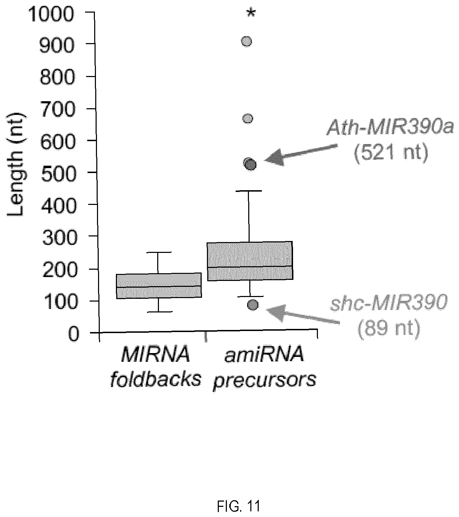

**Figure 11****.** Analysis of the length of *MIRNA* foldbacks and miRNA precursors used for gene silencing in plants.

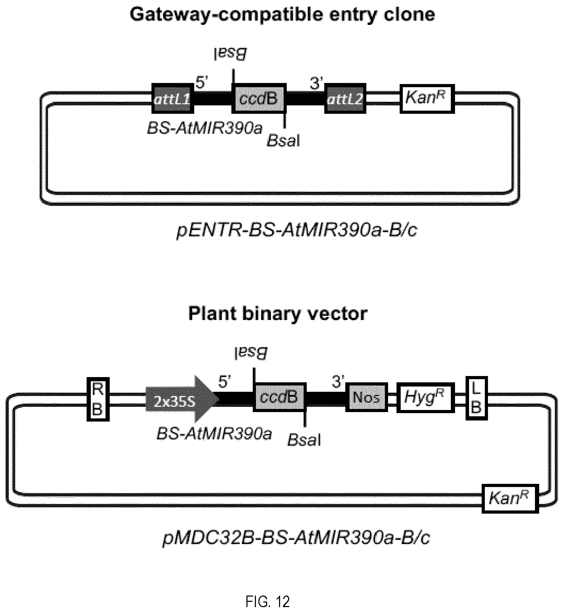

**Figure 12****.** BS-AtMIR390a-B/c-based vectors for direct cloning of amiRNAs. Top, diagram of the Gateway-compatible pENTR-BS-AtMIR390a-B/c entry vector. Bottom, diagram of the pMDC32B-BS-AtMIR390a-B/c binary vector for in plant expression of amiRNAs. RB: right border; 35S: Cauliflower mosaic virus promoter; Bsal: Bsal recognition site, ccdB: gene encoding the gyrase toxin; LB: left border; attL1 and attL2: GATEWAY recombination sites. Kan R: kanamycin resistance gene; Hyg R : hygromycin resistance gene.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

As used herein, the indefinite articles "a" and "an" are synonymous with "at least one" or "one or more." Unless indicated otherwise, definite articles used herein, such as "the" also include the plural of the noun.

For purposes of the present invention, any ranges given include both the lower and the upper endpoints of the range. Ranges given, such as concentrations and the like, should be considered approximate, unless specifically stated. The term "about" refers to a deviation of plus/minus 10 %, preferably plus/minus 5 %.

### I- Viral vectors of the invention

As above described, in a first aspect, the invention provides a viral vector comprising a heterologous nucleotide sequence that encodes, or corresponds to the RNA sequence S1 that is of a length of 56-400 ribonucleotides and that comprises regions R1, R2, R3, R4 and R5 directly linked to each other as indicated in formula (i):

(I) 5'-R1-R2-R3-R4-R5-3',

wherein:
- R1 consists of the RNA sequence of a strand of a basal stem (BS) structure,
- R2 consists of the RNA sequence of a miRNA,
- R3 comprises an RNA sequence that has a length of at least 8 nucleotides,
- R4 consists of an RNA sequence that base pairs with the RNA sequence of R2 to form a miRNA/miRNA* duplex,
- R5 consists of the RNA sequence complementary to R1 in the BS comprising R1,
and wherein the RNA sequence S1 forms a stem-loop structure where the stem comprises regions R1, R2, R4 and R5 wherein region R1 base pairs with region R5 and region R2 base pairs with region R4, and the loop comprises a sequence of at least 4 contiguous nucleotides of region R3.

As well understood by a skilled person, the terms "RNA sequence S1", "sequence S1", or "S1" as used herein, are equivalent and all refer to the RNA sequence S1 as described in the first aspect of the invention. The terms "RNA region R1", "region R1" or "R1" as used herein, are equivalent and all refer to the RNA region R1 as described in the first aspect of the invention. The terms "RNA region R2", "region R2" or "R2" as used herein, are equivalent and all refer to the RNA region R2 as described in the first aspect of the invention. The terms "RNA region R3", "region R3" or "R3" as used herein, are equivalent and all refer to the RNA region R3 as described in the first aspect of the invention. The terms "RNA region R4", "region R4" or "R4" as used herein, are equivalent and all refer to the RNA region R4 as described in the first aspect of the invention. The terms "RNA region R5", "region R5" or "R5" as used herein, are equivalent and all refer to the RNA region R5 as described in the first aspect of the invention.

The term "viral vector", or "plant viral vector", as used herein, refers to the term commonly known by an expert in the field. Viral vectors correspond to the genetic material of a virus or to a modified genetic material of a specific virus, in which a nucleic acid sequence of interest can be inserted to be express in a cell infected with the virus encoded by the viral vector. Indeed,viral vectors have been commonly used to express genetic material of interest in a target cell, by inserting said genetic material of interest in the viral vector. Modifications of the viral genetic material in the viral vectors are commonly addressed to allow or improve the expression of the nucleic acid sequence or protein sequence of interest in the target cell. Several vector design strategies, such as gene replacement, gene insertion using a duplicated subgenomic RNA (sgRNA) promoter (SGP), heterologous SGP, complementation, gene fusions, internal ribosomal entry site (IRES), and deconstructed viruses, can be used for expression of foreign nucleic acid sequences or proteins from virus vectors. Viral vectors are commonly referred to by reference to the virus whose genetic material corresponds or has been used as a template for the obtention of the viral vector. In other words, a viral vector is commonly referred to by reference to the virus from which it derives. For instance, a PVX viral vector corresponds to a viral vector that derives from the PVX virus, hence, with a sequence that corresponds to the PVX genetic material in which a nucleic acid sequence of interest can be inserted, or to the PVX virus genetic material including specific genetic modifications and in which a nucleic acid sequence of interest can be inserted. Non-limiting examples of genetic modifications of the viral genetic material in viral vectors include the deletion of a sequence or part of a sequence encoding a viral protein, for instance, the viral coat protein (CP), the inclusion of a heterologous promoter, such as the *Pea early browning virus* (PEBV) promoter or the *Bamboo mosaic virus* (BaMV) promoter, or the inclusion of a viral protein into the control of a heterologous promoter as just mentioned. In a particular embodiment, the viral vector of the invention comprises a heterologous promoter, particularly the *Pea early browning virus* (PEBV) promoter or the *Bamboo mosaic virus* (BaMV) promoter, particularly the PEBV promoter wherein the viral vector is a TRV vector, more particularly, the BaMV promoter wherein the viral vector is a PVX vector. In an additional embodiment, the sequence encoding the viral CP has been deleted in the viral vector of the invention, and a sequence encoding the viral CP, or a fragment thereof, is inserted under the control of a heterologous promoter, as just mentioned particularly wherein the viral vector is a PVX vector. In a particular embodiment, the sequence encoding the viral CP has been deleted in the viral vector of the invention, and a sequence encoding the viral CP protein with a deletion of an N-terminal fragment, particularly the 29 amino acids at the N-terminal end of the viral CP protein, is inserted under the control of a heterologous promoter as just mentioned, particularly under the BaMV promoter. In a particular embodiment, the sequence encoding the viral CP has been deleted in the viral vector of the invention, and a sequence encoding the viral CP protein with a deletion of an N-terminal fragment, particularly the 29 amino acids at the N-terminal end of the viral CP protein, is inserted under the control of a heterologous promoter as just mentioned, particularly under the BaMV promoter, wherein the viral vector is a PVX vector. In another particular embodiment, the nucleic acid sequence of interest, in particular the nucleic acid sequence encoding or corresponding to S1, is comprised in the viral vector of the invention under the control of an endogenous promoter, particularly under the control of the viral CP promoter. In another particular embodiment, the nucleic acid sequence of interest, in particular the nucleic acid sequence encoding or corresponding to S1, is comprised in the viral vector of the invention under the control of an endogenous promoter, particularly under the control of the viral CP promoterwherein the viral vector is a PVX vector, particularly wherein the viral vector is as further defined in any of the five previous embodiments. In another particular embodiment, the nucleic acid sequence of interest, particularly the sequence encoding or corresponding to S1, is comprised in the viral vector of the invention under the control of a heterologous promoter, particularly under the control of the PEBV promoter or of the BaMV promoter. In another particular embodiment, the nucleic acid sequence of interest, particularly the sequence encoding or corresponding to S1, is comprised in the viral vector of the invention under the control of a heterologous promoter, particularly under the control of the PEBV promoter or of the BaMV promoter wherein the viral vector is a TRV vector. In a further particular embodiment, the sequence encoding or corresponding to S1, is comprised in the viral vector of the invention under the control of the heterologous promoter PEBV promoter, particularly wherein the viral vector is a TRV vector. In a particular embodiment, the PEBV or BaMV promoter, as referred herein, corresponds to or derives from the endogenous promoter of the CP protein in the PEBV, or the endogenous promoter of the CP protein in the BaMV, respectively. In another particular embodiment, the PEBV promoter as referred herein, corresponds to or derives from the endogenous promoter of the CP protein of the PEBV. In another particular embodiment, the BaMV promoter, as used herein, refers to the endogenous promoter of the CP protein of the BaMV. As well understood by a skilled person, the expression promoter is defined by reference to the protein encoded by the gene under the control of said expression promoter, i.e. to which the expression promoter is operatively linked. Thus, a CP promoter, as used herein, refers to a promoter that controls the expression of the gene encoding the viral CP, i.e. to which the gene encoding the viral CP protein is operatively linked.

Viral vectors can be RNA viral vectors or DNA viral vectors, depending on the virus from which they derive.

More particularly, RNA viruses can be single stranded (ss) or double stranded (ds). Single stranded RNA viruses can also be classified according to the sense or polarity of their RNA into negative-sense and positive-sense, or ambisense RNA viruses. Positive-sense viral RNA comprises the viral mRNA sequences that are translated in the cell, and thus can be immediately translated by the host cell. Negative-sense viral RNA is the RNA complementary to that comprising the mRNA sequences that are translated in the cell, and thus must be converted to positive-sense RNA by an RNA-dependent RNA polymerase before translation. Ambisense RNA viruses resemble negative-sense RNA viruses, except they translate genes from their negative and positive strands. DNA viruses can be divided between those that are formed by two strands of DNA, called double-stranded DNA (dsDNA) virueses, and those that have one strand of DNA, called single-stranded DNA (ssDNA) viruses.

Double-stranded RNA (dsRNA) viruses are a polyphyletic group of viruses that have ds genomes made of ribonucleic acid. The ds genome is used as a template by the viral RNA-dependent RNA polymerase (RdRp) to transcribe a positive-strand RNA functioning as messenger RNA (mRNA) for the host cell's ribosomes, which translate it into viral proteins. The positive-strand RNA can also be replicated by the RdRp to create a new ds viral genome.

A viral vector that derives from an RNA virus is herein referred to as an RNA viral vector, and a viral vector derived from a DNA virus is herein referred to as a DNA viral vector. Concomitantly, an RNA viral vector that derives from a ssRNA virus, a ds RNA virus, a ss positive sense viral RNA, or a ss negative sense viral RNA, is herein referred to as a ssRNA viral vector, a dsRNA viral vector, a positive sense ssRNA viral vector, or a negative sense ssRNA viral vector, respectively. Similarly, a viral vector that derives from a ssDNA virus or a dsDNA virus, is herein referred to as a ssDNA viral vector or a dsDNA viral vector, respectively.

In a particular embodiment, the viral vector of the invention is selected from the list consisting of a single stranded RNA (ssRNA) viral vector, a dsRNA viral vector, a ssDNA viral vector and a dsDNA viral vector. In a particular embodiment, the ssRNA viral vector is a positive sense viral vector or a negative sense viral vector, particularly a positive sense viral vector. In a more particular embodiment, the viral vector of the first aspect of the invention is a positive sense ssRNA viral vector.

In a particular embodiment, the viral vector of the first aspect of the invention is a ssRNA viral vector selected from the list consisting of Potato virus X (PVX) viral vector, Tobacco rattle virus (TRV) viral vector, Tobacco mosaic virus (TMV), tomato golden mosaic virus (TGMV), Cucumber mosaic virus (CMV), Barley stripe mosaic virus (BSMV), brome mosaic virus (BMV), and Foxtail mosaic virus (FoMV). In a particular embodiment, the viral vector of the first aspect of the invention is an RNA viral vector selected from the list consisting of PVX viral vector, and TRV viral vector. In a more particular embodiment of the first aspect, the viral vector is a PVX viral vector. In another particular embodiment of the first aspect, the viral vector is a TRV viral vector. In another embodiment, the viral vector of the first aspect of the invention is a DNA viral vector selected from the list consisting of Bean yellow dwarf virus (BYDV), Wheat dwarf virus (WDV), and Cabbage leaf curl virus (CabLCV).

The TRV is a member of the genus *Tobravirus* in the family of *Virgaviridae* and has a bipartite, positive sense ssRNA genome (RNA1 and RNA2). RNA1 and RNA2 are encapsidated separately into tubular, rigid, rod-shaped particles. The RNA1 encoded-proteins are sufficient for replication and movement of the virus within the host plant, while RNA2 encodes proteins for virion formation and nematode-mediated transmission. RNA1 encodes four open reading frames (ORFs) for proteins with predicted molecular weights of 134 and 194 kDa (replicase proteins), 29 kDa (movement protein), and 16 kDa (cysteine-rich protein, a silencing suppressor protein). RNA1 can replicate and move systemically without RNA2. RNA2 encodes three proteins (coat protein and two non-structural proteins, 29.4 kDa and 32.8 kDa). In a particular embodiment, the TRV vector, as referred herein, comprises the TRV RNA1 and RNA2 single stranded RNA genomes, or RNAs derived from the TRV RNA1 and RNA2 single stranded RNA genomes. In another particular embodiment, the viral vector of the invention is a TRV viral vector that comprises the RNA sequences derived from the RNA1 and RNA2 single stranded RNA genomes, wherein the heterologous nucleic acid sequence encoding or corresponding to S1, is comprised in the RNA sequence derived from the RNA2 ssRNA, particularly under the control of a heterologous promoter, more particularly under the control of the PEBV promoter. More particularly, the heterologous nucleic acid sequence encoding or corresponding to S1 is comprised between the first and the second ORF found in the TRV RNA2 ssRNA in the viral vector of the invention, particularly between the sequence encoding the viral CP and the sequence encoding the non-structural proteins of 29.4 kDa in the viral vector of the invention, particularly, wherein the viral vector of the invention comprises the RNA sequences derived from the RNA1 and RNA2

The term "region", in the context of the RNA sequence S1 as defined herein, refers to an RNA sequence that is a subunit of S1. Thus, said region is a short RNA sequence comprised within the RNA sequence S1, as defined herein, and with a length shorter than that of sequence S1, i.e. with a number of nucleotides lower than the number of nucleotides of S1. As well understood by a skilled person, the length of S1 is equal or higher the sum of the lengths of regions R1, R2, R3, R4 and R5 comprised in S1. When the length of S1 is higher than the sum of the lengths of regions R1, R2, R3, R4, and R5, S1 comprises RNA sequences or ribonucleotides in addition to those of regions R1, R2, R3, R4, and R5. In a particular embodiment, the length of S1 is equal to the sum of the lengths of regions R1, R2, R3, R4, and R5. In a particular embodiment, S1 consists of regions R1, R2, R3, R4, and R5.

As well understood by a skilled person, the nucleotides of the RNA sequence S1 are ribonucleotides. Thus, the nucleotides of regions R1, R2, R3, R4 and R5 consist of ribonucleotides. In a particular embodiment, the nucleotides referred herein as part of the RNA sequence S1, of region R1, R2, R3, R4, and/or R5 are ribonucleotides.

As well understood by a skilled person, S1 comprises in the 5' end to the 3' end direction, directly linked to each other, R1, R2, R3, R4, and R5. As well understood by a skilled person, "directly linked to each other" in the context of sequence S1, indicates that R1 is directly linked to R2, R2 is directly linked to R3, R3 is directly linked to R4, and R4 is directly linked to R5.

The term "directly linked", as used herein refers to the fact the 3' end nucleotide of an RNA region, or RNA sequence, is linked to the 5' end nucleotide of another RNA region, or RNA sequence, by a covalent bond, particularly by a phosphodiester bond. More specifically, the phophodiester bond is formed between the 3'-OH of the 3' end nucleotide of one RNA region or RNA sequence, and the 5' phosphate group of the 5' end nucleotide of another RNA region or RNA sequence.

In a particular embodiment of the first aspect of the invention, the RNA sequence S1 has a length 56-400, 56-400, 56-350, 56-300, 56-250, 56-200, 56-150, or 56-100. In another particular embodiment, the RNA sequence S1 has a length of 60-400, 60-350, 60-300, 60-250, 60-200, 60-150, 60-100. In another particular embodiment, the RNA sequence S1 has a length of 56-400, 60-400, 64-400, 74-400, 80-400, 82-400, 85-400, 86-400, 87-400, 88-400, or 89-400 nucleotides, particularly 89-400 nucleotides. In another particular embodiment, the RNA sequence S1 has a length of 56-350, 60-350, 64-350, 74-350, 80-350, 82-350, 85-350, 86-350, 87-350, 88-350, or 89-350 nucleotides, particularly 89-350 nucleotides. In another particular embodiment, of the first aspect of the invention, the RNA sequence S1 has a length of at least 56, at least 60, at least 64, at least 74, at least 80, at least 82, at least 85, at least 87, at least 88, at least 89, at least 90, at least 92, at least 95, at least 100, at least 125, at least 150, at least 175, at least 200, at least 250, at least 275, at least 300, at least 350, or at least 400 nucleotides, particularly at least 89 nucleotides. In another particular embodiment, the RNA sequence S1 has a length of 60, 64, 74, 80, 81, 82, 85, 86,87, 88, 89, 90, 92, 95,100, 125, 150, 175, 200, 250, 275, 300, 350, or 400 nucleotides, particularly of 89 nucleotides.

In a particular embodiment of the first aspect, the length of the sequence comprised in S1 consisting of regions R1, R2, R3, R4, and R5, as defined herein has a length of at least 56, at least 58, at least 60, at least 64, at least 68, at least 70, at least 72, at least 74, at least 76, at least 78, at least 80, at least 81, at least 82, at least 85, at least 86, at least 87, at least 88, at least 89, at least 90, at least 92, at least 95, at least 100, at least 105, at least 107, at least 110, at least 114, at least 115, at least 117, at least 120, at least 125, at least 130, at least 135, at least 140, at least 150, at least 155, at least 158, at least 160, particularly of at least 89 nucleotides. In a particular embodiment, the length of the sequence comprised in S1 consisting of regions R1, R2, R3, R4, and R5, as defined herein has a length of 56, 58, 60, 64, 68, 70, 72, 74, 76, 78, 80, 81, 82, 85, 86,87, 88, 89, 90, 92, 95, 100, 105, 107, 110, 114, 115, 117, 120, 125, 130, 135, 140, 150, 155, 158, 160, particularly of 89 nucleotides.

In another particular embodiment of the first aspect, the RNA sequence S1 consists of the regions R1, R2, R3, R4, R5 and at least 1, at least 2, at least 3, at least 4, at least 5, at least 10, at least 15, at least 20, at least 30, at least 40, at least 50, at least 75, at least 100, at least 150, at least 200 at least 250, at least 300, at least 311, at least 346 nucleotides upstream region R1 in a 5' to 3'-end direction. In another particular embodiment of the first aspect, the RNA sequence S1 consists of the regions R1, R2, R3, R4, R5 and 5-346, 5-311, 5-300, 5-250, 5-200, 5-150, 5-100, 5-50, 5-40, 5-30, 5-20, 5-15, or 5-10 nucleotides upstream region R1 in a 5' to 3'-end direction. In another particular embodiment of the first aspect, the RNA sequence S1 consists of the regions R1, R2, R3, R4, R5 and at least 1, at least 2, at least 3, at least 4, at least 5, at least 10, at least 15, at least 20, at least 30, at least 40, at least 50, at least 75, at least 100, at least 150, at least 200 at least 250, at least 300, at least 311, at least 346 nucleotides downstream region R5 in a 5' to 3'-end direction. In another particular embodiment of the first aspect, the RNA sequence S1 consists of the regions R1, R2, R3, R4, R5 and 5-346, 5-311, 5-300, 5-250, 5-200, 5-150, 5-100, 5-50, 5-40, 5-30, 10-20, 5-15, or 5-10 nucleotides downstream of region R5 in a 5' to 3'-end direction. In another particular embodiment, the RNA sequence S1 consists of the regions R1, R2, R3, R4, R5, and at least 3, particularly at least 15 nucleotides upstream region R1 in a 5' to 3'-end direction, and at least 3, particularly at least 15 nucleotides downstream of region R5 in a 5' to 3'-end direction. In another particular embodiment, the RNA sequence S1 consists of the regions R1, R2, R3, R4, R5, and 5-15, particularly at least 5-100 nucleotides upstream region R1 in a 5' to 3'-end direction, and 5-15, particularly 5-100 nucleotides downstream of region R5 in a 5' to 3'-end direction.

The term "nucleic acid", as used herein, relates to a deoxyribonucleotide or ribonucleotide polymer in either single or double stranded form and, unless otherwise limited, encompasses natural nucleotides and analogues of natural nucleotides that hybridize to nucleic acids in a manner similar to naturally occurring nucleotides. The term "nucleotide" includes, but is not limited to, a monomer that includes a base (such as a pyrimidine, purine or synthetic analogs thereof) linked to a sugar (such as ribose, deoxyribose or synthetic analogs thereof), or a base linked to an amino acid, as in a peptide nucleic acid (PNA). A nucleotide is one monomer in an oligonucleotide or in a polynucleotide. A "nucleotide sequence" or "nucleic acid sequence" refers to the sequence of bases in an oligonucleotide or in a polynucleotide.

The term "length" or "has a length of", as used herein in the context of an RNA sequence, refers to the number of nucleotides that said RNA sequence has. When used in the context of the length of an RNA duplex, it can be indicated as the number of nucleotides comprised in said duplex, o as the number of bp, i.e. nucleotides base paired to each other or pairs of nucleotides complementary to each other, in said duplex. In a particular embodiment, the heterologous nucleotide sequence that encodes, or corresponds to the RNA sequence S1 in the viral vector of the invention is operatively linked to an expression promoter. Examples of suitable expression promoters include those conventionally used in molecular biology and known to the skilled person. In a particular embodiment of the first aspect, the expression promoter to which the heterologous nucleotide sequence that encodes, or corresponds to S1 is operatively linked in the viral vector of the invention, is selected from the list consisting of the PEBV promoter, the BaMV promoter, and the CP promoter, particularly the PVX CP promoter. In another particular embodiment of the first aspect, the expression promoter to which the heterologous nucleotide sequence that encodes, or corresponds to S1 is operatively linked in the viral vector of the invention, is selected from the list consisting of the endogenous promoter of the CP protein in the PEBV, the endogenous promoter of the CP protein in the BaMV, the endogenous promoter of the CP protein in the PVX, and the endogenous promoter of the CP protein in the viral vector of the invention. In another particular embodiment of the first aspect, the expression promoter to which the heterologous nucleotide sequence that encodes, or corresponds to S1 is operatively linked in the viral vector of the invention, is an expression promoter derived from an expression promoter selected from the list consisting of the endogenous promoter of the CP protein in the PEBV, the endogenous promoter of the CP protein in the BaMV, the endogenous promoter of the CP protein in the PVX, and the endogenous promoter of the CP protein of the viral vector of the invention.

In a particular embodiment of the first aspect, the heterologous sequence that encodes, or corresponds to S1 is operatively linked to the PEBV expression promoter, particularly to the expression promoter consisting of or derived from the endogenous CP promoter in the PEBV. In a particular embodiment of the first aspect, the viral vector of the invention is a TRV vector, and the heterologous sequence that encodes, or corresponds to S1 is operatively linked to the PEBV expression promoter, particularly to the expression promoter consisting of or derived from the endogenous CP promoter in the PEBV. In another particular embodiment of the first aspect, the heterologous sequence that encodes, or corresponds to S1 is operatively linked to the endogenous expression promoter of the CP protein in the viral vector of the invention, particularly wherein the viral vector is a PVX viral vector.

The expression "operatively linked to an expression promoter" as used herein in the context of a nucleotide sequence in the viral vector of the invention, indicates that said nucleotide sequence in the viral vector operatively linked to the expression promoter, is under the control of the expression promoter so that synthesis of the corresponding RNA sequence can take place from the sequence of the expression promoter. As well understood by a skilled person, the nucleotide sequence operatively linked to the expression promoter does not need to be directly linked to the corresponding promoter sequence and nucleotides can be comprised between the 3' end of the corresponding sequence of the expression promoter and the 5' end of the nucleotide sequence operatively linked to the expression promoter.

The term "heterologous sequence", as used herein refers to any nucleic acid sequence comprised in the viral vector of the invention that is not comprised in the viral DNA or RNA from which the viral vector derives. In other words, a heterologous sequence is a segment of nucleic acid within or attached to another nucleic acid molecule that is not found in association with the other molecule in nature. For example, a heterologous region of a nucleic acid construct could include a coding sequence flanked by sequences not found in association with the coding sequence in nature.

In a particular embodiment, the viral vector of the invention comprises, in addition to the heterologous nucleotide sequence that encodes, or corresponds to the RNA sequence S1, additional heterologous sequences. In a particular embodiment, said additional heterologous sequences comprise the sequence of a heterologous expression promoter.

In a particular embodiment, the sum of the lengths of all the heterologous sequences comprised in the viral vector of the first aspect is equal to less than 400, 375, 350, 325, 300, 275,250, 225, 220, 215, 210, 200, 190, 180, 170, 160, 150, 140, 130, 120, 110, 105, 100, 95, 94, 93, 92, 91, 90, 89, 87, 86, 85, 82, 80, 75, 70, 65, 62, 60 nucleotides, particularly less than 300 nucleotides. In another particular embodiment, the sum of the lengths of all the heterologous sequences comprised in the viral vector of the first aspect of the invention is equal to less than 250 nucleotides. In another particular embodiment, the sum of the lengths of all the heterologous sequences comprised in the viral vector of the first aspect of the invention is equal to less than 150 nucleotides. In another particular embodiment, the sum of the lengths of all the heterologous sequences comprised in the viral vector of the first aspect of the invention is equal to less than 125 nucleotides. In another particular embodiment, the sum of the lengths of all the heterologous sequences comprised in the viral vector of the first aspect of the invention is equal to less than 100 nucleotides. In another particular embodiment, the sum of the lengths of all the heterologous sequences comprised in the viral vector of the first aspect of the invention is equal to less than 95 nucleotides. In another particular embodiment, the sum of the lengths of all the heterologous sequences comprised in the viral vector of the first aspect of the invention is equal to less than 90 nucleotides. In another particular embodiment, the sum of the lengths of all the heterologous sequences comprised in the viral vector of the first aspect of the invention is equal to less than 89 nucleotides. In another particular embodiment, the sum of the lengths of all the heterologous sequences comprised in the viral vector of the first aspect of the invention is equal to less than 88 nucleotides. In another particular embodiment, the sum of the lengths of all the heterologous sequences comprised in the viral vector of the first aspect of the invention is equal to less than 87 nucleotides. In another particular embodiment, the sum of the lengths of all the heterologous sequences comprised in the viral vector of the first aspect of the invention is equal to less than 86 nucleotides. In another particular embodiment, the sum of the lengths of all the heterologous sequences comprised in the viral vector of the first aspect of the invention is equal to less than 85 nucleotides. In another particular embodiment, the sum of the lengths of all the heterologous sequences comprised in the viral vector of the first aspect of the invention is equal to less than 82 nucleotides. In another particular embodiment, the sum of the lengths of all the heterologous sequences comprised in the viral vector of the first aspect of the invention is equal to less than 80 nucleotides. In another particular embodiment, the sum of the lengths of all the heterologous sequences comprised in the viral vector of the first aspect of the invention is equal to less than 75 nucleotides. In another particular embodiment, the sum of the lengths of all the heterologous sequences comprised in the viral vector of the first aspect of the invention is equal to less than 70 nucleotides. In another particular embodiment, the sum of the lengths of all the heterologous sequences comprised in the viral vector of the first aspect of the invention is equal to less than 65 nucleotides. In another particular embodiment, the sum of the lengths of all the heterologous sequences comprised in the viral vector of the first aspect of the invention is equal to less than 62 nucleotides. In another particular embodiment, the sum of the lengths of all the heterologous sequences comprised in the viral vector of the first aspect of the invention is equal to less than 61 nucleotides. In another particular embodiment, the sum of the lengths of all the heterologous sequences comprised in the viral vector of the first aspect of the invention is equal to less than 57 nucleotides. In another particular embodiment, the sum of the lengths of all the heterologous sequences comprised in the viral vector of the first aspect of the invention is of 56-400, 56-350, 56-300, 56-250, 56-200, 56-150, or 56-100 nucleotides. In another particular embodiment, the sum of the lengths of all the heterologous sequences comprised in the viral vector of the first aspect of the invention is of 60-400, 60-350, 60-300, 60-250, 60-200, 60-150, or 60-100 nucleotides. In another particular embodiment, the sum of the lengths of all the heterologous sequences comprised in the viral vector of the first aspect of the invention is of 85-400, 89-350, 90-350, 95-350, 100-350, 100-300,150-250, or 150-200 nucleotides, particularly, 100-300 nucleotides.

The term "miRNA", "microRNA" or "miR" as used herein refers to the term well-known by an expert in the field. MiRNAs are regulatory RNAs that are expressed in animals and plants and affect the translation or stability of target mRNAs. Mature miRNAs are single stranded RNAs of 20-24 nucleotides (nt) in length that base-pair with their target mRNA to negatively regulate its expression.

Plant miRNAs regulate the expression of genes encoding transcription factors and proteins that impact key biological processes such as growth, development, stress adaptation or hormone regulation. MiRNA biogenesis in plants occurs entirely in the cell nucleus, and initiates with the transcription of MIR genes by RNA polymerase II, resulting in the synthesis of large primary miRNA precursors (or "pri-miRNAs") that are highly variable in length (from hundreds to thousands of nucleotides) and in secondary structure. Pri-miRNAs are capped, spliced and polyadenylated, and harbor a characteristic foldback (or hairpin) structure that also varies in length (from 50 to 900 nt). MiRNA foldbacks have a common structure consisting of a ~13-17 bp basal stem (BS), a miRNA/miRNA* duplex, and a distal stem-loop (DSL) region variable in size and shape and flanked by single-stranded RNA (ssRNA) segments. Processing of most pri-miRNAs occurs through a "base-to-loop" mechanism in which an nuclear RNase III DICER-LIKE (DCL) protein, commonly the DCL1 associated with its accessory proteins SERRATE (SE) and HYPONASTIC LEAVES1 (HYL1), first cleaves at the BS of the foldback to release a shorter precursor miRNA (pre-miRNA). Next, a second cleavage at a 21 nucleotides distance from the first cut releases the miRNA/miRNA* duplex including two-nucleotide 3' overhangs that are 2'-O-methylated by the methyltransferase HUA ENHANCER1 (HEN1). Alternatively, for loop-to-base processed precursors, the DCL protein, commonly DCL1, produces a first cut that releases the terminal loop and then continues towards the base of the precursor to release the miRNA/miRNA* duplex. Typically, one of the strands of the duplex (the guide strand) is sorted out into an ARGONAUTE (AGO) protein (AGO1 for the majority of plant miRNAs) resulting in an RNA-induced silencing complex (RISC) that recognizes and silences complementary target RNAs mostly through their endonucleolytic cleavage, and less frequently through their translational repression. The other strand of the duplex (the "passenger strand" or "star strand") is subject to degradation.

As well understood by a skilled person, the term "miRNA/miRNA* duplex" or "miRNA:miRNA* duplex", as used herein, refers to the duplex molecule formed during the processing of a pri-miRNA consisting of a passenger strand and a mature microRNA strand (or guide strand) commonly denoted miRNA:miRNA*, where the passenger strand is designated miRNA*. Commonly, the miRNA/miRNA* duplex is an imperfect duplex, i.e. comprises unpaired nucleotides within the duplex structure. Thus, the mature miRNA or guide strand of a miRNA and the passenger strand of a miRNA base pair to each other, as defined herein,in the miRNA/miRNA* duplex, as well as miRNA foldback that is part of the pri-mRNA. The term "mature miRNA", or "guide strand of a miRNA", or "guide strand of the miRNA/miRNA* duplex", as used herein, refers to the 20-24 nt long ssRNA molecule of the miRNA/miRNA* duplex that is sorted out into an ARGONAUTE (AGO) protein (AGO1 for the majority of plant miRNAs) resulting in an RNA-induced silencing complex (RISC). The mature miRNA functions as a guide to direct the RISC complex into the target mRNA, by base-pairing with the target mRNA to negatively regulate its expression. Thus, the mature miRNA or guide strand is the RNA strand of a miRNAthat comprises the sequence that base pairs with the target mRNA sequence. The term "passenger strand of a miRNA", or "passenger strand of miRNA/miRNA* duplex" or "star strand of a miRNA" or "star strand of a miRNA/miRNA* duplex", as used herein, refers to the strand that base pairs with the guide strand as defined herein in the miRNA/miRNA* duplex. The passenger strand commonly has the same length as the guide strand. The base pairing rules for the base pairing between the guide strand and the passenger strand in a miRNA/miRNA duplex are known by an expert in the field. For instance, commonly two-nucleotides overhang at the 3' end of the guide strand and at the 3' end of the passenger strand, and at least nucleotides at positions 2-13 of the miRNA guide strand in a 5'- to 3'-end direction base pair with the corresponding nucleotides in the passenger strand. One or two mismatches or bulges, particularly one mismatch or bugle, can be comprised in the duplex formed between nucleotides 2-13 of the guide strand and the corresponding nucleotides in the passenger strand. More detailed base pairing rules for the base pairing between the guide strand and the passenger strand in a miRNA/miRNA duplex are disclosed in the examples below (see for instance Example 1).

In a particular embodiment, the miRNA/miRNA* duplex of the miRNA of the invention comprises a two-nucleotide overhang at the 3' end of the guide strand, and a two-nucleotide overhang a the 3' end of the star strand.

The term "base pairs", "base pairing" or "is complementary" as used herein, refers to the interaction between at least two nucleotides by hydrogen bonds. Base pairing between ribonucleotides, as used herein, includes Watson-crick base pairing (A-U and C-G ribonucleotide base pairing) and wobble base pairing (particularly G-U ribonucleotide base pairing). In a particular embodiment, base pairing as used herein refers to base pairing between two RNA molecules or two regions within an RNA molecule. When several nucleotides in a sequence base pair with several nucleotides of another sequence, said sequences are commonly understood to base pair with each other or to be complementary to each other. Base pairing between two sequences can be complete or perfect, i.e. all the nucleotides within a sequence base pair with all the nucleotides of another sequence, or partial or imperfect, wherein several but not all the nucleotides of one sequence base pair with nucleotides in the other sequence. When at least two contiguous nucleotides in an RNA sequence base pair with nucleotides in another RNA sequence, the resulting structure motif is described as a stem, a duplex or a helix. Those nucleotides that do not base pair within a duplex or stem structure are generally considered to form mismatches or bulges within the duplex. As well known by an expert in the field, a mismatch occurs when two complementary nucleotide pairs are separated by a single non complementary pair of nucleotides in an RNA duplex.. A bulge is the result of at least one unpaired nucleotide in only one strand of duplex. Base pairing, as well as the degree of base pairing (number or percentage of base paired nucleotides within a duplex), between two nucleotide sequences can be easily predicted, following the Watson and Crick or wobble base paring rules indicated above. Additional methods to determine the base pairing that can occur between two RNA sequences are well-known by an expert in the field and include the use of computer programs such as RNASoft, or PairFold.

The term "stem loop" or "hairpin", as used herein refers to a molecular structure formed by a ds stem and a loop structure at an end of the stem. It comprises the intramolecular base pairing between ssRNA sequences within two regions of the same RNA molecule that are complementary to each other. The formation of a stem-loop structure is dependent on the stability of the resulting stem and loop regions. The first prerequisite is the presence of a sequence that can fold back on itself to form a paired double helix. The stability of this helix is determined by its length, the number of mismatches or bulges it contains (a small number are tolerable, especially in a long helix) and the nucleotide composition of the paired region. Pairings between guanine and cytosine have three hydrogen bonds and are more stable compared to adenine-uracil, or guanine-uracil pairings, which have only two. The stability of the loop also influences the formation of the stem-loop structure. The term "loop", corresponds to the RNA structure well-known by an expert in the field, formed by a sequence of single stranded nucleotides at the end of a stem structure that connects the two strands of the stem structure. Loops that are fewer than three bases long are sterically compromised and do not form. Large loops with no secondary structure of their own (such as pseudoknot pairing) are also unstable. Optimal loop length tends to be about 4-8 bases long. Methods to determine if an RNA molecule forms a stem loop structure are well-known by an expert in the field and include those allowing to determine the secondary structure of an RNA molecule. Non-limiting examples of said methods include bioinformatic tools such as RNAfold or mFOLD that predict the secondary structure of RNA sequences, and laboratory techniques such as Nuclear magnetic resonance (NMR) or Cryo-EM.

In a particular embodiment, the base pairing in the stem of the stem loop formed by RNA sequence S1 is not perfect. In another particular embodiment, the base pairing in the stem of the stem loop formed by RNA sequence S1 comprises at least 1 mismatch, at least 2 mismatches, or at least 3 mismatches, particularly at least 1 mismatch. In another particular embodiment, the base pairing in the stem of the stem loop formed by RNA sequence S1 comprises 1 mismatch, 2 mismatches, 3 mismatches, particularly 1 mismatch. In another particular embodiment, the base pairing in the stem of the stem loop formed by RNA sequence S1 comprises at least 1 bulge, at least 2 bulges, at least 3 bulges, particularly at least 1 bulge. In a particular embodiment, the stem of the stem loop formed by RNA sequence S1 comprises 15%, 12%, 11 %, 10%, 9%, 8%, 7%, 6%, 5%, 4%, 3%, 2%, particularly 11% unpaired nucleotides. The "percentage of unpaired nucleotides" in a stem structure, as used herein, refers to the number of unpaired nucleotides within the stem structure divided by the total number of nucleotides forming the stem structure, and multiplied by 100.

The term "RNA sequence variants" or "RNA variants" or "variants" in the context of RNA sequences, are well understood by those of skill in the art and can involve nucleotide sequence modifications. For example, nucleotide sequence modifications typically fall into one or more of three classes: substitutional, insertional, or deletional variants.

In the present invention the term "identical" or "identity" refers to the percentage of nucleotides that are identical in the two sequences when the sequences are optimally aligned. If, in the optimal alignment, a position in a first sequence is occupied by the same nucleotide residue as the corresponding position in the second sequence, the sequences exhibit identity with respect to that position. The percentage of identity determines the number of identical residues over a defined length in a given alignment. Thus, the level of identity between two sequences or ("percent sequence identity") is measured as a ratio of the number of identical positions shared by the sequences with respect to the number of positions compared (i.e., percent sequence identity = (number of identical positions/total number of positions compared) x 100). A gap, i.e., a position in an alignment where a residue is present in one sequence but not in the other, is regarded as a position with non-identical residues and is counted as a compared position.

As an illustration, an RNA region having a nucleotide sequence being at least, for example, 75% identical to a reference RNA sequence is intended that the nucleic sequence of the RNA region is identical to the reference sequence except that the RNA sequence may include up to one nucleic acid alteration per each 4 nucleic acids of the reference sequence. In other words, to obtain an RNA region having a nucleic acid sequence of at least 75% identical to a reference nucleic acid sequence, up to 25% of the nucleic acid residues in the reference sequence may be deleted or substituted with another nucleic acid, or a number of nucleic acids up to 25% of the total nucleic acid residues in the reference sequence may be inserted into the reference sequence. These alterations of the reference sequence may occur at the 5' or 3' end terminal positions of the reference sequence or anywhere between those terminal positions, interspersed either individually among residues in the reference sequence or in one or more contiguous groups within the reference sequence.

A number of mathematical algorithms for rapidly obtaining the optimal alignment and calculating identity between two or more sequences are known and incorporated into a number of available software programs. For purposes of the present invention, the sequence identity between two nucleic acid sequences is preferably determined using algorithms based on global alignment, such as the Needleman-Wunsch algorithm (Needleman and Wunsch, 1970, J. Mol. Biol. 48: 443-453), preferably implemented in the Needle program of the EMBOSS package (EMBOSS: The European Molecular Biology Open Software Suite, Rice et al., 2000, Trends Genet. 16: 276-277); or the BLAST Global Alignment tool (Altschul et al., "Basic local alignment search tool", 1990, J. Mol. Biol, v. 215, pages 403-410), using default settings. Local alignment also can be used when the sequences being compared are substantially the same length.

### I. 1- Regions R1 and R5 of the RNA sequence S1

As indicated in the definition of the term "miRNA", a miRNA foldback structure corresponds to a foldback or hairpin structure commonly comprised in a primary miRNA precursor, wherein the miRNA foldback structure consists of a BS, the miRNA/miRNA* duplex and a distal loop (DSL). The miRNA foldback structure is the structure within a pri-miRNA that is recognized and processed by a DCL protein (particularly DCL1 or DCL4), which results in the release of the miRNA/miRNA* duplex and subsequence silencing of the target mRNA. Thus, the term "basal stem" or "BS", as used herein, refers to the basal stem of a miRNA foldback structure. Thus, the basal stem, as used herein, corresponds to the stem structure that is immediately previous to the miRNA/miRNA* duplex in a miRNA foldback structure, in a base to loop direction. The term "immediately previous", as used herein, indicates that each strand of the basal stem is directly linked to a strand of the miRNA/miRNA* duplex, i.e. no nucleotides are comprised between the basal stem of the miRNA foldback structure and the miRNA/miRNA* duplex. The term "base to loop direction", or "base to loop direction of the miRNA foldback structure", as used herein, refers to the term commonly known by an expert in the field. Particularly, said term indicates that the direction used corresponds to that starting at the open end of the stem loop structure and ending at the closed loop end of the stem loop structure, i.e. from the stem to the loop of the stem loop structure.

The region of the stem loop formed by the RNA sequence S1 consisting of regions R1 and R5 base paired to each other is herein also referred to as the "R1-R5 region". As well understood by a skilled person, the R1-R5 region consists of an RNA stem structure as defined herein.

The term "miRNA foldback", or "miRNA stem loop", as used herein, refers to the hairpin or stem loop structure comprised in a primary miRNA precursor, as indicated in the definition of "miRNA" above, that consists of a BS, a miRNA/miRNA* duplex and a distal stem loop. As well known by an expert in the field, miRNA foldbacks are formed by imperfect base-pairing.

The term "basal stem of miRNA foldback", or "BS of a miRNA foldback", or "BS", or "basal stem of miRNA stem loop structure", or "lower stem structure of a miRNA foldback", or "lower stem structure of a miRNA stem loop structure", or "BS" as used herein, refers to the basal stem or stem structure comprised in the miRNA foldback as defined above, i.e. in the miRNA foldback of the corresponding primary miRNA precursor or comprised in the corresponding primary miRNA precursorAs indicated therein, the formation of the miRNA foldback structure allows the processing and release of the corresponding miRNA/miRNA* duplex from a pri-miRNA.

As well understood by a skilled person, the basal stem in a miRNA foldback structure is formed by two strands, or consists of two strands, that base pair to each other:
- one strand of the BS is at the 5' end of the foldback structure, or is at the 5' end of the RNA sequence (or RNA) that forms the miRNA foldback structure, or is the strand of the BS whose RNA sequence is located upstream the miRNA sequence in the primary miRNA precursor in a 5'- to 3'-end direction, and
- the other strand of the BS is at 3' end of the foldback structure or is at the 3' end of the RNA sequence (or RNA) that forms the miRNA foldback structure, or is the strand of the BS whose RNA sequence is located downstream the mature miRNA sequence in the primary miRNA precursor in a 5'- to 3'-end direction.

Thus, in a particular embodiment of the first aspect, R1 consists of the RNA sequence of a strand of the BS in a miRNA foldback structure, particularly that is at the 5'end of a miRNA foldback structure, more particularly at the 5' end of the RNA sequence (or RNA) that forms the miRNA foldback structure. In a more particular embodiment, R1 consists of the RNA sequence of a strand of a BS of a miRNA foldback structure, wherein the sequence of said BS strand is located, in a 5'- to 3'-end direction, upstream the mature miRNA sequence in the primary miRNA precursor comprising said miRNA foldback structure.

In another particular embodiment, R5 consists of the RNA sequence complementary to R1 in the BS comprising R1, and that is at the 3'end of the miRNA foldback structure comprising said BS, more particularly, that is at the 3'end of the RNA sequence (or RNA) that forms the miRNA foldback structure comprising said BS. In another particular embodiment, R5 consists of the RNA sequence of the BS comprising the sequence of R1, that is located, in a 5'- to 3'-end direction, downstream the mature miRNA sequence in the primary miRNA precursor comprising said miRNA foldback structure.

Base pairing, or complementarity between the strands of a BS in a miRNA foldback structure is commonly imperfect. Indeed, it is currently thought that the presence of at least one bulge, or of at least one mismatch within the BS is important for DCL-mediated processing and release of the miRNA/miRNA* duplex. In a particular embodiment of the first aspect, R5 consists of the RNA sequence complementary to R1 in the BS comprising R1, particularly consists of the RNA sequence of the strand of the BS comprising R1 that is complementary to R1, wherein the complementarity between the two strands of the BS is not perfect. In a particular embodiment, the BS formed by, or consisting of, R1 and R5 base paired to each other, as defined in the first aspect of the invention comprises at least 1, at least 2, at least 3 at least 4 at least 5 at least 6 at least 7 at least 8, at least 9, at least 10, particularly at least 1 unpaired nucleotide. In a particular embodiment the BS formed by, or consisting of, R1 and R5 base paired to each other comprises 1, 2, 3, 4, 5, 6, 7, 8, 9., 10, particularly 2 unpaired nucleotides.

In another particular embodiment of the first aspect, the BS formed by, or consisting of, R1 and R5 base paired to each other, as defined in the first aspect of the invention, comprises at least one bulge, at least two bulges, at least 3 bulges, more particularly, at least one bulge structure. In another particular embodiment of the first aspect, the BS formed by, or consisting of, R1 and R5 base paired to each other, as defined in the first aspect of the invention, comprises one bulge, two bulges, 3 bulges, more particularly, comprises one bulge structure. In another particular embodiment of the first aspect, the BS formed by, or consisting of, R1 and R5 base paired to each other, as defined in the first aspect of the invention comprises at least 1, at least 2, at least 3 at least 4 at least 5 at least 6 at least 7 at least 8, at least 9, at least 10, particularly at least 1 mismatches. In a particular embodiment of the first aspect, the BS formed by, or consisting of, R1 and R5 base paired to each other comprises 1, 2, 3, 4, 5, 6, 7, 8, 9., 10, particularly 2 mismatches.

In another embodiment, the stem structure formed by, or consisting of regions R1 and R5 base paired to each other in the stem loop structure formed by the RNA sequence S1 of the first aspect of the invention, comprises at least 1, at least 2, at least 3 at least 4 at least 5 at least 6 at least 7 at least 8, at least 9, at least 10, particularly at least 1 unpaired nucleotides. In a particular embodiment, the stem structure formed by, or consisting of regions R1 and R5 base paired to each other in stem loop structure formed by the RNA sequence S1 of the first aspect of the invention comprises 1, 2, 3, 4, 5, 6, 7, 8, 9., 10, particularly 2 unpaired nucleotides. In another particular embodiment, the stem structure formed by, or consisting of regions R1 and R5 base paired to each other in stem loop structure formed by the RNA sequence S1 of the first aspect of the invention, comprises at least one bulge, at least two bulges, at least 3 bulges, more particularly, at least one bulge structure. In another particular embodiment, the stem structure formed by, or consisting of regions R1 and R5 base paired to each other in stem loop structure formed by the RNA sequence S1 of the first aspect of the invention, comprises one bulge, two bulges, 3 bulges, more particularly, comprises one bulge structure. In another particular embodiment, the stem structure formed by, or consisting of regions R1 and R5 base paired to each other in stem loop structure formed by the RNA sequence S1 of the first aspect of the invention comprises at least 1, at least 2, at least 3 at least 4 at least 5 at least 6 at least 7 at least 8, at least 9, at least 10, particularly at least 1 mismatches, the stem structure formed by, or consisting of, regions R1 and R5 base paired to each other in stem loop structure formed by the RNA sequence S1 of the first aspect of the invention comprises 1, 2, 3, 4, 5, 6, 7, 8, 9., 10, particularly 2 mismatches. In another embodiment, the stem structure formed by, or consisting of regions R1 and R5 base paired to each other in stem loop structure formed by the RNA sequence S1 of the first aspect of the invention, comprises around 5%, 10%, 15%, 20%, 25%, 30%, 35%, 40%, 45%, 50%, 55%, 60%, particularly around 20% of unpaired nucleotides. The terms "bulge" and "mismatch" have been defined above.

Methods to determine the number of bulges, mismatches and unpaired nucleotides in an RNA structure are well-known by an expert in the field. Non-limiting examples of said methods include determining the secondary structure of an RNA molecule, with any of the methods provided above in the definition of "stem loop structure", and counting the number of bulges, mismatches, or unpaired nucleotides within said structure. To determine a percentage of unpaired nucleotides in a stem structure, the number of unpaired nucleotides in said stem structure is divided by the total number of nucleotides that form the stem structure and multiplying by 100.

In a particular embodiment, region R1 has a length of at least 4, at least 5, at least 6, at least 7, at least 8, at least 9, at least 10, at least 11, at least 12, at least 13, at least 14, at least 15, at least 16, at least 18, at least 19, at least 20, at least 22, at least 25, at least 27, at least 30, at least 40 at least 50, at least 75, or at least 100 nucleotides, particularly of at least 14 nucleotides, more particularly at least 18 nucleotides. In another particular embodiment, region R1 has a length of 4, 5, 6, 7, 8, 9, 10,. 11, 12, 13, 14, 15, 16, 17, 18, 19, 20, 22, 25, 27, 30, 40, 50, 75, or 100 nucleotides, particularly 14 nucleotides, more particularly of 18 nucleotides. In another particular embodiment, region R1 has a length of 4-30, 4-27, 4-25, 4-22, 4-20, 6-20, 9-19, or 14-18 nucleotides, particularly 14-18 nucleotides. In a particular embodiment, region R5 has a length of at least 4, at least 5, at least 6, at least 7, at least 8, at least 9, at least 10, at least 11, at least 12, at least 13, at least 14, at least 15, at least 16, at least 17, at least 18, at least 19, at least 20, at least 22, at least 25, at least 27, or at least 30, at least 40, at least 50, at least 75, at least 100 nucleotides particularly of at least 12 nucleotides, more particularly at least 15 nucleotides. In another particular embodiment, region R5 has a length of 4, 5, 6, 7, 8, 9, 10, 11, 12, 13, 14, 15, 16, 17, 18, 19, 20, 22, 25, 27, 30, 40, 50, 75, or 100 nucleotides, particularly of 12 nucleotides, more particularly of 15 nucleotides. In another particular embodiment, region R5 has a length of 4-30, 4-27, 4-25, 4-22, 4-20, 6-20, 9-19, 10-15, 12-18, or 12-15 nucleotides, particularly 12-15 nucleotides

In a particular embodiment, R1 and R5 have each one a length of 4-30, 4-27, 4-25, 4-22, 4-20, 6-20, 8-20, 10-20, 12-20, 14-18, 14-17, 15-16, particularly of 12-18 nucleotides. In another particular embodiment, the stem structure formed, or consisting of, region R1 and R5 base paired to each other as defined in the first aspect has a length of 4-30, 4-27, 4-25, 4-22, 4-20, 6-20, 8-20, 10-20, 12-20, 13-18, 14-18, 14-17, 15-16, particularly of 14-17 base paired nucleotides (bp). In a more particular embodiment, the stem structure formed by, or consisting of, region R1 and R5 base paired to each other, as defined in the first aspect has a length of 4, 6, 8, 10, 12, 13, 14, 15, 16, 17, 18, 19, 20, 22, 25, 27, or 30 bp, particularly 13 bp, more particular 14 bp, even more particularly 15 bp. In another particular embodiment, the stem structure formed, or consisting of, region R1 and R5 base paired to each other as defined in the first aspect has a length of at least 8, at least 10, at least 12, at least 14, at least 16, at least 18, at least 20, at least 22, at least 24, t least 26, at least 28 at least 30, at least 32, at least 33, at least 34, at least 35, at least 40 nucleotides, at least 44, at least 50, at least 54, at least 60 nucleotides, at least 70, at least 100, at least 150, or at least 200 nucleotides, particularly at least 26 nucleotides, more particularly at least 33 nucleotides. In another embodiment, the stem structure formed, or consisting of, region R1 and R5 base paired to each other as defined in the first aspect has a length of about 8, 9, 10, 12, 14, 16, 18, 20, 22, 24, 26, 28, 30, 32, 33, 34, 35, 40, 44, 50, 54, 60, 100, 150 or 200 nucleotides, particularly of about 26 nucleotides, more particularly of about 33 nucleotides. As well understood by a skilled person, the length of the stem structure formed, or consisting of, region R1 and R5 base paired to each other, as defined in the first aspect by a number of bp, or base paired nucleotides, corresponds to the number of base paired nucleotides, i.e. the number of pairs of nucleotides complementary to each other, in said R1-R5 duplex. As well understood by a skilled person, the length of the stem structure formed, or consisting of, region R1 and R5 base paired to each other, as defined in the first aspect by a number of nucleotides corresponds to the sum of the length of region R1 and of the length of region R5.

In a particular embodiment, R1 and R5 correspond to the strands of the BS structure of the miRNA foldback structures of a miRNA selected from the list consisting of: miR390a, miR172a, miR171a, miR167a, and miR166b, more particularly from the list consisting of AtMIRR390a, AtMIR172a, AtMIR171a, AtMIR167a and AtMlR166b, even more particularly from AtMIR390a. As well known by an expert in the field, the term "AtMIR" refers to a miRNA endogenously expressed in a plant from the species *Arabidopsis thaliana.* The nucleotides forming the BS of the just mentioned miRNAs are well known by an expert in the field. In another particular embodiment, the stem structure formed, or consisting of, region R1 and R5 base paired to each other as defined in the first aspect corresponds to the 4-30, 4-27, 4-25, 4-22, 4-20, 6-20, 8-20, 10-20, 12-20, 13-18, 14-18, 14-17, 15-16 bp, particularly to the 13-18 bp in length stem structure more proximal to the miRNA/miRNA* duplex in a miRNA foldback structure of a miRNA selected from the list consisting of: miR390a, miR172a, miR171a, and miR166b, particularly from a list consisting of AtMIR390a, AtMIIR172a, AtMIR171a, AtMIR167a and AtMlR166b. In a more particular embodiment, the stem structure formed, or consisting of, region R1 and R5 base paired to each other as defined in the first aspect corresponds to the stem structure of 13 bp, 14 bp, 15 bp, 16 bp, 17 bp, particularly the 15 bp, more proximal to the miRNA/miRNA* duplex in a miRNA foldback structure of a miRNA selected from the list consisting of: miR390a, miR172a, miR171a, and miR166b, particularly from a list consisting of AtMIR390a, AtMIR172a, AtMIR171a, AtMIR167a and AtMIR166b. In another particular embodiment, the stem structure formed, or consisting of, region R1 and R5 base paired to each other as defined in the first aspect corresponds to the stem structure region of 3 bp (8 nucleotides in length) comprised in the BS of the miR166b foldback structure more proximal to the miR166b miRNA/miRNA* duplex, particularly of the AtMIR166b foldback structure more proximal to the miR166b miRNA/miRNA* duplex.

The term "AtMIR390a", as used herein, refers to the miRNA encoded by gene MIR390a with Gene ID number 5007944 in the NCBI database (version 10 April 2023).

The term "AtMIR172a", as used herein, refers to the miRNA encoded by gene with Gene ID number 28718310 in the NCBI database (version 10 April 2023).

The term "AtMIR171a", as used herein, refers to the miRNA encoded by gene MIR171a with Gene ID number 28719395 in the NCBI database (version 10 April 2023).

The term "AtMIR167a", as used herein, refers to the miRNA encoded by gene MIR167a with Gene ID number 28719305 in the NCBI database (version 10 April 2023).

The term "AtMIR166b", as used herein, refers to the miRNA encoded by gene MIR166b with Gene ID number 3769765 in the NCBI database (version 10 April 2023).
In a particular embodiment, the BS of AtMIR390a is formed by, or consists of, SEQ ID NO. 20 (AGUAGAGAAGAAUCUGUA) and SEQ ID NO. 23 (UUGGCUCUUCUUACU).

In a particular embodiment, the BS of AtMIR172a is formed by or consists of SEQ ID NO. 57 (**Ggtcgttgttggctg**) and SEQ ID NO. 58 (**cggcaatcaacgact**). Thus, in a particular embodiment, region R1 consists of SEQ ID NO. 57 and region R5 consists of SEQ ID NO.58.

In another particular embodiment, the BS of AtMIR171a is formed by or consists of SEQ ID NO. 59 (**agcatgagagagtcccttt**) and SEQ ID NO. 60 (**tcagtactctctcgtct**). Thus, in a particular embodiment, region R1 consists of SEQ ID NO. 59 and region R5 consists of SEQ ID NO.60.

In another particular embodiment, the BS of AtMIR167a is formed by or consists of SEQ ID NO: 61 (**ggtgcaccggcatctga**) and SEQ ID NO. 62 (**cgttgactgtcgcaccc**). Thus, in a particular embodiment, region R1 consists of SEQ ID NO. 61 and region R5 consists of SEQ ID NO.62.

In another particular embodiment, the BS of AtMIR166b is formed by or consists of SEQ ID NO: 63 (gagggatttttcttttga) and SEQ ID NO. 64 (ccaattatcactccctc). Thus, in a particular embodiment, region R1 consists of SEQ ID NO. 63 and region R5 consists of SEQ ID NO. 64.

In another particular embodiment, the stem structure region of 3 bp comprised in the BS of the miR166b foldback structure more proximal to the miR166b miRNA/miRNA* duplex is formed by, or consists of, SEQ ID NO: 65 (ttga) and SEQ ID NO.66 (ccaa). Thus, in a particular embodiment, region R1 consists of SEQ ID NO. 65 and region R5 consists of SEQ ID NO.66.

In another particular embodiment, R1 and R5 correspond to the RNA sequence of the strands of a BS of miRNA foldback structure as defined in the first aspect of the invention, wherein the BS is an artificial BS of a miRNA foldback structure. The term "artificial BS" as used herein, refers to a BS structure of a miRNA foldback structure, wherein the BS is not found in nature, and is thus artificially designed, particularly, by means of computer programs. As well understood by a skilled person, the artificial BS as defined herein has the same characteristics of a BS as defined in any of the definitions and embodiments above, except that it is not found in nature. In a particular embodiment, the basal stem structure formed by, or consisting of, regions R1 and R5 base paired to each other, as defined in the first aspect of the invention, is an artificial BS of a miRNA foldback structure as defined in the first aspect of the invention, wherein the artificial BS has at least 60%, particularly at least 70%, more particularly at least 80% the activity of the BS of the miRNA stem loop structure of the AtMIR390a primary precursor. In another particular embodiment, the artificial BS substantially maintains or improves the activity of the BS of the miRNA stem loop structure of the AtMIR390a primary precursor.

In another particular embodiment of the first aspect, the stem structure formed by, or consisting of, regions R1 and R5 base paired to each other, as defined in the first aspect of the invention, has at least 60%, particularly at least 70%, more particularly at least 80% the activity of the BS of the miRNA stem loop structure of the AtMIR390a primary precursor. In another particular embodiment, the basal stem structure formed by, or consisting of, regions R1 and R5 base paired to each other, as defined in the first aspect of the invention, substantially maintains or improves the activity of the BS of the miRNA stem loop structure of the AtMIR390a primary precursor.

The term "substantially maintains the function of the BS of the miRNA stem loop structure of the AtMIR390a primary precursor" as used herein indicates that the corresponding structure (the artificial BS or the stem structure formed or consisting of regions R1 and R5 base paired to each other) has at least 80%, at least 85%, at least 90%, at least 95%, at least 99%, at least 100%, particularly at least 90%, of the activity of the BS of the miRNA stem loop structure of the AtMIR390a primary precursor. The term "improves the function of the BS of the miRNA stem loop structure of the AtMIR390a primary precursor", as used herein, indicates that the corresponding structure (the artificial BS or the stem structure formed or consisting of regions R1 and R5 base paired to each other) has at least 105%, at least 110%, at least 115%, at least 120%, at least 130%, at least 150%, at least 175%, at least 200%, particularly at least 110% the activity of the BS of the miRNA stem loop structure of the AtMIR390a primary precursor.

As well known by an expert in the field, the BS of the miRNA stem loop structure of the AtMIR390a primary precursor is required for the proper binding and activity of the DCL1 protein in the AtMIR390a primary precursor, and the subsequent release of the miRNA/miRNA* duplex. Methods to determine the activity of the BS in the miRNA stem loop structure of the AtMIR390a primary precursor include those disclosed in the examples bellow. Non-limiting examples of said methods comprise the agroinfiltration of the AtMIR390a primary precursor comprising a miRNA/miRNA* duplex of a miRNA of interest in a tissue of a plant, particularly in *Arabidopsis thaliana,* and comparing the level of the corresponding mature miRNAs accumulated in said tissue with that accumulated in another tissue of said plant agroinfiltrated with a negative control, wherein the negative control corresponds to the AtMIR390a primary precursor lacking the sequences of the BS. Determining the % of activity of the stem structure formed by or consisting of regions R1 and R5 base paired to each other, or of an artificial BS, can be performed by the same methods indicated just above, wherein the sequences of the AtMIR390a BS is substituted in the AtMIR390a primary precursor used in the previous method ,by the sequences of the stem structure formed by or consisting of R1 and R5 base paired to each other (the sequences of R1 and R5) or by the sequences of the corresponding artificial BS. Dividing the activity obtained for the stem structure formed by, or consisting of, regions R1 and R5 base paired to each other, or for the artificial BS, by that obtained for the AtMIR390a BS in the methods just described, allows determining the percentage of activity of the stem structure formed by or consisting of regions R1 and R5 base paired to each other, or of the artificial BS as compared to that of the AtMIR390a BS. Alternatively, or additionally, in the methods just described, the expression level of the target mRNA of the corresponding miRNA can be determined, instead of the miRNA levels in each case. Methods to determine the miRNA levels in a plant tissue are well known by an expert in the field and include those disclosed in the examples bellow. For instance, the miRNA levels can be determined by q-RT-PCR. Methods to determine the mRNA expression level of a target mRNA in a plant are also well known by an expert in the field and include those disclosed in the examples below. For instance, it can be determined by q-RT-PCR and/or western blot analysis of the expression level of the protein encoded by the target mRNA.

In a particular embodiment, region R1 comprises a sequence selected from the list consisting of SEQ ID NO. 1 (UCUGUA), SEQ ID NO. 18 (GAAUCUGUA), SEQ ID No. 19 (GAGAAGAAUCUGUA), SEQ ID NO. 20 (AGUAGAGAAGAAUCUGUA), or a variant thereof at least 75% identical to SEQ ID NO.1, SEQ ID NO. 18, SEQ ID NO. 19, or SEQ ID NO. 20, particularly comprises SEQ ID NO. 20 (AGUAGAGAAGAAUCUGUA), or a variant thereof at least 75% identical to SEQ ID NO. 20. In a particular embodiment, R1 comprises a sequence selected from the list consisting of SEQ ID NO. 1 (UCUGUA), SEQ ID NO. 18 (GAAUCUGUA), SEQ ID NO. 19 (GAGAAGAAUCUGUA), SEQ ID NO. 20 (AGUAGAGAAGAAUCUGUA), or a variant thereof at least 75%, at least 80%, at least 90%, at least 95%, identical to SEQ ID NO.1, SEQ ID NO. 18, SEQ ID NO. 19, or SEQ ID NO. 20, particularly comprises SEQ ID NO. 20 (AGUAGAGAAGAAUCUGUA), or a variant thereof at least 75%, at least 80%, at least 90%, at least 95%, identical to SEQ ID NO. 20. In another particular embodiment, R1 comprises SEQ ID NO. 1 (UCUGUA), or a variant thereof at least 75%, at least 80%, at least 90%, at least 95%, identical to SEQ ID NO. 1. In another particular embodiment, R1 comprises SEQ ID NO. 18 (GAAUCUGUA), or a variant thereof at least 75%, at least 80%, at least 90%, at least 95%, identical to SEQ ID NO.18. In another particular embodiment, R1 comprises SEQ ID NO. 19 (GAGAAGAAUCUGUA), or a variant thereof at least 75%, at least 80%, at least 90%, at least 95%, identical to SEQ ID NO.19.

In a particular embodiment, region R1 consists of a sequence selected from the list consisting of SEQ ID NO. 1 (UCUGUA), SEQ ID NO. 18 (GAAUCUGUA), SEQ ID No. 19 (GAGAAGAAUCUGUA), SEQ ID NO. 20 (AGUAGAGAAGAAUCUGUA), or a variant thereof at least 75% identical to SEQ ID NO.1, SEQ ID NO. 18, SEQ ID NO. 19, or SEQ ID NO. 20, particularly consists of SEQ ID NO. 20 (AGUAGAGAAGAAUCUGUA), or a variant thereof at least 75% identical to SEQ ID NO. 20. In a particular embodiment, R1 consists of a sequence selected from the list consisting of SEQ ID NO. 1 (UCUGUA), SEQ ID NO. 18 (GAAUCUGUA), SEQ ID NO. 19 (GAGAAGAAUCUGUA), SEQ ID NO. 20 (AGUAGAGAAGAAUCUGUA), or a variant thereof at least 75%, at least 80%, at least 90%, at least 95%, identical to SEQ ID NO.1, SEQ ID NO. 18, SEQ ID NO. 19, or SEQ ID NO. 20, particularly consists of SEQ ID NO. 20 (AGUAGAGAAGAAUCUGUA), or a variant thereof at least 75%, at least 80%, at least 90%, at least 95%, identical to SEQ ID NO. 20.

In another particular embodiment, R1 consists of SEQ ID NO. 1 (UCUGUA), or a variant thereof at least 75%, at least 80%, at least 90%, at least 95%, identical to SEQ ID NO.1. In another particular embodiment, R1 consists of SEQ ID NO. 18 (GAAUCUGUA), or a variant thereof at least 75%, at least 80%, at least 90%, at least 95%, identical to SEQ ID NO.18. In another particular embodiment, R1 consists of SEQ ID NO. 19 (GAGAAGAAUCUGUA), or a variant thereof at least 75%, at least 80%, at least 90%, at least 95%, identical to SEQ ID NO.19.

In a particular embodiment, region R1 consists of SEQ ID NO. 20 (AGUAGAGAAGAAUCUGUA) or a variant thereof at least 75%, at least 80%, at least 90%, at least 95%, identical to SEQ ID NO. 20, particularly consists of SEQ ID NO. 20.

As well understood by a skilled person, the variants of SEQ ID NO. 1, SEQ ID NO. 18, SEQ ID NO. 19, and SEQ ID NO. 20 as defined above are suitable to form the stem structure, particularly the BS, by base pairing of region R1 and R5 as defined in any of the embodiments above. In a more particular embodiment, the variants of SEQ ID NO. 1, SEQ ID NO. 18, SEQ ID NO. 19, and SEQ ID NO. 20 as defined above are suitable to form the stem structure, particularly the BS, by base pairing of region R1 and R5 as defined in any of the embodiments above, wherein said stem structure or BS has at least 60%, particularly at least 70%, more particularly at least 80% the activity of the BS of the miRNA stem loop structure of the AtMIR390a primary precursor. In another particular embodiment, the variants of SEQ ID NO. 1, SEQ ID NO. 18, SEQ ID NO. 19, and SEQ ID NO. 20 as defined above are suitable to form the stem structure, particularly the BS, by base pairing of region R1 and R5 as defined in any of the embodiments above, wherein said stem structure or BS substantially maintains or improves the activity of the BS of the miRNA stem loop structure of the AtMIR390a primary precursor. Methods to determine if two RNA molecules form a stem structure have been provided above in the definition of stem loop structure and also apply for determining if two RNA molecules or RNA sequences form a stem structure. Methods to determine the number of bulges, mismatches and unpaired nucleotides within a stem structure have also been provided above and apply to the R1-R5 region or stem structure formed with the variants as defined herein. Methods to determine the activity of the stem structure formed by, or consisting of, regions R1 and R5 bas paired to each other as compared to the BS of the AtMIR390a foldback structure have been provided above and also apply to the stem structure formed by the variants as defined herein.

In another embodiment, region R5 comprises SEQ ID NO. 2 (UUGG), SEQ ID NO. 21 (UUGGCUC), SEQ ID NO. 22 (UUGGCUCUUCUU), SEQ ID NO. 23 (UUGGCUCUUCUUACU), or a variant thereof at least 75% identical to SEQ ID NO. 2, SEQ ID NO. 21, SEQ ID NO. 22, or SEQ ID NO. 23, particularly comprises SEQ ID NO. 23 (UUGGCUCUUCUUACU) or a variant thereof at least 75% identical to SEQ ID NO. 23. In a particular embodiment, region R5 comprises SEQ ID NO. 2 (UUGG), SEQ ID NO. 21 (UUGGCUC), SEQ ID NO. 22 (UUGGCUCUUCUU), SEQ ID NO. 23 (UUGGCUCUUCUUACU), or a variant thereof at least 75%, at least 80%, at least 90%, at least 95%, identical to SEQ ID NO. 2, SEQ ID NO. 21, SEQ ID NO. 22, or SEQ ID NO. 23, particularly comprises SEQ ID NO. 23 (UUGGCUCUUCUUACU) or a variant thereof at least at least 75%, at least 80%, at least 90%, at least 95%, identical to SEQ ID NO. 23. In another particular embodiment, region R5 comprises SEQ ID NO. 2 (UUGG), or a variant thereof at least 75%, at least 80%, at least 90%, at least 95%, identical to SEQ ID NO. 2. In another particular embodiment, region R5 comprises SEQ ID NO. 21 (UUGGCUC), or a variant thereof at least 75%, at least 80%, at least 90%, at least 95%, identical to SEQ ID NO. 21 (UUGGCUC). In another particular embodiment, region R5 comprises SEQ ID NO. 22 (UUGGCUCUUCUU), or a variant thereof at least 75%, at least 80%, at least 90%, at least 95%, identical to SEQ ID NO. 22 (UUGGCUCUUCUU).

In another embodiment, region R5 consists of SEQ ID NO. 2 (UUGG), SEQ ID NO. 21 (UUGGCUC), SEQ ID NO. 22 (UUGGCUCUUCUU), SEQ ID NO. 23 (UUGGCUCUUCUUACU), or a variant thereof at least 75% identical to SEQ ID NO. 2, SEQ ID NO. 21, SEQ ID NO. 22, or SEQ ID NO. 23, particularly consists of SEQ ID NO. 23 (UUGGCUCUUCUUACU) or a variant thereof at least 75% identical to SEQ ID NO. 23. In a particular embodiment, region R5 consists of SEQ ID NO. 2 (UUGG), SEQ ID NO. 21 (UUGGCUC), SEQ ID NO. 22 (UUGGCUCUUCUU), SEQ ID NO. 23 (UUGGCUCUUCUUACU), or a variant thereof at least 75%, at least 80%, at least 90%, at least 95%, identical to SEQ ID NO. 2, SEQ ID NO. 21, SEQ ID NO. 22, or SEQ ID NO. 23, particularly consists of SEQ ID NO. 23 (UUGGCUCUUCUUACU) or a variant thereof at least at least 75%, at least 80%, at least 90%, at least 95%, identical to SEQ ID NO. 23. In another particular embodiment, region R5 consists of SEQ ID NO. 2 (UUGG), or a variant thereof at least 75%, at least 80%, at least 90%, at least 95%, identical to SEQ ID NO. 2. In another particular embodiment, region R5 consists of SEQ ID NO. 21 (UUGGCUC), or a variant thereof at least 75%, at least 80%, at least 90%, at least 95%, identical to SEQ ID NO. 21 (UUGGCUC). In another particular embodiment, region R5 consists of SEQ ID NO. 22 (UUGGCUCUUCUU), or a variant thereof at least 75%, at least 80%, at least 90%, at least 95%, identical to SEQ ID NO. 22 (UUGGCUCUUCUU).

In a particular embodiment, region R5 consists of SEQ ID NO. 23 (UUGGCUCUUCUUACU), or a variant thereof at least 75%, at least 80%, at least 90%, at least 95% identical to SEQ ID NO. 23, particularly consists of SEQ ID NO. 23.

As well understood by a skilled person, the variants of SEQ ID NO. 2, SEQ ID NO. 21, SEQ ID NO. 22, and SEQ ID NO. 23 as defined above form the stem structure, particularly the BS, by base pairing of region R1 and R5 as defined in any of the embodiments above for region R1-R5. In a more particular embodiment, the variants of SEQ ID NO. 2, SEQ ID NO. 21, SEQ ID NO. 22, and SEQ ID NO. 23 as defined above form the stem structure, particularly the BS, by base pairing of region R1 and R5 as defined in any of the embodiments above, wherein said stem structure or BS has at least 60%, particularly at least 70%, more particularly at least 80% the activity of the BS of the miRNA stem loop structure of the AtMIR390a primary precursor (i.e. the AtMIR390a BS) In another particular embodiment, the variants of SEQ ID NO. 2, SEQ ID NO. 21, SEQ ID NO. 22, and SEQ ID NO. 23 as defined herein form the stem structure, particularly the BS, by base pairing of region R1 and R5 as defined in any of the embodiments above, wherein said stem structure or BS substantially maintains or improves the activity of the BS of the miRNA stem loop structure of the AtMIR390a primary precursor. Methods to determine if two RNA molecules form a stem structure have been provided above in the definition of stem loop structure and also apply for determining if two RNA molecules or RNA sequences form a stem structure. Methods to determine the number of bulges, mismatches and unpaired nucleotides within a stem structure have also been provided above and apply to the R1-R5 region or stem structure formed with the variants as defined herein. Methods to determine the activity of the stem structure formed by, or consisting of, regions R1 and R5 bas paired to each other as compared to the BS of the AtMIR390a foldback structure have been provided above and also apply to the stem structure formed by the variants as defined herein.

In another particular embodiment, R1 comprises SEQ ID NO. 1 (UCUGUA), or a variant thereof at least 80%, identical to SEQ ID NO.1 and region R5 comprises SEQ ID NO. 2 (UUGG), or a variant thereof at least 75%, identical to SEQ ID NO. 2. In another particular embodiment, R1 comprises SEQ ID NO. 18 (GAAUCUGUA), or a variant thereof at least 75%, at least 80%, at least 90%, or at least 95%, particularly at least 75% identical to SEQ ID NO.18 and region R5 comprises SEQ ID NO. 21 (UUGGCUC), or a variant thereof at least 75%, at least 80%, at least 90%, or at least 95% identical, particularly at least 80% identical to SEQ ID NO. 21 (UUGGCUC). In another particular embodiment, R1 comprises SEQ ID NO. 19 (GAGAAGAAUCUGUA), or a variant thereof at least 80%, at least 90%, or at least 95% identical, particularly at least 80% identical to SEQ ID NO.19 and region R5 comprises SEQ ID NO. 22 (UUGGCUCUUCUU), or a variant thereof at least 80%, at least 90%, or at least 95% identical, particularly at least 80% identical to SEQ ID NO. 22 (UUGGCUCUUCUU). In another particular embodiment, R1 comprises SEQ ID NO. 20 (AGUAGAGAAGAAUCUGUA), or a variant thereof at least 80%, at least 80%, at least 90%, or at least 95%, particularly at least 80% identical to SEQ ID NO.20 and region R5 comprises SEQ ID NO. 23 (UUGGCUCUUCUUACU), or a variant thereof at least 80%, at least 90%, or at least 95% identical, particularly at least 80% identical to SEQ ID NO. 23.

In another particular embodiment, R1 consists of SEQ ID NO. 1 (UCUGUA), or a variant thereof at least 80%, identical to SEQ ID NO.1 and region R5 consists of SEQ ID NO. 2 (UUGG), or a variant thereof at least 75% identical to SEQ ID NO. 2. In another particular embodiment R1 consists of SEQ ID NO. 18 (GAAUCUGUA), or a variant thereof at least 75%, at least 80%, at least 90%, or at least 95%, particularly at least 80% identical to SEQ ID NO.18 and region R5 consists of SEQ ID NO. 21 (UUGGCUC), or a variant thereof at least 75%, at least 80%, at least 90%, or at least 95%, particularly at least 80% identical to SEQ ID NO. 21 (UUGGCUC). In another particular embodiment, R1 consists of SEQ ID NO. 19 (GAGAAGAAUCUGUA), or a variant thereof at least 80%, at least 90%, or at least 95%, particularly at least 80% identical to SEQ ID NO.19 and region R5 consists of SEQ ID NO. 22 (UUGGCUCUUCUU), or a variant thereof at least 80%, at least 90%, or at least 95%, particularly at least 80% identical to SEQ ID NO. 22 (UUGGCUCUUCUU). In another particular embodiment, R1 consists of SEQ ID NO. 20 (AGUAGAGAAGAAUCUGUA), or a variant thereof at least 80%, at least 90%, or at least 95%, particularly at least 80% identical to SEQ ID NO.20 and region R5 consists of SEQ ID NO. 23 (UUGGCUCUUCUUACU), or a variant thereof at least 80%, at least 90%, or at least 95%, particularly at least 80% identical to SEQ ID NO. 23.

In a particular embodiment, R1 consists of SEQ ID NO. 20 (AGUAGAGAAGAAUCUGUA), and region R5 consists of SEQ ID NO. 23 (UUGGCUCUUCUUACU). As well understood by a skilled person, the variants of SEQ ID NO. 1, SEQ ID NO. 18, SEQ ID NO. 19, and SEQ ID NO. 20 and the variants of SEQ ID NO. 2, SEQ ID NO. 21, SEQ ID NO. 22, and SEQ ID NO. 23 as defined any of the embodiments above form the stem structure, particularly the BS, by base pairing of region R1 and R5 as defined in any of the embodiments above. In a more particular embodiment, the variants of SEQ ID NO. 1, SEQ ID NO. 18, SEQ ID NO. 19, and SEQ ID NO. 20 and the corresponding variants of SEQ ID NO. 2, SEQ ID NO. 21, SEQ ID NO. 22, and SEQ ID NO. 23 as defined any of the embodiments above form the stem structure, particularly the BS, by base pairing of region R1 and R5 as defined in any of the embodiments above, wherein said stem structure or BS has at least 60%, particularly at least 70%, more particularly at least 80% the activity of the BS in the miRNA stem loop structure of the AtMIR390a precursor (i.e. the AtMIR390a BS). In another particular embodiment, the variants of SEQ ID NO. 1, SEQ ID NO. 18, SEQ ID NO. 19, and SEQ ID NO. 20 and the corresponding variants of SEQ ID NO. 2, SEQ ID NO. 21, SEQ ID NO. 22, and SEQ ID NO. 23 as defined above form the stem structure, particularly the BS, by base pairing of region R1 and R5 as defined in any of the embodiments above, wherein said stem structure or BS substantially maintains or improves the activity of the BS in the miRNA stem loop structure of the AtMIR390a primary precursor.

### I. 2- Regions R2 and R4 of the RNA sequence S1

The region of the stem loop formed by the RNA sequence S1 consisting of regions R2 and R4 base paired to each other is herein also referred to as the "R2-R4 region". As well understood by a skilled person, the R2-R4 region consists of an RNA stem structure as defined herein.

As indicated in the first aspect of the invention, R2 consists of the RNA sequence of a miRNA.

The term "miRNA sequence" or "sequence of a miRNA", as used herein, refers to the sequence of any of the strands of a miRNA/miRNA* duplex, as described above. Thus, in a particular embodiment, region R2 consists of the RNA sequence of the guide or of the passenger strand of a miRNA. In a particular embodiment, R2 consists of the RNA sequence of the guide strand of a miRNA.

As indicated in the first aspect of the invention, R4 consists of an RNA sequence that base pairs with the RNA sequence of R2 to form a miRNA/miRNA* duplex.

The expression "base pairs with the RNA sequence of R2 to form a miRNA/miRNA* duplex" as used herein, refers to the fact that R4 consists of the complementary sequence of R2, in a miRNA/miRNA* duplex as defined above. Thus, base pairing between R2 and R4 follows the base pairing rules of a miRNA/miRNA* duplex, such as those indicated in the definition of miRNA/miRNA* duplex indicated above. As well understood by a skilled person, once the sequence of the guide strand of miRNA is defined, the sequence of the corresponding passenger strand is easily defined, and vis versa, following the miRNA/miRNA* duplex base pairing rules, such as those indicated in the definition of miRNA/miRNA* duplex above. R4 is thus the passenger strand in the miRNA/miRNA* duplex when R2 is the guide strand, and R4 is the guide strand in the miRNA/miRNA* duplex when R2 is the passenger strand. Thus, in a particular embodiment, region R2 consists of the RNA sequence of the guide or of the passenger strand of a miRNA and R4 consists of the RNA sequence of the passenger strand of the said miRNA (i.e. of the same miRNA as R2) when R2 is the guide strand, or of the guide strand of the miRNA when R2 is the passenger strand. In a particular embodiment, region R2 consists of the RNA sequence of the passenger strand of a miRNA and R4 consists of the RNA sequence of the guide strand of the said miRNA. In a more particular embodiment, region R2 consists of the RNA sequence of the guide strand of a miRNA and R4 consists of the RNA sequence of the passenger strand of the said miRNA. In another particular embodiment, R2 consists of the RNA sequence of the guide or passenger strand of a miRNA/miRNA* duplex, and R4 consists of the opposite strand of the same miRNA/miRNA* duplex. In another particular embodiment, R2 consists of the RNA sequence of the passenger strand of a miRNA/miRNA* duplex, and R4 consists of the guide strand of the same miRNA/miRNA* duplex In a more particular embodiment, R2 consists of the RNA sequence of the guide strand of a miRNA/miRNA* duplex, and R4 consists of the passenger strand of the same miRNA/miRNA* duplex. As well understood by a skilled person, the miRNA of the first aspect of the invention, or the miRNA of the invention, as use herein, refers to the miRNA having a guide strand equivalent to that of the miRNA/miRNA* duplex formed by the base pairing of regions R2 and R4 as defined herein. In a particular embodiment, the miRNA of the invention is the guide strand that of the miRNA/miRNA* duplex formed by the base pairing of regions R2 and R4 as defined herein. Additionally, as used herein "the miRNA/miRNA* duplex of the invention", or "the miRNA/miRNA* duplex as defined in the first aspect of the invention" refers to the miRNA/miRNA* duplex formed by, or consisting of, the base pairing of region R2 and R4 as defined in the first aspect of the invention. In an embodiment, the miRNA of the invention has a length of 20, 21, 22, 23, or 24 nucleotides, particularly of 22 nucleotides, more particularly of 21 nucleotides. In another embodiment, the guide strand and the passenger strand of the miRNA of the invention have the same length. Thus, in an embodiment, R2 and R4 have a length of 20, 21, 22, 23, or 24 nucleotides, particularly of 22 nucleotides, more particularly of 21 nucleotides.

In a particular embodiment, the stem structure formed by the base pairing of regions R2 and R4 comprises a two nucleotides overhang at the 3' end of region R2, and a two nucleotides overhang at the 3' end of the R4 region. The term "two-nucleotide overhang at the 3' end" of an RNA region or RNA sequence, as used herein, refers to the term well-known by an expert in the field. Particularly, it indicates that two single stranded nucleotides stand out of an RNA duplex at the 3' end of a strand of the duplex. As well understood by a skilled person, the two nucleotides that overhang at the 3' end of R2 and/or at the 3' end of R4 can base pair with nucleotides of other regions of the RNA sequence S1 in the stem loop structure formed by S1.

In an embodiment, the 3' end nucleotide of region R4 is complementary, as defined herein, to the second to last nucleotide at the 3' end of region R1. The term "3' end nucleotide" of an RNA region, as used herein, refers to the nucleotide in the RNA sequence of said RNA region that is at the 3'end of the sequence. The term "second to last nucleotide at the 3' end" of an RNA region, as used herein, refers to the second nucleotide in the RNA sequence of said RNA region in the 3'- to 5'-end direction. In a particular embodiment, the 3' end nucleotide of region R4 is an adenosine nucleotide, and the second to last nucleotide at the 3' end of region R1 is an uracil nucleotide.

In another embodiment, the second to last nucleotide at the 3' end of region R4 is not complementary, as defined herein, to the last nucleotide at 3' end of region R1. Thus, in a particular embodiment, the last nucleotide of the R1 region is selected from the list consisting of an A, U, C and G ribonucleotide, and the second to last nucleotide at the 3' end of region R4 is a nucleotide other than those indicated in the definition of "is complementary" to base pair with the selected nucleotide (i.e. the last nucleotide of the R1 region), following a Watson-Crick base pairing base pairing or a wobble base pairing. Thus, in a particular embodiment, the 3'end nucleotide of the R1 region is selected from the list consisting of an A, U, C and G ribonucleotide, and:
when the 3'end nucleotide of the R1 region is an A ribonucleotide, the second to last nucleotide at the 3' end of region R4 is selected from the list consisting of ribonucleotides A, C and G,
- when the 3'end nucleotide of the R1 region is an U ribonucleotide, the second to last nucleotide at the 3' end of region R4 is selected from the list consisting of ribonucleotides U, C and G, more particularly from the list consisting of ribonucleotides U and C.
- when the 3'end nucleotide of the R1 region is a C ribonucleotide, the second to last nucleotide at the 3' end of region R4 is selected from the list consisting of ribonucleotides A, U and C,
- when the 3'end nucleotide of the R1 region is a G ribonucleotide, the second to last nucleotide at the 3' end of region R4 is selected from the list consisting of ribonucleotides A, U and G, particularly is selected from the list consisting of ribonucleotides A and G.

In a particular embodiment, the second to last nucleotide at the 3' end of region R4 is a C ribonucleotide and the last nucleotide at 3' end of region R1 is an A ribonucleotide.

In another particular embodiment, the second to last nucleotide at the 3' end of region R4 is complementary to, as defined herein, to the last nucleotide at the 3' end of region R1. Thus, in a particular embodiment, the last nucleotide at the 3'-end of R1 region is selected from the list consisting of ribonucleotides A, U, C and G , and the second to last nucleotide at the 3' end of region R4 is a nucleotide indicated in the definition of "is complementary" to base pair with the selected nucleotide (i.e. the last nucleotide of the R1 region), following a Watson-Crick base pairing or a wobble base pairing. Thus, in a particular embodiment, the last nucleotide at the 3'end of R1 region is selected from the list consisting of an A, U, C and G ribonucleotide, and:
when the last nucleotide at the 3'end of the R1 region is an A ribonucleotide, the second to last nucleotide at the 3' end of region R4 is an U ribonucleotide,
- when the last nucleotide at the 3'end of the R1 region is an U ribonucleotide, the second to last nucleotide at the 3' end of region R4 is an A ribonucleotide,
- when the last nucleotide at the 3'end of the R1 region is a C ribonucleotide, the second to last nucleotide at the 3' end of region R4 is a G ribonucleotide,
- when the last nucleotide at the 3'end of the R1 region is a G ribonucleotide, the second to last nucleotide at the 3' end of region R4 is selected from the list consisting of ribonucleotides C and U particularly is a C ribonucleotide.

In a particular embodiment, the second to last nucleotide at the 3' end of region R4 is a C nucleotide and the last nucleotide at 3' end of region R1 is a G nucleotide.

In a more particular embodiment, the last two nucleotides at the 3' end of region R4 form the RNA sequence 5'-CA-3' and the last two nucleotides at the 3' end of region R1 form the RNA sequence 5'-UA-3' or the sequence 5'-UG-3'. In a particular embodiment, the last two nucleotides at the 3'end of region R4 form the RNA sequence 5'-CA-3', and the last two nucleotides at the 3'end of region R1 form the RNA sequence 5'-UA-3'. In another particular embodiment, the last two nucleotides at the 3'end of region R4 form the RNA sequence 5'-CA-3' and the last two nucleotides at the 3'end of region R1 form the RNA sequence 5'-UG-3'.

In another particular embodiment, the nucleotide at the 5'-end of the guide strand of the miRNA/miRNA* duplex as defined in the first aspect is an U ribonucleotide, particularly, the nucleotide at the 5'-end of the guide strand of the miRNA/miRNA* duplex as defined in the first aspect is an U ribonucleotide and the nucleotide at position 19 in the 5'- to 3'-end direction in the passenger strand is an A ribonucleotide. In a particular embodiment, the nucleotide at the 5'-end of region R2 is an uracil ribonucleotide, particularly, the nucleotide at the 5'-end of region R2 is an U ribonucleotide and the nucleotide at position 19 in the 5'- to 3'-end direction of region R4 is an A ribonucleotide

In another particular embodiment, the nucleotide at position 19 in the 5'- to 3'-end direction of the guide strand of the miRNA/miRNA* duplex as defined in the first aspect is a C ribonucleotide and the nucleotide at the 5' end of the passenger strand of the miRNA/miRNA* duplex as defined in the first aspect is a G ribonucleotide. In another particular embodiment, the nucleotide at position 19 in the 5'- to 3'-end direction of region R2 is a C ribonucleotide and the nucleotide at the 5'end of region R4 is a G ribonucleotide.

In another embodiment, nucleotides at positions 1-19 of the guide strand of the miRNA/miRNA duplex of the invention in a 5' to 3'-end direction, perfectly base pair with nucleotides at positions 1-19 of the passenger strand in the miRNA/miRNA* duplex of the invention. In another particular embodiment, nucleotides at positions 1-19 of region R2 in a 5' to 3'-end direction, perfectly base pair with nucleotides at positions 1-19 of region R4 in a 5'- to 3'-end direction.

In another particular embodiment, the nucleotide at position 11 in the 5'- to 3'-end direction of the guide strand of the miRNA/miRNA* duplex as defined in the first aspect is not complementary to the nucleotide in position 9 in the 5'- to 3'-end direction of the passenger strand of the miRNA/miRNA* duplex as defined in the first aspect. In another particular embodiment, the nucleotide at position 11 in the 5'- to 3'-end direction of region R2 is not complementary to the nucleotide in position 9 in the 5'- to 3'-end direction of region R4. In a particular embodiment, the nucleotide at position 11 in the 5'- to 3'-end direction of the guide strand of the miRNA/miRNA* duplex as defined in the first aspect or of the R2 region is selected from the list consisting of G, C, A or U nucleotide, and:
- when the selected nucleotide is an A ribonucleotide, the nucleotide at position 9 in the 5'- to 3'-end direction of the passenger strand of the miRNA/miRNA* duplex as defined in the first aspect or of region R4 is selected from the list consisting of ribonucleotides A, C and G,
- when the selected nucleotide is an U ribonucleotide, the nucleotide at position 9 in the 5'- to 3'-end direction of the passenger strand of the miRNA/miRNA* duplex as defined in the first aspect or of region R4 is selected from the list consisting of ribonucleotides U, C and G, more particularly from the list consisting of ribonucleotides U and C.
- when the selected nucleotide is a C ribonucleotide, the nucleotide at position 9 in the 5'- to 3'-end direction of the passenger strand of the miRNA/miRNA* duplex as defined in the first aspect or of region R4 is selected from the list consisting of ribonucleotides A, U and C,
- when the selected nucleotide is a G ribonucleotide, the nucleotide at position 9 in the 5'- to 3'-end direction of the passenger strand of the miRNA/miRNA* duplex as defined in the first aspect or of region R4 is selected from the list consisting of ribonucleotides A, U and G, particularly is selected from the list consisting of ribonucleotides A and G.

In another particular embodiment, the miRNA/miRNA* duplex as defined in the first aspect comprises a mismatch at position 11 of the guide strand in the 5'- to 3'-end direction. Particularly, the miRNA/miRNA* duplex as defined in the first aspect comprises a mismatch at position 11 of the guide strand in the 5'- to 3'-end direction., formed by nucleotides G and A, each one in a strand of the duplex, or C and U nucleotides, each one in a strand of the duplex. In a particular embodiment, the stem structure formed by, or consisting of regions R2 and R4 base paired to each other, comprises a mismatch at position 11 of e region R2 in the 5'- to 3'-end direction. In a particular embodiment, the stem structure formed by, or consisting of regions R2 and R4 base paired to each other, comprises a mismatch at position 11 of e region R2 in the 5'- to 3'-end direction formed by nucleotides G and A, each one in a strand of the stem structure, or C and U nucleotides, each one in a strand of the stem structure.

In a particular embodiment, the miRNA/miRNA* duplex as defined in the first aspect of the invention comprises at least 1, at least 2, at least 3, at least 4, at least 5, at least 6, particularly at least 1 unpaired nucleotide within the duplex structure. In another particular embodiment, the miRNA/miRNA* duplex as defined in the first aspect of the invention comprises 1, 2, 3, 4, 5 or 6 unpaired nucleotides, particularly 1 unpaired nucleotide within the duplex structure. In a particular embodiment, the miRNA/miRNA* duplex as defined in the first aspect of the invention comprises at least 1, at least 2, at least 3, at least 4, at least 5, at least 6, particularly at least 1 mismatch. In another particular embodiment, comprises 1, 2, 3, 4, 5 or 6 mismatches, particularly 1 mismatch. In another particular embodiment, the miRNA/miRNA* duplex as defined in the first aspect of the invention comprises a bulge structure.

In a particular embodiment, the stem structure formed by or consisting of regions R2 and R4 base paired to each other, comprises at least 1, at least 2, at least 3, at least 4, at least 5, at least 6, particularly at least 1 unpaired nucleotide within the stem structure. In another particular embodiment, the stem structure formed by or consisting of regions R2 and R4 base paired to each other comprises 1, 2, 3, 4, 5 or 6 unpaired nucleotides, particularly 1 unpaired nucleotide within the stem structure. In a particular embodiment, the stem structure formed by or consisting of regions R2 and R4 base paired to each other comprises at least 1, at least 2, at least 3, at least 4, at least 5, at least 6, particularly at least 1 mismatch. In another particular embodiment, the stem structure formed by or consisting of regions R2 and R4 base paired to each other comprises 1, 2, 3, 4, 5 or 6 mismatches, particularly 1 mismatch. In another particular embodiment, the stem structure formed by or consisting of regions R2 and R4 base paired to each other comprises a bulge structure.

The term "amiRNA", or "Artificial miRNA", as used herein refers to a miRNA designed (generally computationally designed) for redirecting the endogenous miRNA biogenesis and silencing machineries to selectively suppress specific target RNAs with high efficacy and specificity (with ideally no predicted off-targets). Thus, as well understood by a skilled person, an amiRNA is not necessarily found in nature, or endogenously expressed in the plant in which the amiRNA is expressed or designed to be expressed. However, it can coincide with miRNA endogenously expressed in the plant in which the amiRNA is expressed or designed to be expressed. Methods to design amiRNAs are commonly by an expert in the field and include the use of the P-SAMS script (https://github.com/carringtonlab/p-sams) as disclosed in Fahlgren,N , et al., (2016) P-SAMS: a web site for plant artificial microRNA and synthetic trans-acting small interfering RNA design. Bioinformatics, 32, 157-8.

In a particular embodiment, the miRNA referred in the first aspect of the invention is a miRNA endogenously expressed in a plant cell, particularly in the plant comprising the viral vector of the invention as defined herein. In a particular embodiment, the miRNA referred in the first aspect of the invention is an amiRNA. In a more particular embodiment, the term "miRNA" in each embodiment of the present invention is replaced by the term "amiRNA" and the term "miRNA/miRNA*" in each embodiment of the present invention is replaced by "amiRNA/amiRNA*". Thus, in a particular embodiment of the first aspect, R2 consists of the RNA sequence of an amiRNA, and R4 consists of an RNA sequence that base pairs with the RNA sequence of R2 to form an amiRNA/amiRNA* duplex.

In a particular embodiment, region R2 has the sequence of a miRNA guide as defined in section I-4. In a particular embodiment, region R2 has a sequence selected from the miRNA sequences indicated in section I-4 below. Methods to design the passenger strand base on a guide strand have been provided above, and include the base pairing rules specified in the examples below (see Materials and Methods section). Thus, in a particular embodiment, region R4 has the sequence of the miRNA passenger strand corresponding to the sequence of the miRNA guide strand defined in section I-4 below, applying the miRNA/miRNA* duplex base pairing rules, such as those indicated in the definition of miRNA/miRNA* duplex above, particularly those described in the examples below.

### I. 3- Region R3 of the RNA sequence S1

As indicated in the first aspect of the invention, region R3 of the RNA sequence S1 comprises the loop structure of the stem loop structure formed by the RNA sequence S1.

In a particular embodiment, the loop structure of the stem loop structure formed by the RNA sequence S1 has a length of at least 4, at least 5, at least 6, at least 7, at least 8, at least 9, at least 10 nucleotides, particularly at least 4 nucleotides. In another particular embodiment, the length of the loop structure of the stem loop structure formed by the RNA sequence S1 has a length of 4, 5, 6, 7, 8, 9, 10 nucleotides, particularly of 8 nucleotides.

The term "loop" has been defined above in the definition of "stem loop structure". Methods to determine if a sequence can form a loop structure in a stem loop include the methods provided above to determine the secondary structure of an RNA sequence in the definition of "stem loop structure".

In a particular embodiment, R3 comprises a sequence selected from the list consisting of SEQ ID NO. 3 (AAAUCAAA), SEQ ID NO. 4 (GAAAUCAAA), SEQ ID NO. 5 (CGAAAUCAAACU), SEQ ID NO. 6 (UCGAAAUCAAACUA), SEQ ID NO. 7 (AUUC), SEQ ID NO. 8 (CAUUCG), SEQ ID NO. 9 (ACAUUCGU), SEQ ID NO. 10 (UCACAUUCGUUA), SEQ ID NO. 11 (AUCACAUUCGUUAU), SEQ ID NO. 12 ( GAUCACAUUCGUUAUC), SEQ ID NO. 13 (UGAUCACAUUCGUUAUCUA), SEQ ID NO. 14 (AUGAUCACAUUCGUUAUCUAU), SEQ ID NO. 15 (GAUGAUCACAUUCGUUAUCUAUU), SEQ ID NO. 16 (AUGAUGAUCACAUUCGUUAUCUAUUUUUU) and SEQ ID NO. 17 (UCGAUUCCUA). In a more part particularly. Region R3 comprises SEQ ID NO. 5 (CGAAAUCAAACU).

In another particular embodiment, region R3 comprises at the 3'end, two nucleotides X₁ and X₂ that form an RNA sequence 5'-X₁X₂-3' that is the reverse complement of the RNA sequence 5'-Y₁Y₂-3' formed by the last two nucleotides Y₁ and Y₂ at the 3' end of region R2 sequence. Nucleotides Y₁ and Y₂ in the RNA sequence 5'-Y₁Y₂-3' just defined, is each one independently selected from the list consisting of a G, C A and a U ribonucleotide. As well understood by a skilled person, the selection of nucleotides Y₁ and Y₂ directly results from the selection of the miRNA of the invention, i.e. the miRNA/miRNA* duplex as defined in the first aspect of the invention. Methods to select the miRNA of the invention to silence a gene of interest, have been provided above in the definition of the term "amiRNA". The reverse complement sequence of another sequence is well known by an expert in the field. As well understood by a skilled person, nucleotide X₁ as defined herein, is complementary to nucleotide Y₂ as defined herein, and nucleotide X₂ as defined herein, is complementary to X₂ as defined herein. Complementary nucleotides have been described above in the definition of "base pairing". In a particular embodiment, complementarity between X₁ and Y₂ and between X₂ and Y₁ comprises Watson-Crick base pairing (A-U and C-G ribonucleotide base pairing) and wobble base pairing (particularly G-U ribonucleotide base pairing). More particularly, complementarity between X₁ and Y₂ and between X₂ and Y₁ consists of Watson-Crick base pairing (A-U and C-G ribonucleotide base pairing).

In a particular embodiment, R3 comprises or consist of SEQ ID NO. 27 (AAAUCAAAX₁X₂), SEQ ID NO. 28 (GAAAUCAAAX₁X₂), SEQ ID NO. 24(CGAAAUCAAACUX₁X₂), SEQ ID NO. 29 (UCGAAAUCAAACUAX₁X₂), SEQ ID NO. 30 (AUUCX₁X₂), SEQ ID NO. 31 (CAUUCGX₁X₂), SEQ ID NO. 32 (ACAUUCGUX₁X₂), SEQ ID NO. 33 (UCACAUUCGUUAX₁X₂), SEQ ID NO. 34 (AUCACAUUCGUUAUX₁X₂), SEQ ID NO. 35 ( GAUCACAUUCGUUAUCX₁X₂), SEQ ID NO. 36 (UGAUCACAUUCGUUAUCUAX₁X₂), SEQ ID NO. 37 (AUGAUCACAUUCGUUAUCUAUX₁X₂), SEQ ID NO. 38 (GAUGAUCACAUUCGUUAUCUAUUX₁X₂), SEQ ID NO. 39 (AUGAUGAUCACAUUCGUUAUCUAUUUUUUX₁X₂), SEQ ID NO. 40 (UCGAUUCCUAX₁X₂), particularly region R3 comprises or consists of SEQ ID NO. 24 (5'-CGAAAUCAAACUX₁X₂), wherein X₁ and X₂ are as defined above. In another particular embodiment, R3 consists of SEQ ID NO. 27 (AAAUCAAAX₁X₂), SEQ ID NO. 28 (GAAAUCAAAX₁X₂), SEQ ID NO. 24 (CGAAAUCAAACUX₁X₂), SEQ ID NO. 29(UCGAAAUCAAACUAX₁X₂), SEQ ID NO. 30 (5'-AUUCX₁X₂), SEQ ID NO. 31 (CAUUCGX₁X₂), SEQ ID NO. 32 (ACAUUCGUX₁X₂), SEQ ID NO. 33 (UCACAUUCGUUAX₁X₂), SEQ ID NO. 34 (AUCACAUUCGUUAUX₁X₂), SEQ ID NO. 35 (GAUCACAUUCGUUAUCX₁X₂), SEQ ID NO. 36 (UGAUCACAUUCGUUAUCUAX₁X₂), SEQ ID NO. 37 (AUGAUCACAUUCGUUAUCUAUX₁X₂), SEQ ID NO. 38 (GAUGAUCACAUUCGUUAUCUAUUX₁X₂), SEQ ID NO. 39 ( AUGAUGAUCACAUUCGUUAUCUAUUUUUUX₁X₂), SEQ ID NO. 40 (UCGAUUCCUAX₁X₂), particularly region R3 consists of SEQ ID NO. 24 (CGAAAUCAAACUX₁X₂), wherein X₁ and X₂ are as defined above.

In a particular embodiment, region R3 has a length of at least 6, at least 7, at least 8, at least 9, at least 10, at least 11, at least 12, at least 13, at least 14, at least 15, at least 16, at least 17, at least 18, at least 19, at least 20, at least 22, at least 25, at least 26, at least 28, at least 30, at least 31, at least 32, at least 33, at least 34, at least 35, at least 40, at least 45, at least 50, particularly at least 10 nucleotides. In a particular embodiment, region R3 has a length of 6, 7, 8, 9, 10, 11, 12, 13, 14, 15, 16, 17, 18, 19, 20, 22, 25, 26, 28, 30, 31, 32, 33, 34, 35, 40, 45, 50, particularly 12 nucleotides, more particularly 16 nucleotides. In a particular embodiment, region R3 has a length of 6-50, 6-40, 6-35, 6-33, 6-32, 6-31, 7-31, 8-31, 9-31, 10-31, 12-31, 14-31, or 16-31, particularly, 14-31 nucleotides. In another particular embodiment, region R3 has a length of 10-25, 10-18, 10-14, 10-12, particularly 10-14 nucleotides. In another particular embodiment, region R3 has a length of 12-16, particularly of 14-16 nucleotides. In a particular embodiment, region R3 has a length of 16 nucleotides, more particularly of 14 nucleotides.

In a particular embodiment, region R1 comprises or consists of SEQ ID NO. 20 (AGUAGAGAAGAAUCUGUA), or a variant thereof at least 80% identical to SEQ ID NO. 20, region R3 comprises SEQ ID NO. 5 (CGAAAUCAAACU) or consists of SEQ ID NO. 24 (CGAAAUCAAACUX₁X₂) wherein nucleotides X₁ and X₂ at the 3'end of SEQ ID NO. 24 form the sequence 5'-X₁X₂-3' that is the reverse complement of the sequence 5'-Y₁Y₂-3' formed by the last two nucleotides Y₁ and Y₂ at the 3' end of region R2 sequence, and region R5 comprises or consists of SEQ ID NO. 23 (UUGGCUCUUCUUACU), or a variant thereof at least 80% identical to SEQ ID NO. 23. More particularly, R1 comprises SEQ ID NO. 20 (AGUAGAGAAGAAUCUGUA), region R3 comprises SEQ ID NO. 5 (5'-CGAAAUCAAACU), and region R5 comprises SEQ ID NO. 23 (UUGGCUCUUCUUACU). In another particular embodiment, R1 comprises SEQ ID NO. 20 (AGUAGAGAAGAAUCUGUA), region R3 consists of SEQ ID NO. 24 (CGAAAUCAAACUX₁X₂) wherein nucleotides X₁ and X₂ at the 3'end of SEQ ID NO. 24 form the sequence 5'-X₁X₂-3' that is the reverse complement of the sequence 5'-Y₁Y₂-3' formed by the last two nucleotides Y₁ and Y₂ at the 3' end of region R2 sequence, and region R5 comprises SEQ ID NO. 23 (UUGGCUCUUCUUACU).

In another particular embodiment, region R1 consists of SEQ ID NO. 20 (AGUAGAGAAGAAUCUGUA), or a variant thereof at least 80% identical to SEQ ID NO. 20, region R3 consists of SEQ ID NO. 24 (CGAAAUCAAACUX₁X₂) wherein nucleotides X₁ and X₂ at the 3'end of SEQ ID NO. 24 form the sequence 5'-X₁X₂-3' that is the reverse complement of the sequence 5'-Y₁Y₂-3' formed by the last two nucleotides Y₁ and Y₂ at the 3' end of region R2 sequence, and region R5 consists of SEQ ID NO. 23 (UUGGCUCUUCUUACU), or a variant thereof at least 80% identical to SEQ ID NO. 23. More particularly, region R1 consists of SEQ ID NO. 20 (AGUAGAGAAGAAUCUGUA), region R3 consists of SEQ ID NO. 24 (CGAAAUCAAACUX₁X₂) wherein nucleotides X₁ and X₂ at the 3'end of SEQ ID NO. 24 form the sequence 5'-X₁X₂-3' that is the reverse complement of the sequence 5'-Y₁Y₂-3' formed by the last two nucleotides Y₁ and Y₂ at the 3' end of region R2 sequence, and region R5 consists of SEQ ID NO. 23 (UUGGCUCUUCUUACU).

### 1.4- Additional embodiments of the first aspect of the invention

In an embodiment of the first aspect of the invention, the miRNA of the invention targets a gene in the genome of a plant pathogen or a gene in the genome of a plant.

The term "plant pathogen", as used herein, refers to organisms that can infect a plant, referred to as the host of the plant pathogen, and induces a disease in the host plant, at least in an organ of the host plant, including the leaf, stem, root, vascular system, flower and/or the fruit of the host plant. In a particular embodiment, the plant pathogen is selected from the group consisting of viruses, viroids, bacteria, fungi, oomycetes, and nematodes. In another particular embodiment, the plant pathogen is selected from the group consisting of viruses, viroids, bacteria, fungi, oomycetes, and nematodes. In a more particular embodiment, the plant pathogen is a virus selected from the group consisting of Tomato spotted wilt virus (TSWV), Tomato brown rugose fruit virus (TOBRFV), Tomato leaf curl new delhi virus (ToLCNDV), Tomato mosaic virus (ToMV), Cucumber mosaic virus (CMV), Turnip mosaic virus (TuMV), Tomato yellow leaf curl virus (TYLCV), Potato virus Y (PVY), Cauliflower mosaic virus (CaMV), African cassava mosaic virus (ACMV), Plum pox virus (PPV), Brome mosaic virus (BMV), Potato virus X (PVX), Tobacco mosaic virus (TMV), Alfalfa mosaic virus (AMV) and Citrus tristeza virus (CTV). In a particular embodiment, the plant pathogen is virus selected from the list consisting of Tomato spotted wilt virus (TSWV), Tomato brown rugose fruit virus (TOBRFV), and Tomato leaf curl new delhi virus (ToLCNDV), particularly, the plant pathogen is the Tomato spotted wilt virus (TSWV). In another particular embodiment, the plant pathogen is a viroid, from a genus selected from the list consisting of Pospiviroid, Hostuviroid, Cocadviroid, Apscaviroid, Coleviroi, Avsunviroid, Pelamoviroid, Elaviroid, more particularly, the viroids are selected from the list consisting of Potato spindle tuber viroid (PSTVd), Tomato chlorotic dwarf viroid (TCDVd), Mexican papita viroid (MPVd), the Tomato planta macho viroid; (TPMVd), Chrysanthemum stunt viroid; (CSVd), Tomato apical stunt viroid; (TASVd), Iresine viroid 1; (IrVd-1), Columnea latent viroid; (CLVd), Hop stunt viroid, Coconut cadang-cadang viroid, Coconut tinangaja viroid (CTiVd), Hop latent viroid; (HLVd), Citrus IV viroid; (CVd-IV), Citrus III viroid; (CVd-III), Apple dimple fruit viroid; (ADFVd), Grapevine yellow speckle viroid 1 (GVYSd-1), Grapevine yellow speckle viroid 2 (GVYSd-2), Citrus bent leaf viroid (CBLVd), Pear blister canker viroid; (PBCVd), Australian grapevine viroid (AGVd), Coleus blumei viroid 2 (CbVd-2), Coleus blumei viroid 3 (CbVd-3), Avocado sunblotch viroid, Peach latent mosaic viroid, and Eggplant latent viroid. In a particular embodiment, the plant pathogen is the Potato spindle tuber viroid (PSTVd).

In another embodiment, the plant pathogen is a virus selected from the list of viruses indicated provided in Barathi et al., (2023), Recent trends and advances of RNA interference (RNAi) to improve agricultural crops and enhance their resilience to biotic and abiotic stresses. Plant Physiology and Biochemistry 194:600-618, particularly from the list of viruses indicated in table 5 of said document (Barathi et al. (2023) *supra).*

In another embodiment, the plant pathogen is an insect selected from the list of insects indicated provided in Barathi et al., (2023), supra, particularly from the list of insects indicated in tables 2 and 3 of said document (Barathi et al. (2023) *supra).*

In another embodiment, the plant pathogen is a fungi selected from the list of fungus indicated provided in Barathi et al., (2023), supra, particularly from the list of fungus indicated in table 4 of said document (Barathi et al. (2023) *supra).*

In another embodiment, the plant pathogen is a nematode selected from the list of nematodes indicated provided in Barathi et al., (2023), supra, particularly from the list of nematodes indicated in table 6 of said document (Barathi et al. (2023) *supra).*

In a particular embodiment of the first aspect, the host plant of the plant pathogen is from a genus selected from the list consisting of *Solanum sp, Arabidopsis sp., Nicotiana sp., Pyrus sp, Capsicum sp., Citrullus sp., Cucumis sp., Cucurbita sp., Triticum sp., Oryza sp., Pisum sp., Cicer sp., and Citrus sp.,* particularly is from a species selected from the list consisting of *Solanum lycopersicum, Arabidopsis thaliana, Nicotiana benthamiana, Pyrus communis L., Solanum tuberosum, Solanum melongena, Capsicum annuum, Cucumis melo, Cucumis sativus, Citrullus lanatus, Cucurbita moschata, Zea mays, Triticum aestivum, Triticum turgidum, Oryza sativa, Pisum sativum, Cicer arietinum, Citrus sinensis, Citrus limon, Citrus paradisi and Citrus reticulata.* In another particular embodiment, the host plant of the plant pathogen is selected from the plants disclosed in Barathi et al. (2023) *supra,* particularly in tables 1, 2, 4-7 of Barathi et al. (2023) *supra.* In another particular embodiment of the first aspect, the host plant of the plant pathogen is the plant as defined in the eighth aspect of the invention.,

In an embodiment, the gene in the genome of the plant pathogen targeted by the miRNA of the invention is a gene encoding a viral protein selected from the list consisting of coat protein (CP), movement protein (MP), RNA polymerases, RNA-dependent RNA polymerase (RdRp), and viral suppressors of RNA silencing (VSRs). In a particular embodiment, the genes of the previous embodiment, are genes from the genome of a plant virus, i.e. the plant pathogen in the previous embodiment is a virus. Methods for determining the sequence of a miRNA that can be used to target a specific gene, as those just indicated, are well known by an expert in the field. Said methods include, for instance, the use of the P-SAMS script (P-SAMS script (https://github.com/carringtonlab/p-sams) as disclosed in Fahlgren, N , et al., (2016) P-SAMS: a web site for plant artificial microRNA and synthetic trans-acting small interfering RNA design. Bioinformatics, 32, 157-8. In addition, functional assays can be performed, using `B/c' compatible vectors, as those mentioned in Fahlgren, N , et al., (2016) *supra,* described in Carbonell A. et al., (2019), Fast-forward identification of highly effective artificial small RNAs against different Tomato spotted wilt virus isolates. MPMI, 32, 142-156, or described in the examples below.

In a particular embodiment, the sequence encoding the target sequence of the miRNA of the invention in the gene of the plant pathogen, is comprised in a conserved region of the gene. In other words, the miRNA of the invention targets a conserved region of a gene in a plant pathogen genome. The term "conserved region of a gene", as used herein refers to a sequence within a gene that is conserved between genus, species, or strains of a particular plant pathogen. In a particular embodiment, the region of a gene is considered to be a conserved region if the nucleic acid sequence of said region is at least 70%, 75% 80%, 85%, 90%, 95%, 97%, 98%, 99%, or 100% identical between at least 3 different genus, species or strains of the plant pathogen, particularly between at least 3 different strains of the plant pathogen. In another particular embodiment, the region of a gene is considered to be a conserved region if the nucleic acid sequence of said region is at least 70%, 75% 80%, 85%, 90%, 95%, 97%, 98%, 99%, or 100% identical between at least 4 different genus, species or strains of the plant pathogen, particularly between at least 4 different strains of the plant pathogen. In another particular embodiment, the region of a gene is considered to be a conserved region if the nucleic acid sequence of said region is at least 70%, 75% 80%, 85%, 90%, 95%, 97%, 98%, 99%, or 100% identical between at least 5 different genus, species or strains of the plant pathogen, particularly between at least 5 different strains of the plant pathogen. Methods to determine the conservation level of a sequence between genus, species or strains of a particular organism are known by an expert in the field. Non-limiting examples of such methods, include comparing the % identity between the corresponding sequences, following any of the methods indicated in the definition of "identity" above.

In a particular embodiment, the miRNA target gene is the gene encoding the protein RdRp in the genome of a plant virus, or the gene encoding the putative movement protein NSm in the genome of a plant virus, more particularly, wherein the plant pathogen is the virus TSWV.

In an embodiment, the RNA sequence of the guide strand of the miRNA of the invention is selected from the sequences provided in table 1 in Carbonell A. et al., (2019), Fast-forward identification of highly effective artificial small RNAs against different Tomato spotted wilt virus isolates. MPMI, 32, 142-156. In a more particular embodiment, the RNA sequence of the guide strand of the miRNA of the invention, particularly the sequence of region R2, is selected from the list consisting of SEQ ID NO. 42 (UGUAAGACGUGAUUGUGUCCU), SEQ ID NO. 43 (UAUCAGCUCUGGGUGAAUCGG), SEQ ID NO. 44 (UUGGUAUAGUGGGGCAUACCG), and SEQ ID NO. 45 (UCAGAGUGCACAAUCCAUCUU), particularly, wherein the plant pathogen is the TSWV.

In another embodiment, the RNA sequence of the guide strand of the miRNA of the invention, particularly the sequen of reion R2, is selected from the list consisting of SEQ ID NO. 46 (UCGGCCGCUGGGCACUCCCCU), SEQ ID NO. 47 (UGCGGGCGCGAGGAAGGACAG), SEQ ID NO. 48 (UUUCCACCGGGUAGUAGCCGU), SEQ ID NO: 49 (UUAGUUCCGAGGAACCAACUC), , SEQ ID NO: 50 (UCAAGGGCUAAACACCCUCGG), SEQ ID NO. 51 (UGGAACCGCAGUUGGUUCCUG), SEQ ID NO. 52 (UGAAGCUCCCGAGAACCGCUA), SEQ ID NO: 53 (UGUCGCUUCGGCUACUACCCC), SEQ ID NO: 54 (UGUCCUUCCUCGCGCCCGCAC), SEQ ID NO. 55 (UGGUUCACACCUGACCUCCUC), SEQ ID NO. 56 (UCCCGGGGAAACCUGGAGCGU), particularly, wherein the plant pathogen is the PsTVd. In a more particular embodiment, the RNA sequence of the guide strand of the miRNA of the invention, particularly the sequence of region R2, is selected from the list consisting of SEQ ID NO. 47, SEQ ID NO. 49, SEQ ID NO. 54, and SEQ ID NO. 56, particularly wherein the plant pathogen is the PsTVd.

In an another embodiment, the miRNA of the invention targets a gene in the genome of a plant. In a particular embodiment, the miRNA target gene in the genome of a plant is a gene encoding a protein selected from the list consisting of SFT (single flower truss), particularly from *Solanum lycopersium,* TFL1 (terminal flower 1), particularly from *Pyrus communis L.,* DET1 (DE-ETIOLATED 1), particularly from *Solanum lycopersicum,* ALS (acetolactate synthase), ACCase (acetyl-CoA carboxylase) and EPSPS (5-enolpyruvylshikimate-3-phosphate). In a particular embodiment, the miRNA target gene in the genome of a plant is selected from those disclosed in Barathi et al. 2023, *supra,* particularly in tables 1 and 7 of Barathi et al. 2023, *supra.* In another particular embodiment, the target gene in the genome of a plant is selected from those disclosed in Dong H. et al., (2021) The Development of Herbicide Resistance Crop Plants Using CRISPR/Cas9-Mediated Gene Editing. Genes, 12(6), 912 (https://doi.org/10.3390/genes12060912).

In a particular embodiment, the plant is selected from the lists of plants provided above as plant pathogen host plants. Thus, in an embodiment, the genus of the plant with the gene targeted by the miRNA of the invention is selected from the list of plant genus provided above for the host plant of the plant pathogen. In another embodiment, the species of the plant with the gene targeted by the miRNA of the invention is selected from the list of plant species provided above for the host plant of the plant pathogen. In a particular embodiment, the plant with the gene targeted by the miRNA of the invention is the plant as defined in the eighth aspect of the invention.,

In a particular embodiment, the miRNA of the invention is an amiRNA, as defined above. In another particular embodiment, the miRNA of the invention is miRNA found in nature, more particularly, an endogenous miRNA of the plant that is the host of the plant pathogen comprising the miRNA target gene, as any of the plants listed above, or an endogenous miRNA of the plant that comprise the miRNA target gene, as any of the plants listed above.

In a second aspect, the invention provides a viral vector comprising the reverse complement sequence of the sequence of the viral vector as defined in the first aspect of the invention. In a particular embodiment, the viral vector of the second aspect of the invention comprises a heterologous sequence encoding or comprising the reverse complement of the RNA sequence S1 defined in the first aspect of the invention. In another particular embodiment, the terms, embodiments and definitions of the first aspect of the invention apply to the second aspect of the invention, wherein the RNA sequence S1, regions R1, R2, R3, R4 and R5 defined in any of said embodiments and definitions, is substituted by the corresponding reverse complement sequence.

In a particular embodiment, the RNA sequence S1 corresponds to the RNA sequence of a functional minimal miRNA precursor, particularly, of a functional minimal miRNA precursor of the miRNA of the invention. In a more particular embodiment, the RNA sequence S1 corresponds to the RNA sequence upstream the poly-A tail of a functional minimal miRNA precursor, particularly the RNA sequence upstream the poly-A tail of a functional minimal miRNA precursor of the miRNA of the invention. The term "functional minimal miRNA precursor", as used herein, refers to an RNA molecule that comprises the sequences necessary for the expression of a specific miRNA, particularly the hairpin structure allowing the processing by a DCL protein, particularly by the DCL1, or the DCL4 protein and the corresponding release of a miRNA/miRNA* duplex.

In a particular embodiment, the sequence of the region of S1 consisting of regions R1, R2, R3, R4, and R5 (herein referred to as region F of the RNA sequence S1) forms a miRNA foldback structure, particularly, wherein regions R1 and R5 form the basal stem of the miRNA foldback structure, R4 and R2 form the miRNA/miRNA* duplex, and R3 forms, or comprises, the loop structure of the miRNA foldback structure. Particularly, region R1 base paired to region R5 forms the basal stem of the miRNA foldback structure, region R2 base paired to region R4 forms the miRNA/miRNA* duplex of the miRNA foldback structure, and R3 forms, or comprises, the loop structure of the miRNA foldback structure. The definition of the miRNA foldback structure, of the basal stem of the miRNA foldback structure and the definition of the miRNA/miRNA* duplex as well as of the loop structure within a stem loop structure are provided above. In another particular embodiment, the RNA sequence S1 consists of regions R1, R2, R3, R4 and R5 as defined herein. Thus, in a more particular, the RNA sequence S1 forms a miRNA foldback structure, as indicated in the embodiments above, particularly, wherein regions R1 and R5 form the basal stem of the miRNA foldback structure, R4 and R2 form the miRNA/miRNA* duplex, and R3 forms, or comprises, the loop structure of the miRNA foldback structure.

In a particular embodiment, the miRNA of the invention is expressed from the viral vector of the first or second aspect of the invention, particularly in a plant cell comprising said viral vector. In a particular embodiment, the plant cell is a cell from a plant as defined above, particularly from a plant selected from the list of host plants of the plant pathogen provided above, more particularly, from a plant selected from the list of plant species provided above for the host plant of the plant pathogen.

As well understood by a skilled person, a first RNA sequence upstream a second RNA sequence or RNA region, as described in any aspects of the invention, indicates that the 3' end nucleotide of the first RNA sequence is directly linked to the 5'-end nucleotide of the second RNA sequence of said RNA region. Thus, a first RNA sequence upstream a second RNA sequence or RNA region, as described in any aspects of the invention, is herein indicated in a 5'- to 3'-end direction. Similarly, a first RNA sequence downstream a second RNA sequence or RNA region, as described in any aspects of the invention, indicates that the 5' end nucleotide of the first RNA sequence is directly linked to the 3'-end nucleotide of the second RNA sequence of said RNA region. Concomitantly, a first RNA sequence downstream a second RNA sequence or RNA region, as described in any aspects of the invention, is herein also indicated in a 5'- to 3'- end direction. Additionally, unless indicated otherwise, the position of nucleotides, regions or sequences within an RNA sequence are provided in the 5'- to 3'-end direction in all the aspects of the invention.

### II-The virus of the invention

In a third aspect, the invention provides a virus encoded by or comprising the viral vector of the first or the second aspect of the invention. In a particular embodiment, the virus of the third aspect of the invention induces mild symptoms or is asymptomatic in the plant defined in section I-4 of the first aspect of the invention as the host plant of the plant pathogen, or as the plant comprising the target gene of the miRNA of the invention. Thus, infection of the plant as defined in section I-4 of the first aspect of the invention with the virus of the invention causes mild or no symptoms in the plant. In a particular embodiment, the virus of the invention is an attenuated virus. In another particular embodiment, the viral vector of the invention is modified to express an attenuated virus in the plant comprising the viral vector, wherein the plant is as just defined. The term "attenuated virus", as used herein, refers to a virus that produces milder damage in the infected organism than the wild-type counterpart from which it is derived.

In a particular embodiment, the virus of the invention is variant of the PVX, TRV, TMV, TGMV, CMV, BSMV, BMV, FoMV, BYDV, WDV, or CaLCuV.

In a particular embodiment, the virus of the invention expresses the RNA sequence S1 of the invention in a host plant of said virus, particularly in the plant as defined in section I-4 of the first aspect of the invention as the host plant of the plant pathogen, or as the plant comprising the target gene of the miRNA of the invention. In another particular embodiment, the virus of the invention expresses the miRNA of the invention in a host plant of said virus, particularly in the plant as defined in section I-4 of the first aspect of the invention.

### III- The polynucleotides of the invention

In a fourth aspect, the invention provides an RNA polynucleotide comprising the RNA sequence S1 as defined in the first aspect, provided that the RNA polynucleotide does not comprise:
(i) in the region upstream of region R1 in a 5'- to 3'-end direction, a sequence comprised in SEQ ID NO. 25 of at least 10 nucleotides, and/or
(ii) in the region downstream of region R5 in a 5'- to 3'-end direction, a sequence comprised in SEQ ID NO. 26 of at least 10 nucleotides.

In a fifth aspect, the invention provides a DNA polynucleotide encoding the polynucleotide as defined in the fourth aspect of the invention.

In a particular embodiment, the RNA polynucleotide of the fourth aspect of the invention comprises the RNA sequence S1 as defined in the first aspect, provided that the RNA polynucleotide does not comprise in the region upstream of region R1 in a 5'- to 3'-end direction,, a sequence comprised in SEQ ID NO. 25 of at least 10, at least 20, at least 30, at least 40, at least 50, at least 70, at least 100, at least 150, at least 180, at least 200, at least 210, at least 220, at least 230, or at least 232 nucleotides, particularly of at least 100 nucleotides. In a more particular embodiment, the RNA polynucleotide of the fourth aspect of the invention comprises the RNA sequence S1 as defined in the first aspect, provided that the RNA polynucleotide does not comprise in the region upstream of region R1 in a 5'- to 3'-end direction" sequence SEQ ID NO. 25.

In another particular embodiment, the RNA polynucleotide of the fourth aspect of the invention comprises the RNA sequence S1 as defined in the first aspect, provided that the RNA polynucleotide does not comprise in the region downstream of region R5, a sequence comprised in SEQ ID NO. 26 of at least 10, at least 20, at least 30, at least 40, at least 50, at least 70, at least 90, at least 100, at least 125, at least 150, at least 160, at least 170, at least 180, or at least 183 nucleotides, particularly of at least 100 nucleotides. In a more particular embodiment, the RNA polynucleotide of the fourth aspect of the invention comprises the RNA sequence S1 as defined in the first aspect, provided that the RNA polynucleotide does not comprise in the region downstream of region R5 in a 5'- to 3'-end direction,, sequence SEQ ID NO. 26.

In another particular embodiment, the RNA polynucleotide of the fourth aspect of the invention comprises the RNA sequence S1 as defined in the first aspect, provided that the RNA polynucleotide does not comprise:
(i) in the region upstream of region R1 in a 5'- to 3'-end direction, a sequence comprised in SEQ ID NO. 25 of at least 150 nucleotides, and/or
(ii) in the region downstream of region R5 in a 5'- to 3'-end direction, a sequence comprised SEQ ID NO. 26 of at least 110 nucleotides.

In another particular embodiment, the RNA polynucleotide of the fourth aspect of the invention comprises the RNA sequence S1 as defined in the first aspect, provided that the RNA polynucleotide does not comprise:
(i) in the region upstream of region R1 in a 5'- to 3'-end direction, a sequence comprised in SEQ ID NO. 25 of at least 200 nucleotides, and/or
(ii) in the region downstream of region R5 in a 5'- to 3'-end direction, a sequence comprised SEQ ID NO. 26 of at least 150 nucleotides.

In a particular embodiment, the RNA polynucleotide of the fourth aspect of the invention comprises the RNA sequence S1 as defined in the first aspect, provided that the RNA polynucleotide does not comprise:
(i) in the region upstream of region R1 in a 5'- to 3'-end direction, a sequence comprised in SEQ ID NO. 25 of at least 220 nucleotides, and/or
(ii) in the region downstream of region R5 in a 5'- to 3'-end direction, a sequence comprised SEQ ID NO. 26 of at least 170 nucleotides.

In a more particular embodiment, the RNA polynucleotide of the fourth aspect of the invention comprises the RNA sequence S1 as defined in the first aspect, provided that the RNA polynucleotide does not comprise:
(i) in the region upstream of region R1 in a 5'- to 3'-end direction, sequence SEQ ID NO. 25, and/or
(ii) in the region downstream of region R5 in a 5'- to 3'-end direction, sequence SEQ ID NO. 26.

In a particular embodiment, the length of the polynucleotide of the fourth aspect of the invention is of at least 950, at least 925, at least 900, at least 875, at least 850, at least 827, at least 826, at least 825, at least 824, at least 823, at least 822, at least 820, at least 810, at least 800, at least 750, at least 700, at least 650, at least 600, at least 550, at least 525, at least 520, at least 510, at least 500, at least 490, at least 489, at least 488, at least 487, at least 486, at least 485, at least 484, at least 483, at least 482, at least 480, at least 475, at least 450, 400, at least 350, at least 300, at least 275, at least 250, at least at 225, at least 200, at at least 170, at least 150, at least 140, at least 130, at least 129, at least 128, at least 127, at least 125, at least 124, at least 123, at least 122, at least 121, at least 120, at least 117, at least 115, at least 110, at least 107, at least 105, at least 102, at least 100, at least 95, at least 90, or at least 89 nucleotides, particularly at least 824 nucleotides, more particularly at least 486 nucleotides. In another particular embodiment, the length of the polynucleotide of the fourth aspect of the invention is of 950, 925, 900, 875, 850, 827, 826, 825, 824, 823, 822, 820, 810, 800, 750, 700, 650, 600, 550, 525, 520, 510, 500, 490, 489, 488, 487, 486, t 485, 484, 483, 482, 480, 475, 450, 400, 350, 300, 275, 250, 225, 200, 170, 150, 140, 130, 129, 128, 127, 125, 124, 123, 122, 121, 120, 117, 115, 110, 107, 105, 102, 100, 95, 90, 89 nucleotides, particularly 824 nucleotides, more particularly 486 nucleotides

In a particular embodiment, the polynucleotide of the fourth aspect of the invention comprises a poly-A tail at the 3'end. In another particular embodiment, the polynucleotide of the fourth aspect of the invention comprises a cap structure at the 5' end. In a more particular embodiment, the polynucleotide of the fourth aspect of the invention comprises a poly-A tail at the 3'end and a cap structure at the 5' end.

In a particular embodiment, the sequence of the polynucleotide of the fourth aspect of the invention consists of the RNA sequence S1 and at least 1, at least 10, at least 20, at least 0, at least 100, at least 150, atleast 250, at least 300, at least 350, at least 375, at least 380, at least 390, at least 395, at least 396 at least 397, at least 398, at least 399, at least 400, at least 450, at least 500, at least 600, at least 700, at least 710, at least 720, at least 730,n at least 735, at least 740, at least 750, at least 800, particularly at least 397 nucleotides upstream the RNA sequence S1. In another particular embodiment, the sequence of the polynucleotide of the fourth aspect of the invention consists of the RNA sequence S1 and at least 1, at least10, at least 20, at least 0, at least 100, at least 150, atleast 250, at least 300, at least 350, at least 375, at least 380, at least 390, at least 395, at least 396 at least 397, at least 398, at least 399, at least 400, at least 450, at least 500, at least 600, at least 700, at least 710, at least 720, at least 730, at least 735, at least 740, at least 750, at least 800, particularly at least 735 nucleotides downstream the RNA sequence S1. In another particular embodiment, In another particular embodiment, the polynucleotide of the fourth aspect of the invention consists of the RNA sequence S1 and at least 1, particularly at least 397 nucleotides upstream the RNA sequence S1, and/or at least 1, at least 735 nucleotides downstream the RNA sequence S1.

In a particular embodiment, the sequence of the polynucleotide of the fourth aspect of the invention consists of the RNA sequence S1. In another particular embodiment, the sequence of the polynucleotide of the fourth aspect of the invention consists of the RNA sequence S1 and a poly-A tail at the 3'end. In another particular embodiment, the sequence of the polynucleotide of the fourth aspect of the invention consists of the RNA sequence S1 with a cap structure at the 5'end. In another particular embodiment, the sequence of the polynucleotide of the fourth aspect of the invention consists of the RNA sequence S1 with a cap structure at the 5' end and a poly-A tail at the 3'end. In another particular embodiment, the sequence of the polynucleotide of the fourth aspect of the invention consists of the RNA of region F (i.e. the region of the RNA sequence S1 consisting of regions R1, R2, R3, R4 and R5). In another particular embodiment, the sequence of the polynucleotide of the fourth aspect of the invention consists of the RNA sequence of region F, and a poly-A tail at the 3'end. In another particular embodiment, the sequence of the polynucleotide of the fourth aspect of the invention consists of the RNA sequence of region F with a cap structure at the 5'end. In another particular embodiment, the sequence of the polynucleotide of the fourth aspect of the invention consists of the RNA sequence of a fragment R with a cap structure at the 5' end and a poly-A tail at the 3'end.

The term "poly-A tail" as used herein, refers to a long chain of adenine ribonucleotides that is added to the 3'end of an RNA molecule during RNA processing and is generally of 50-250 nucleotides long. The poly-A tail is known to increase the stability of the RNA molecule and to be involved in the translation of the mRNA molecules. In a particular embodiment, the poly-A tail of the polynucleotide of the fourth aspect of the invention is of 50, 70, 80, 90, 100, 120, 150, 170, 200, 220, or 250 adenine ribonucleotides.

The term "cap structure" as used herein, refers to a chemical modification of RNA molecules in eukaryotes, which increases the stability of the RNA and that is important for the transport of the RNA from the nucleus to the cytoplasm and for the subsequent translation of mRNAs by the ribosomes. In eukaryotes, the 5' cap consists of a guanine nucleotide connected to the RNA via an unusual 5' to 5' triphosphate linkage. This guanosine is methylated on the 7 position directly after capping in vivo by a methyltransferase. It is referred to as a 7-methylguanylate cap, abbreviated m7G. In a particular embodiment, the cap structure of the polynucleotide of the fourth aspect is an m7G cap.

In an additional aspect, the invention provides an RNA polynucleotide that comprises the RNA sequence S1 as defined in the first aspect, provided that the RNA polynucleotide does not comprise:
(i) in the region upstream of region R1 in a 5'- to 3'-end direction, a sequence of at least 10, at least 20, at least 30, at least 40, at least 50, at least 70, at least 100, at least 150, at least 180, at least 200 nucleotides, particularly at least 10 nucleotides, more particularly at least 100 nucleotides, comprised upstream sequence R1 in a 5'- to 3'-end direction in the miRNA primary precursor whose miRNA foldback BS has a strand with sequence R1, and/or
(ii) in the region downstream of region R5 in a 5'- to 3'-end direction, a sequence of at least 10, at least 20, at least 30, at least 40, at least 50, at least 70, at least 100, at least 150, at least 180, at least 200 nucleotides, particularly at least 10 nucleotides, more particularly at least 100 nucleotides, comprised downstream sequence R5 in a 5'- to 3'-end direction in the miRNA primary precursor whose miRNA foldback BS has a strand with sequence R1.

In a particular embodiment of this additional aspect, the RNA polynucleotide comprises the RNA sequence S1 as defined in the first aspect, provided that the RNA polynucleotide does not comprise:
(i) in the region upstream of region R1 in a 5'- to 3'-end direction, the sequence comprised upstream sequence R1 in a 5'- to 3'-end direction in the miRNA primary precursor whose miRNA foldback BS has a strand with sequence R1, and/or
(ii) in the region downstream of region R5 in a 5'- to 3'-end direction, the sequence comprised downstream sequence R5 in a 5'- to 3'-end direction in the miRNA primary precursor whose miRNA foldback BS has a strand with sequence R1.

In a particular embodiment of this additional aspect, the RNA polynucleotide comprises the RNA sequence S1 as defined in the first aspect, provided that the RNA polynucleotide does not comprise:
(i) in the region upstream of region R1 in a 5'- to 3'-end direction, a sequence of at least 50 nucleotides, particularly the whole sequence comprised upstream SEQ ID NO. 20, SEQ ID NO. 57, SEQ ID NO. 59, SEQ ID NO: 61, SEQ ID NO: 63 in a 5'- to 3'-end direction in the miRNA primary precursor of AtMIR390a, AtMIR172a, AtMIR171a, AtMIR167a or AtMIR166b, respectively, and/or
(ii) in the region downstream of region R5 in a 5'- to 3'-end direction, a sequence of at least 50 nucleotides, particularly the whose sequence comprised downstream SEQ ID NO. 23, SEQ ID NO. 58, SEQ ID NO. 60, SEQ ID NO. 62, or SEQ ID NO. 64 in a 5'- to 3'-end direction in the miRNA primary precursor of AtMIR390a, AtMIR172a, AtMIR171a, AtMIR167a or AtMIR166b, respectively.

In another particular embodiment of this additional aspect, the RNA polynucleotide comprises the RNA sequence S1 as defined in the first aspect, provided that the RNA polynucleotide does not comprise:
(i) in the region upstream of region R1 in a 5'- to 3'-end direction, a sequence of at least 50 nucleotides, more particularly the whole sequence comprised upstream SEQ ID NO. 20 in a 5'- to 3'-end direction in the primary precursor of AtMIR390a, and in the region downstream of region R5 in a 5'- to 3'-end direction, a sequence of at least 50, more particularly the whole sequence comprised downstream SEQ ID NO. 23 in a 5'- to 3'-end direction in the primary precursor of AtMIR390a, particularly wherein region R1 has SEQ ID NO. 20, and/or
(ii) in the region upstream of region R1 in a 5'- to 3'-end direction, a sequence of at least 50 nucleotides, more particularly the whole sequence comprised upstream SEQ ID NO. 57 in a 5'- to 3'-end direction in the primary precursor of AtMIR172a, and in the region downstream of region R5 in a 5'- to 3'-end direction, a sequence of at least 50, more particularly the whole sequence comprised downstream SEQ ID NO. 58 in a 5'- to 3'-end direction in the primary precursor of AtMIR172a, particularly wherein region R1 has SEQ ID NO. 56, and/or
(iii) in the region upstream of region R1 in a 5'- to 3'-end direction, a sequence of at least 50 nucleotides, more particularly the whole sequence comprised upstream SEQ ID NO. 59 in a 5'- to 3'-end direction in the primary precursor of AtMIR171a, and in the region downstream of region R5 in a 5'- to 3'-end direction, a sequence of at least 50, more particularly the whole sequence comprised downstream SEQ ID NO. 60 in a 5'- to 3'-end direction in the primary precursor of AtMIR171a, particularly wherein region R1 has SEQ ID NO. 58, and/or
(iv) in the region upstream of region R1 in a 5'- to 3'-end direction, a sequence of at least 50 nucleotides, more particularly the whole sequence comprised upstream SEQ ID NO. 61 in a 5'- to 3'-end direction in the primary precursor of AtMIR167a, and in the region downstream of region R5 in a 5'- to 3'-end direction, a sequence of at least 50, more particularly the whole sequence comprised downstream SEQ ID NO. 62 in a 5'- to 3'-end direction in the primary precursor of AtMIR167a, particularly wherein region R1 has SEQ ID NO. 60, and/or
(v) in the region upstream of region R1 in a 5'- to 3'-end direction, a sequence of at least 50 nucleotides, more particularly the whole sequence comprised upstream SEQ ID NO. 63 in a 5'- to 3'-end direction in the primary precursor of AtMlR166b, and in the region downstream of region R5 in a 5'- to 3'-end direction, a sequence of at least 50, more particularly the whole sequence comprised downstream SEQ ID NO. 64 in a 5'- to 3'-end direction in the primary precursor of AtMIR166b, particularly wherein region R1 has SEQ ID NO. 62, and/or
(vi) in the region upstream of region R1 in a 5'- to 3'-end direction, a sequence of at least 50 nucleotides, more particularly the whole sequence comprised upstream SEQ ID NO. 65 in a 5'- to 3'-end direction in the primary precursor of AtMlR166b, and in the region downstream of region R5 in a 5'- to 3'-end direction, a sequence of at least 50, more particularly the whole sequence comprised downstream SEQ ID NO. 65 in a 5'- to 3'-end direction in the primary precursor of AtMIR166b, particularly wherein region R1 has SEQ ID NO. 66.

In a particular embodiment, all the embodiments of the fourth aspect apply to this additional aspect. In a particular embodiment of the fifth aspect, the DNA polynucleotide encodes the RNA polynucleotide as defined in the fourth aspect or the polynucleotide of additional aspect just defined above.

### IV- The plasmid and bacterium of the invention

In a sixth aspect, the invention provides a plasmid comprising a sequence that encodes or corresponds to the sequence of the viral vector as defined in the first or second aspect of the invention, or the sequence encoding the polynucleotide as defined in the fourth aspect of the invention.

In a seventh aspect, the invention provides a bacterium that comprises the plasmid as defined in the sixth aspect of the invention.

In a particular embodiment, the plasmid of the sixth aspect is suitable for transfection in plant cells. In a more particular embodiment, the plasmid of the sixth aspect is suitable for transfection in plants mediated by the *Agrobacterium tumefaciens.* In other words, the plasmid of the sixth aspect of the invention is suitable for agroinfiltration. As well understood by a skilled person, plasmid suitable for plant transfection or agroinfiltration need to comprise a plasmids backbone suitable for those purposes. Plasmids backbones suitable for plant transfection and agroinfiltration are well known by an expert in the field. Non-limitative examples include those disclosed in the examples bellow. In a particular, the plasmid backbone of the plasmid of the sixth aspect of the invention is selected from the list consisting of pBIN, pGA, SEV, pEND4K, pBI, pCIB10, pMRK63, pGPTV, pCGN1547, pART, pGKB5, pMJD80 and 81, pPZP, pBINPLUS, pRT100, BIBAC, pCB, pGreen, pPZP-RCS2, pMDC, pRCS2, pEarleyGate, pGWTAC, pORE, pSITE, pMSP, pCAMBIA, PGD, *pMDC32, pLX-B2,* particularly, consists of the *pLX-B2* plasmid. Thus, in a particular embodiment, the plasmid of the sixth aspect of the invention is a *pMDC32* plasmid, or a pLX-B2 plasmid, particularly a pLX-B2 plasmid.
In another particular embodiment, the plasmid of the sixth aspect of the invention is a *pENTR* plasmid.

In a particular embodiment of the sixth aspect, the sequence that encodes or corresponds to the sequence of the viral vector as defined in the first or second aspect of the invention, or the sequence encoding the polynucleotide as defined in the fourth aspect of the inventio, is operatively linked to an expression promoter in the plasmid of the sixth aspect of the invention. The term "operatively linked" has already been defined in the first aspect of the invention and also applies to the sixth aspect of the invention. Suitable expression promoters are known by an expert in the field. In a particular embodiment, the expression promoter is the 35S promoter from the plant pathogen Cauliflower mosaic virus (CaMV).

In a particular embodiment of the seventh aspect of the invention, the bacterium is from the genus *Agrobacterium,* particularly from the species *Agrobacterium tumefaciens.*

In a particular embodiment, the embodiments and definitions of the first, second, third, fourth and fifth aspects of the invention apply to the sixth and seventh aspects of the invention.

### V- Plants and plant extracts of the invention and methods for obtaining them

In an eighth aspect, the invention provides a plant that comprises the viral vector as defined in the first or second aspect of the invention, the virus as defined in the third aspect of the invention, the polynucleotide as defined in the fourth or fifth aspects of the invention, the plasmid as defined in the sixth aspect and/or the bacterium as defined in the seventh aspect.

In a particular embodiment, the plant of the eighth aspect of the invention comprises the viral vector as defined in the first or second aspect of the invention, the virus as defined in the third aspect of the invention, the polynucleotide as defined in the fourth or fifth aspect, the plasmid as defined in the sixth aspect and/or the bacterium as defined in the seventh aspect, in the cells of the plant, particularly in the cells from at least one organ of the plant. In a particular embodiment, the at least one organ of the plant is selected from the list consisting of leaves, the stem, roots, flowers, fruits and combinations thereof. In a particular embodiment, cells of the plant that comprise the viral vector as defined in the first or second aspect of the invention, the virus as defined in the third aspect of the invention, the polynucleotide as defined in the fourth or fifth aspect, the plasmid as defined in the sixth aspect and/or the bacterium as defined in the seventh aspect, are the cells of at least one leaf, more particularly of one leaf of the plant.

In another embodiment, the plant of the eighth aspect of the invention comprises in cells of all the organs of the plant, the viral vector as defined in the first or second aspect of the invention, the virus as defined in the third aspect of the invention, the polynucleotide as defined in the fourth or fifth aspect, the plasmid as defined in the sixth aspect and/or the bacterium as defined in the seventh aspect. In another particular embodiment, plant of the eighth aspect of the invention, comprises in all the cells of the plant, the viral vector as defined in the first or second aspect of the invention, the virus as defined in the third aspect of the invention, the polynucleotide as defined in the fourth or fifth aspect, the plasmid as defined in the sixth aspect and/or the bacterium as defined in the seventh aspect.

In a particular embodiment, the fact that the plant, particularly the cells of the plant as described in the paragraph above, comprise the virus as defined in the third aspect of the invention, refers to the fact that the plant, particularly said cells of the plant, are infected with the virus as defined in the third aspect of the invention. The fact that the plant, particularly the cells of the plant as described in the paragraph above, comprise the bacterium as defined in the seventh aspect of the invention, refers to the fact that the plant, particularly said cells of the plant, are infected with the bacterium as defined in the seventh aspect of the invention.

In a particular embodiment, the plant of the eighth aspect of the invention comprises the virus of the invention, wherein the virus of the invention replicates within the cells of the plant, particularly, wherein the virus of the invention replicates within the cells of the plant and spreads between cells of the plant, particularly between adjacent cells of the plant and/or by the vascular system of the plant. In a particular embodiment, the plant of the invention presents the symptoms of infection by the virus from which the viral vector of the invention derives. In another particular embodiment, the plant of the invention does not present symptoms of infection by the virus from which the viral vector of the invention derives.

In a particular embodiment, the plant of the eighth aspect is resistant to the plant pathogen as defined in any of the embodiments of the first aspect of the invention. In another embodiment, the plant of the eighth aspect is tolerant to the plant pathogen as defined in any of the embodiments of the first aspect of the invention. In a particular embodiment, the plant of the invention comprises the plant pathogen as defined in the first aspect of the invention.

The term "resistant to the plant pathogen", as used herein, refers to a plant that inhibits the infection, multiplication and/or invasion of the plant pathogen within the plant. Methods to determine if a plant is resistant to a pathogen are well known by an expert in the field and include determining the plant pathogen concentration in an organ of the plant exposed to the plant pathogen, as compared to the concentration in the same organ in a control plant not resistant to the plant pathogen and equivalently exposed to the plant pathogen. If the concentration of plant pathogen in the organ of the plant analyzed is lower than in the organ of the control plant, it is considered the plant is resistant to the plant pathogen. In a particular embodiment, the plant is considered to be resistant to the plant pathogen if the concentration of the plant pathogen determined in the organ of the plant analyzed is at least 20%, at least 30%, at least 40%, at least 50%m, at least 60% at least 70%, at least 80%, at least 90%, at least 95%, at least 98% lower than in the organ of the control plant. Methods to determine the concentration of a plant pathogen in a plant organ are well known by an expert in the field. For instance, it can be determined by the quantitative PCR (qPCR), Quantitative reverse transcription PCR (RT-qPCR), or flow cytometry. In a particular embodiment, the control plant not resistant to the plant pathogen is a plant of the same species, the same age and grown under the same conditions as the plant being analyzed, but that does not comprise the viral vector as defined in the first or second aspect of the invention, the virus as defined in the third aspect of the invention, the polynucleotide as defined in the fourth or fifth aspect, the plasmid as defined in the sixth aspect nor the bacterium as defined in the seventh aspect.

The term "tolerant to a plant pathogen", as used herein, refers to a plant that a pathogen can infect, replicate in it, and invade without leading to severe symptoms or diminishing plant growth or marketable yield. Methods to determine if a plant is tolerant to a plant pathogen include determining: a) the level of symptoms associated to said pathogen developed by a plant exposed to the plant pathogen, and comparing them to the level of symptoms developed by a control plant equivalently exposed to the plant pathogen; and b) the concentration of plant pathogen in an organ of the plant analyzed exposed to said plant pathogen, as indicated in the definition of "resistant plant". If the level of symptoms developed by the plant analyzed is lower than those developed by the control plant, and the concentration of plant pathogen in the plant analyzed is comparable to that in the control plant, it is considered that the plant is tolerant to the plant pathogen. In a particular embodiment, the plant is considered to be tolerant to the plant pathogen if the level of symptoms associated to said pathogen in the plant analyzed is at least 20%, at least 30%, at least 40%, at least 50%m, at least 60% at least 70%, at least 80%, at least 90%, at least 95%, at least 98%, particularly at least 60% lower than in the control plant, and the concentration of the plant pathogen determined in the organ of the plant analyzed is 75%-125%, 80%-120%, 90-110% of the concentration in the organ of the control plant. Methods to measure the level of symptoms in a plant are well known by an expert in the field and include, for instance, those disclosed in the examples below. Methods to determine the concentration of a plant pathogen are provided in the definition of "resistant to the plant pathogen".

In a particular embodiment, the plant pathogen mentioned in any of the embodiments of the eight aspect of the invention is selected from the plant pathogens disclosed in the first aspect of the invention. In a particular embodiment, the plant pathogen mentioned in the eighth aspect of the invention is selected from the list consisting of the TSWV, ToBRFV, ToLCNDV, and PSTVd, particularly is the TSWV.

In another embodiment, the plant of the eighth aspect of the invention shows an improved trait, particularly as compared to a control plant. In a particular embodiment, the improved trait, particularly as compared to the control plant, is selected from the list consisting of crop yield, shelf life of fruits, shelf life of vegetables, nutritional value, organoleptic properties, abiotic stress resistance (for instance, to drought, salinity, high temperatures, or cold), or mal sterility. In a particular embodiment, the improved trait, particularly as compared to the control plant, is selected from those disclosed in tables 1 and in table 7 of Barathi et al., (2023), *supra.*

In a particular embodiment, the plant of the eighth aspect of the invention shows an improved trait, particularly as compared to a control plant. wherein said trait is at least 10%, 20%, 30%, 40%, 50%, 75%, 90%, 95%, 100%, 150%, 200%, 250%, 300%, 350%, 400% higher, particularly at least 20% higher, in the plant of the eighth aspect of the invention than in a control plant. In a more particular embodiment, the improved trait is at least 50% higher in the plant of the eighth aspect of the invention than in a control plant. In a yet more particular embodiment, the improved trait is at least 100% higher in the plant of the eighth aspect of the invention than in a control plant. Methods to compare the level of plant traits between different plants, as those disclosed above, are well known by an expert in the field, and include analyzing the plant characters and protein levels mentioned in Barathi et al. (2023) *supra* for each trait, with methods well known by an expert in the field.

In a particular embodiment, the control plant indicated in the embodiments of the plant of the eighth with an improved trait, particularly in the two previous paragraphs, is a plant of the same species, the same age and grown in the same conditions as the plant of the eighth aspect of the invention, but that does not comprise the viral vector as defined in the first or second aspect of the invention, the virus as defined in the third aspect of the invention, the polynucleotide as defined in the fourth or fifth aspect, the plasmid as defined in the sixth aspect nor the bacterium as defined in the seventh aspect.

In an embodiment, the plant of the eighth aspect is a non-transgenic plant. The term "non-transgenic plant", as used herein, refers to the term commonly known by an expert in the field. Particularly, it refers to a plant that has not been genetically modified with genetic engineering techniques. Thus, the genome of the non-transgenic plant only comprises nucleic acid sequences that can be part of the genome of the plant upon mating and/or natural recombination. In a particular embodiment of the eighth aspect, the non-transgenic plant does not comprise artificial sequences integrated in its genome, wherein the artificial sequences is any sequence not found in nature in the genome of said plant. In a particular embodiment of the eighth aspect, the non-transgenic plant does not comprise heterologous sequences integrated in its genome, wherein the heterologous sequences is as defined in the first aspect of the invention. In a particular embodiment of the eighth aspect of the invention, nor the sequence of the viral vector of the first or second aspect of the invention, nor the sequence of the polynucleotide of the fifth aspect of the invention, nor the sequence of the plasmid of the sixth aspect of the invention is comprised or integrated in the genome of the plant as defined in the eighth aspect of the invention. In another particular embodiment, the plant of the eighth aspect of the invention comprises heterologous sequences, or is a transgenic plant, but does not comprise the sequence of the viral vector of the first or second aspect of the invention, nor the sequence of the polynucleotide of the fifth aspect of the invention, nor the sequence of the plasmid of the sixth aspect of the invention. In another particular embodiment, the plant of the eighth aspect of the invention does not comprise heterologous or artificial sequences integrated in its genome, nor the sequence of the viral vector of the first or second aspect of the invention, nor the sequence of the polynucleotide of the fifth aspect of the invention, nor the sequence of the plasmid of the sixth aspect of the invention.

In a particular embodiment of the eighth aspect, the non-transgenic plant comprises the virus as defined in the third aspect of the invention. In a more particular, the non-transgenic plant comprises the virus as defined in the third aspect of the invention, wherein the virus of the third aspect replicates within the cells of the plant, particularly, wherein the virus of the invention replicates within the cells of the plant and spreads between cells of the plant, particularly between adjacent cells of the plant and/or by the vascular system of the plant. In another particular embodiment, the plant of the eighth aspect of the invention does not comprise heterologous or artificial sequences integrated in its genome, but comprises the virus as defined in the third aspect of the invention. In a more particular, the plant of the eighth aspect of the invention does not comprise heterologous or artificial sequences integrated in its genome, but comprises the virus as defined in the third aspect of the invention, wherein the virus of the third aspect replicates within the cells of the plant, particularly, wherein the virus of the invention replicates within the cells of the plant and spreads between cells of the plant, particularly between adjacent cells of the plant and/or by the vascular system of the plant.

In a particular embodiment, plant of the eighth aspect of the invention is from a genus selected from the list consisting of *Solanum sp, Arabidopsis sp., Nicotiana sp., Pyrus sp, Capsicum sp., Citrullus sp., Cucumis sp., Cucurbita sp., Triticum sp., Oryza sp., Pisum sp., Cicersp., and Citrus sp.* particularly is from a species selected from the list consisting of *Solanum lycopersicum, Arabidopsis thaliana, Nicotiana benthamiana, Pyrus communis L, Solanum tuberosum, Solanum melongena, Capsicum annuum, Cucumis melo, Cucumis sativus, Citrullus lanatus, Cucurbita moschata,* Zea *mays, Triticum aestivum, Triticum turgidum, Oryza sativa, Pisum sativum, Cicer arietinum, Citrus sinensis, Citrus limon, Citrus paradisi and Citrus reticulata.*In another particular embodiment, the plant of the eighth aspect of the invention is selected from the plants disclosed in Barathi et al. (2023) *supra,* particularly in tables 1, 2, 4-7 of Barathi et al. (2023) *supra.*

In a ninth aspect, the invention provides a plant extract from the plant of the eighth aspect of the invention.

In an embodiment of the ninth aspect, the plant extract is from a plant that comprises the virus as defined in the third aspect of the invention, particularly, from an organ of the plant that comprises said virus. In a more particular embodiment, the plant extract is from a plant organ infected with said virus.

In an embodiment, the plant extract is from a plant agroinfiltrated with the bacterium as defined in the seventh aspect, more particularly, from an organ of the plant agroinfiltrated with the bacterium as defined in the seventh aspect. In a particular embodiment, the organ of the plant from which the plant extract is obtained is a leaf of the plant, particularly from a 2-8 weeks old plant, more particularly 4-6 weeks old plant. In another particular embodiment, the organ from which the plant extract is obtained is the apical tissue, particularly the apical meristem of the plant, particularly of a 2-8 weeks old plant, more particularly of a 4-6 weeks old plant.

In another embodiment of the ninth aspect, the plant extract comprises plant tissue and a buffer, particularly in a proportion of plant tissue:buffer in g/ml of 1:50, 1:30, 1:20, 1; 15, 1:10, 1:5, 1:2, or 1:1, particularly of 1:5.

In a particular embodiment, the plant tissue comprised in the plant extract of the ninth aspect is the tissue of the plant organ from which the plant extract is obtained. In another particular embodiment, the buffer comprised in the plant extract of the ninth aspect is a potassium phosphate buffer, particularly 50 mM potassium phosphate, wherein the pH of the phosphate buffer is pH 6-9, particularly pH 8. In an additional embodiment, the buffer further comprises polyvinylpyrrolidone 10, particularly 1% polyvinylpyrrolidone 10, and/or polyethylene glycol 6000, particularly 1% polyethylene glycol 6000. In another particular embodiment, the buffer comprises mercaptoethanol, particularly 10 mM mercaptoethanol. In an additional embodiment, the buffer of the plant extract of the ninth aspect comprises carborundum, particularly 10% (g/l) of carborundum.

In a tenth aspect, the invention provides a method for obtaining the plant as defined in the eighth aspect, the method comprising the step of:
(i) transfecting a plant with the plasmid as defined in the sixth aspect, particularly by agroinfiltration with the bacterium as defined in the seventh aspect, or alternatively,
(ii) contacting the surface of at least one organ of a plant with the virus as defined in the third aspect.

In a particular embodiment, transfecting the plant with the plasmid as defined in the sixth aspect in step (i) of the method, is performed by agroinfiltration with the bacterium as defined in the seventh aspect, particularly with the bacterium of the species *Agrobacterium tumefaciens.*

In a particular embodiment of the tenth aspect, plant transfection in step (i) of the method is performed on at least one leaf of the plant, particularly on at least 4, at least 3, at least 2, at least 1, particularly at least 1 leaf of the plant. In a more particular embodiment of the tenth aspect, plant transfection in step (i) of the method is performed in 5, 4, 3, 2,1 particularly 2 leaves of the plant. In another embodiment of the tenth aspect, the plants transfected with the plasmid of the sixth aspect in step (i), particularly by agroinfiltration with the bacterium of the seventh aspect, are 2-8 weeks old plant, more particularly 4-6 weeks old plant.

In an embodiment of the tenth aspect, contacting the surface of at least one organ of a plant with the virus as defined in the third aspect in step (ii) of the method, comprises applying a composition that comprises said virus on the surface of the at least one organ of the plant. In a particular embodiment, the composition that comprises said virus, comprises the virus defined in the third aspect, and a buffer, particularly potassium phosphate buffer, more particularly 50mM potassium phosphate, wherein the pH of the phosphate buffer is pH 6-9, particularly pH 8. In an additional embodiment, the buffer further comprises polyvinylpyrrolidone 10, particularly 1% polyvinylpyrrolidone 10, and/or polyethylene glycol 6000, particularly 1% polyethylene glycol 6000. In another particular embodiment of the tenth aspect, the buffer of the composition of step (ii) of the method, as defined in any of the embodiments of the present paragraph, comprises mercaptoethanol, particularly 10 mM mercaptoethanol. In another particular embodiment, the buffer of the composition of step (ii) of the method, as defined in any of the embodiments of the present paragraph, comprises carborundum, particularly 10% (g/l) of carborundum.

In a particular embodiment, the composition that comprises the virus as defined in the third aspect of the invention and used in step (ii) of the method of the tenth aspect, is the plant extract as defined in the ninth aspect of the invention.

In another embodiment, the at least one organ of a plant on which the virus is applied in step (ii) of the method of the tenth aspect, as indicated in any of the embodiments of the tenth aspect of the invention, is at least one leaf, particularly, the third true leaf of an at least 1, at least 2, at least 3, at least 4, at least 5, at least 6, particularly at least 3-week-old-plants. More particularly, the at least one organ of a plant is at least one leaf, particularly, the third true leaf of a 1, 2, 3, 4, 5, 6, particularly 3, week-old-plants.

In a particular embodiment of the tenth aspect, the composition used in step (ii) of the method comprising the virus as defined in the third aspect, is applied by spraying on the plants, particularly, on the surface of the plants, more particularly, on the at least one organ of the plant, wherein said organ is as defined in the embodiments just above. In a more particular embodiment, spraying of the composition used in step (ii) of the method of the tenth aspect comprising the virus of the invention is performed on at least one leaf, particularly on at least 2 leaves, more particularly in all said leaves simultaneously. In another particular embodiment, spraying of the composition used in step (ii) of the method of the tenth aspect comprising the virus of the invention in the method of the tenth aspect of the invention is performed by spraying the surface of the plant.

In a particular embodiment, the method of the tenth aspect, particularly the that comprising step (ii) of the method, is for obtaining a non-transgenic plant as defined in the eighth aspect of the invention. In another particular embodiment, the method of tenth aspect, particularly that comprising step (ii) of the method, is for obtaining a plant wherein nor the sequence of the viral vector of the first or second aspect of the invention, nor the sequence of the polynucleotide of the fifth aspect of the invention, the sequence of the plasmid of the sixth aspect of the invention is comprised or integrated in the genome of the plant. Particularly, the method for obtaining the non-transgenic plant or the plant defined in the previous embodiment comprises the steps indicated herein in the tenth aspect.

In a particular embodiment, the plant obtained from the method of the tenth aspect, particularly that comprising step (ii) of the method, is a non-transgenic plant. In another particular embodiment, the plant obtained from the method of the tenth aspect, particularly that comprising step (ii) of the method, does not comprise in its genome or integrated in its genome, nor the sequence of the viral vector of the first or second aspect of the invention, nor the sequence of the polynucleotide of the fifth aspect of the invention, nor the sequence of the plasmid of the sixth aspect of the invention.

As indicated in the eighth aspect of the invention, in a particular embodiment the plant of the invention is resistant to a plant pathogen. Thus, in a particular embodiment of the tenth aspect, the method of tenth aspect is for obtaining a plant resistant to a plant pathogen, particularly wherein the plant is as the plant resistant to a plant pathogen as described in the eighth aspect. Particularly, the method for obtaining a plant resistant to a plant pathogen comprises the steps indicated herein in the tenth aspect. In a particular embodiment, all the embodiments of the plant resistant to a plant pathogen of the eight aspect of the invention apply to the method for obtaining a plant resistant to a plant pathogen of the tenth aspect of the invention. Particularly, the plant pathogen of the method for obtaining a plant resistant to a plant pathogen as defined herein is the plant pathogen as defined in the eighth aspect of the invention.

As indicated in the eight aspect of the invention, in a particular embodiment the plant of the invention is tolerant to a plant pathogen. Thus, in another particular embodiment of the tenth aspect, the method of the tenth aspect is for obtaining a plant tolerant a plant pathogen, particularly wherein the plant is as the plant tolerant to a plant pathogen as described in the eighth aspect. Particularly, the method for obtaining a plant tolerant to a plant pathogen, comprises the steps indicated herein in the tenth aspect. In a particular embodiment, all the embodiments of the plant tolerant to a plant pathogen of the eighth aspect of the invention apply to the method for obtaining a plant tolerant to a plant pathogen of the tenth aspect of the invention. Particularly, the plant pathogen of the method for obtaining a plant tolerant to a plant pathogen as defined herein is the plant pathogen as defined in the eighth aspect of the invention.

As indicated in the eighth aspect of the invention, in a particular embodiment the plant of the invention is a plant with an improved trait, particularly as compared to a control plant. Thus, in another particular embodiment of the tenth aspect, the method is for obtaining a plant with an improved trait, particularly as compared to a control plant, more particularly wherein the plant with the improved trait is as the plant with an improved trait as described in the eighth aspect of the invention. In a particular embodiment, all the embodiments of the plant with an improved trait of the eighth aspect of the invention apply to the method for obtaining a plant with an improved trait of the tenth aspect of the invention.

In a particular embodiment, of the tenth aspect, the method is for obtaining a plant with an improved trait, particularly as compared to a control plant, more particularly wherein the plant with the improved trait is as the plant with an improved trait as described in the eighth aspect of the invention, and resistant to a plant pathogen, particularly wherein the plant is as the plant resistant to one or more plant pathogens as described in the eighth aspect. Particularly, the method for obtaining a plant with an improved trait, particularly as compared to a control plant and resistant to a plant pathogen comprises the steps indicated herein in the tenth aspect. In a particular embodiment, all the embodiments of the plant with an improved trait of the eighth aspect of the invention and all the embodiments of the plant resistant to a plant pathogen of the eighth aspect of the invention apply to the method for obtaining a plant with an improved trait, particularly as compared to a control plant and resistant to a plant pathogen. Particularly, the plant pathogen of the method is the plant pathogen as defined in the eighth aspect of the invention.

In an eleventh aspect, the invention provides a method for obtaining the plant extract according to the ninth aspect that comprises the steps of:
(i) Grinding to powder at least one organ or a section thereof of the plant as defined in the eighth aspect, and
(ii) Homogenizing the powder obtained in step (i) in a buffer.

In a particular embodiment, the plant organ and the plant are as defined in the ninth aspect of the invention. In a particular embodiment, the plant organ or section thereof of step (i) of the method of the eleventh aspect comprises the virus as defined in the third aspect of the invention.

The term "grinding to powder", as used herein refers to reducing to powder or small fragments by friction. Methods to reduce to powder an organ of a plant are well-known by an expert in the field, and include, for instance freezing in liquid nitrogen the plant organ, and milling the obtained product to powder.

In a particular embodiment of the eleventh aspect, the buffer of step (ii) comprises potassium phosphate buffer, particularly 50mM potassium phosphate, wherein the pH of the phosphate buffer is pH 6-9, particularly pH8. In an additional embodiment, the buffer further comprises polyvinylpyrrolidone 10, particularly 1% polyvinylpyrrolidone 10, and/or polyethylene glycol 6000, particularly 1% polyethylene glycol 6000. In another particular embodiment, the buffer of the composition of step (ii) of the method of the eleventh aspect, as defined in any of the embodiments of the present paragraph comprises mercaptoethanol, particularly 10 mM mercaptoethanol. In another particular embodiment, the buffer of the composition of step (ii) of the method of the eleventh aspect, as defined in any of the embodiments of the present paragraph, comprises carborundum, particularly 10% (g/l) of carborundum.

In a particular embodiment, the method for obtaining the plant extract according to the ninth aspect that comprises the steps of:
(i) Grinding to powder at least one organ or a section thereof of the plant as defined in the eighth aspect, particularly at least 1 g of tissue from said at least one organ and
(ii) Homogenizing the powder obtained in step (i) in a buffer, particularly in a proportion of plant tissue:buffer in g/ml of 1:50, 1:30, 1:20, 1; 15, 1:10, 1:5, 1:2, or 1:1, particularly of 1:5.

In an additional embodiment, the method of the eleventh aspect comprises a step (iii) of filtrating the product obtained from step (ii), particularly with sterile filter paper.

### VI- Additional aspects of the invention

In a twelfth aspect, the invention provides a kit of parts comprising the viral vector as defined in the first or second aspect, the virus as defined in the third aspect, the polynucleotide as defined the fourth or fifth aspect, and/or the plasmid as defined in the sixth aspect, particularly, wherein the kit further comprises instructions for its use.

In a thirteenth aspect, the invention provides a device comprising the virus as defined in the third aspect, the bacterium as defined in the seventh aspect, or the plant extract as defined in the ninth aspect. In a particular embodiment, the device is selected from the list consisting of a syringe, a sample vial, a tube, a bag, a tissue, and a bottle.

In a fourteenth aspect, the invention provides a method for obtaining the viral vector as defined in the first or second aspect that comprises:
iv) Transfecting an organ of a plant with the plasmid as defined in sixth aspect, particularly by agroinfiltration with the bacterium as defined in the seventh aspect,
v) Allowing the plant to grow for at least 1 week, and
vi) Isolating the viral vector comprised in the organ transfected in step (i), from the plant obtained from step (ii).

In a particular embodiment of the fourteenth aspect, step (i) of the fourteenth aspect is performed as indicated in any of the embodiments of the tenth aspect for step (i) of the method of the tenth aspect. In another embodiment, the plant is allowed to grow in step (ii) of the method of the fourteenth aspect for at least 1 week, at least 2 weeks, at least 3, weeks, at least 4 weeks, at least 5 weeks, at least 6 weeks, particularly at least 2 weeks. In a more particular embodiment, the plant is allowed to grow in step (ii) of the method of the fourteenth aspect for 1-5, 2-4, 2-3 weeks, particularly, 2-3 weeks. In another embodiment, the organ of the plant is any organ as defined in the tenth aspect of the invention, particularly the apical tissue of the plant, more particularly the apical meristem of the plant.

In a fifteenth aspect, the invention provides a method for obtaining the virus as defined in the third aspect that comprises:
i) Transfecting an organ of a plant with the plasmid as defined in sixth aspect, particularly by agroinfiltration with the bacterium as defined in the seventh aspect,
ii) Allowing the plant to grow for at least 1 week, and
iii) Isolating the viral vector comprised in the organ transfected in step (i), from the plant obtained from step (ii).

In a particular embodiment, the embodiments provided above for steps (i), (ii) and (iii) of the method of the fourteenth aspect, apply to steps (i), (ii) and (iii), respectively, of the method of the fifteenth aspect.

In a sixteenth aspect, the invention provides a method for protecting a plant from a plant pathogen, the method comprising:
(i) transfecting a plant with the plasmid as defined in the sixth aspect, particularly by agroinfiltration with the bacterium as defined in the seventh aspect, or alternatively,
(ii) contacting the surface of at least one organ of a plant with the virus as defined in the third aspect.

In a particular embodiment, the definitions and embodiments of the eighth aspect and of the tenth aspect apply to the sixteenth aspect. In a particular embodiment, step (i) of the method of the sixteenth aspect is performed as step (i) of the method of the tenth aspect. In another particular embodiment, step (ii) of the method of the sixteenth aspect is performed as step (ii) of the method of the tenth aspect.

In a seventeenth aspect, the invention provides a method for the treatment of a plant pathogen disease in a plant, the method comprising:
(i) transfecting a plant with the plasmid as defined in the sixth aspect, particularly by agroinfiltration with the bacterium as defined in the seventh aspect, or alternatively,
(ii) contacting the surface of at least one organ of a plant with the virus as defined in the third aspect.

In a particular embodiment of the seventeenth aspect, contacting the surface of at least one organ of a plant with the virus as defined in the third aspect in step (ii) of the method, comprises applying a composition that comprises said virus on the surface of the at least one organ of the plant. In a particular embodiment, the composition comprising the virus is the plant extract as defined in the ninth aspect of the invention.

The term "treating a plant pathogen disease", as used herein, refers to reducing the concentration of a plant pathogen in a plant, particularly, in a cell of a plant, or at least reducing the symptoms and the damage caused by a plant pathogen in a plant. In an embodiment, the embodiments and definitions provided in the eighth aspect of the invention apply to the seventeenth aspect of the invention. In a particular embodiment, the embodiments and definitions provided in the eighth aspect of the invention for the plant resistant to a plant pathogen and to the plant tolerant to plant pathogen, apply to the seventeenth aspect of the invention. In a more particular embodiment, treating a plant pathogen disease comprises reducing the concentration of plant pathogen in a plant, particularly in an organ of the plant exposed to the plant pathogen, more particularly in the cells of said organ, in a percentage as that indicated in the eighth aspect for the plant resistant to a plant pathogen. In another particular embodiment, treating a plant pathogen disease comprises reducing the level of symptoms associated to a plant pathogen in a plant, in a percentage as that indicated in the eighth aspect for the plant tolerant to a plant pathogen, even when maintaining the plant pathogen concentration in the plant organ exposed to said pathogen in a percentage a that indicated in the eighth aspect for the plant tolerant to a plant pathogen. The definitions and embodiments of the tenth aspect also apply to the method of the seventeenth aspect.

In a particular embodiment, step (i) of the method of the seventeenth aspect is performed as step (i) of the method of the tenth aspect. In another particular embodiment, step (ii) of the method of the seventeenth aspect is performed as step (ii) of the method of the tenth aspect.

In an eighteenth aspect, the invention provides a method for improving a trait of a plant, the method comprising:
(i) transfecting a plant with the plasmid as defined in the sixth aspect, particularly by agroinfiltration with the bacterium as defined in the seventh aspect, or alternatively,
(ii) contacting the surface of at least one organ of a plant with the virus as defined in the third aspect

In a particular embodiment of the eighteenth aspect, contacting the surface of at least one organ of a plant with the virus as defined in the third aspect in step (ii) of the method, comprises applying a composition that comprises said virus on the surface of the at least one organ of the plant. In a particular embodiment, the composition comprising the virus is the plant extract as defined in the ninth aspect of the invention.

In a particular embodiment, the plant trait improved with the method of the eighteenth aspect of the invention is the as the improved trait indicated in the eighth aspect of the invention for the plant with an improved trait. In another particular embodiment, the trait improved with the method of the eighteenth aspect of the invention is selected from the improved traits indicated in the eight aspect of the invention, particularly is selected from the list consisting of crop yield, shelf life of fruits, shelf life of vegetables, nutritional value, organoleptic properties, abiotic stress resistance (for instance, to drought, salinity, high temperatures, or cold), or mal sterility. In a particular embodiment, the trait improved with the method of the eighteenth aspect of the invention is selected from those disclosed in tables 1 and in table 7 of Barathi et al., (2023), *supra.*

In a particular embodiment of the eighteenth aspect, improving a trait of a plant comprises, particularly consists of, increasing the level of said trait in the plant in at least 10%, 20%, 30%, 40%, 50%, 75%, 90%, 95%, 100%, 150%, 200%, 250%, 300%, 350%, 400%, particularly in at least 20%, as compared to a control plant. In a more particular embodiment of the eighteenth aspect, improving a trait of a plant comprises, particularly consists of, increasing the level of said in the plant in at least 50% as compared to a control plant. In a yet more preferred embodiment of the eighteenth aspect, improving a trait of a plant comprises, particularly consists of, increasing the level of said in the plant in at least 100%, as compared to a control plant.

In a particular embodiment, the control plant is as the control plant indicated in the eighth aspect of the invention for the plant with an improved trait as compared to a control plant.

In a particular embodiment, the embodiments of the eighth and tenth aspect of the invention apply to the eighteenth aspect of the invention.

In a particular embodiment, step (i) of the method of the eighteenth aspect is performed as step (i) of the method of the tenth aspect. In another particular embodiment, step (ii) of the method of the eighteenth aspect is performed as step (ii) of the method of the tenth aspect.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention.

The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

### Examples

### Example 1 - Materials and methods

### Plant materials and growth conditions

*Nicotiana benthamiana* and *Arabidopsis thaliana* Col-0 plants were grown in a growth chamber at 25°C with a 12 h-light/12 h-dark photoperiod or at 22°C with a 16 h-light/8 h-dark photoperiod, respectively. Arabidopsis Col-0 plants were genetically transformed using the floral dip method (Clough,S.J. et al., (1998) Floral dip: a simplified method for Agrobacterium-mediated transformation of Arabidopsis thaliana". Plant J, 16, 735-43) with the *Agrobacterium tumefaciens* GV3101 strain (for instance, from Goldbiotechnology^{®}). T1 transgenic Arabidopsis were grown as previously (L6pez-Dolz,L., Spada,M. et al., (2020) Fine-tune control of targeted RNAi efficacy by plant artificial small RNAs. Nucleic Acids Res, 48, 6234-6250) . Photographs were taken with a Nikon D3000 digital camera with AF-S DX NIKKOR 18-55 mm f/3.5-5.6G VR lens.

### Plant phenotyping

Plant phenotyping analyses were conducted in blind as described (Lopez-DoIz,L., et al., supra). Briefly, the flowering time of each independent line is the number of days elapsed from seed plating to first bud opening (or "days to flowering"). The "CH42" phenotype was scored in 10 days old seedlings and was reported as "weak", "intermediate" or "severe" if seedlings had more than two leaves, exactly two leaves or no leaves at all (only two cotyledons), respectively.

### Artificial small RNA design

AmiR-GUS_{Nb}, amiR-GUS_{At}, amiR-NbSu, and amiR-AtCH42 amiRNA design was described before (Cisneros, A.E., et al., (2022) Systemic silencing of an endogenous plant gene by two classes of mobile 21-nucleotide artificial small RNAs. Plant J, 110, 1166-1181; L6pez-Dolz, L., et al., supra; Schwab,R., et al., (2006) Highly specific gene silencing by artificial microRNAs in Arabidopsis. Plant Cell, 18, 1121-33).

P-SAMS script (https://github.com/carringtonlab/p-sams) (Fahlgren,N., et al., (2016) P-SAMS: a web site for plant artificial microRNA and synthetic trans-acting small interfering RNA design. Bioinformatics, 32, 157-8) returning unlimited optimal results was used to obtain the complete list of optimal amiRNAs targeting *NbDXS* with high specificity. The off-targeting filtering in *N. benthamiana* transcriptome v5.1 (Nakasugi,K., et al., (2014) Combining transcriptome assemblies from multiple de novo assemblers in the allo-tetraploid plant Nicotiana benthamiana. PLoS One, 9, e91776) was enabled in the design to increase amiRNA specificity.

### Design of the amiRNAs downstream the BS region in BS-AtMIR390a-Bsal/ccdB-based ('B/c') vectors.

### A- Selection of the amiRNA sequence

It was used the amiRNA Designer app from the P-SAMS webtool at http://p-sams.carringtonlab.org/amirna/designer.

### B- Design of amiRNA oligonucleotides

It was used the amiRNA Designer app from the P-SAMS webtool at http://p-sams.carringtonlab.org/amirna/designer.

### B. 1 Sequence of the BS-AtMIR390a cassette containing the amiRNA

The following FASTA sequence includes amiRNA/amiRNA* sequences inserted in the AtMIR390a precursor sequence downstream the BS region:
- amiRNA in *BS-AtMIR390a:*

AGTAGAGAAGAATCTGTAX1X2X3X4X5X6X7X8X9X10X11X12X13X14X15X16X17X18X19X20X21CGAAATCAAACTX1X
2X1X2X3X4X5X6X7X8X9X10X11X12X13X14X15X16X17X18X19CATTGGCTCTTCTTACT (SEQ ID NO. 41)

Where:
- ***X (bold and italics)*** is a DNA base of the amiRNA sequence, and the subscript number is the base position in the amiRNA 21-mer
- *X (regular and italics)* is a DNA base of the amiRNA* sequence, and the subscript number is the base position in the amiRNA* 21-mer
- **X (bold)** is a DNA base of the BS region of the AtMIR390a precursor
- X (regular) is a DNA base of the OsMIR390 precursor included in the oligonucleotides required to clone the amiRNA insert in B/c vectors
- **X (bold underlined)**is a DNA base of the AtMIR390a precursor included in the oligonucleotides required to clone the amiRNA insert in B/c vectors
- X (regular underlined) is a DNA base of the OsMIR390a precursor that may be modified to preserve the authentic *AtMIR390a* duplex structure

In the sequence above:
- The amiRNA sequence with the following characteristics is inserted:
   ***X₁X₂X₃X₄X₅X₆X₇X₈X₉X₁₀X₁₁X₁₂X₁₃X₁₄X₁₅X₁₆X₁₇X₁₈X₁₉X₂₀X₂₁***
- The amiRNA* sequence that has to verify the following base-pairing in inserted:

X1 X2 X3 X4 X5 X6 X7 X8 X9 X10X11X12X13X14X15X16X17X18 X19 X20X21

| | | | | | | | | | | | | | | | | | | |

X19X18X17X16X15X14X13X12X11X10X9 X8 X7 X6 X5 X4 X3 X2 X1 X2 X1

Note that:
- In general, ***X₁*=T** for amiRNA association with AGO1. In this case, *X₁₉*=**A**
- Bases ***X₁₁* (from the amiRNA sequence)** and *X₉ (from the amiRNA* sequence)* DO NOT base-pair to preserve the central bulge of the authentic AtMIR390a duplex. The following base-pair rule applies:
   - If ***X₁₁ (amiRNA sequence)***=G***,*** then *X₉ (amiRNA* sequence)*=A
   - If ***X₁₁ (amiRNA sequence)***=C***,*** then *X₉ (amiRNA* sequence)*=T
   - If ***X₁₁ (amiRNA sequence)***=A*,* then *X₉ (amiRNA* sequence)*=G
   - If ***X₁₁ (amiRNA sequence**)*=U*,* then *X₉(amiRNA* sequence)*=C

### B-.2- Sequence of the amiRNA oligonucleotides

The sequences of the two amiRNA oligonucleotides are:
- Forward oligonucleotide (58 b), 
TGTAX1X2X3X4X5X6X7X8X9X10X11X12X13X14X15X16X17X18X19X20X21CGAAATCAAACTX1X2X1X2X3X4X5X6X7X8X9X
10X11X12X13X14X15X16X17X18X19 (SEQ ID NO. 169)
- Reverse oligonucleotide (58 b),

AATGY19Y18Y17Y16Y15Y14Y13Y12Y11Y10Y9Y8Y7Y6Y5Y4Y3Y2Y1Y2Y1AGTTTGATTTCGY21Y20Y19Y18Y17Y16Y15Y14Y13
Y12Y11Y10Y9Y8Y7Y6Y5Y4Y3Y2Y1 (SEQ ID NO: 170)

Where:
- *X₁X₂X₃X₄X₅X₆X₇X₈X₉X₁₀X₁₁X₁₂X₁₃X₁₄X₁₅X₁₆X₁₇X₁₈X₁₉X₂₀X₂₁*=amiRNA. sequence
- *X₁X₂X₃X₄X₅X₆X₇X₈X₉X₁₀X₁₁X₁₂X₁₃X₁₄X₁₅X₁₆X₁₇X₁₈X₁₉*=partial amiRNA* sequence
- *Y₂₁Y₂₀Y₁₉Y₁₈Y₁₇Y₁₆Y₁₅Y₁₄Y₁₃Y₁₂Y11Y₁₀Y₉Y₈Y₇Y₆Y₅Y₄Y₃Y₂Y₁*=amiRNA reverse-complement sequence
- *TGY₁₉Y₁₈Y₁₇Y₁₆Y₁₅Y₁₄Y₁₃Y₁₂Y₁₁Y₁₀Y₉Y₈Y₇Y₆Y₅Y₄Y₃Y₂Y₁*=amiRNA* reverse-complement sequence
- X₁X₂ = OsMIR390 sequence that may be modified to preserve authentic OsMIR390a duplex structure.
- Y₂Y₁ = reverse-complement of X₁X₂

### Example:

The sequences of the two oligonucleotides to clone the amiRNA 'amiR-NbSu' (TCCCATTCGATACTGCTCGCC) (SEQ ID NO. 171) are:
- Sense oligonucleotide (58 b):
   **TGTA*TAACCGTGGTGGACTTCCCGC***CGAAATCAAACTGCGGGAAGTCAACCACGGTTA
- Antisense oligonucleotide (58 b), (SEQ ID NO: 172)
   **AA***TGTAACCGTGGTTGACTTCCC*GCAGTTTGATTTCG***GCGGGAAGTCCACCACGGTTA (SEQ ID NO. 173)***

**Note:** *the* 58 *b long oligonucleotides can be ordered desalted, no purification is required.*

### Design of the dsDNA amiRNA insert to generate PVX-based amiRNA constructs (she precursor)

A dsDNA (129 bp, eg. ultramer duplex in IDT) is designed including the sequences of the amiRNA/amiRNA* inserted into the *shc* (MIR390-based) precursor, as follows:

agaggtcagcaccagctagcAGTAGAGAAGAATCTGTAX1X2X3X4X5X6X7X8X9X10X11X12X13X14X15X16X17X18X19X20X2
1CGAAATCAAACTX1X2X1X2X3X4X5X6X7X8X9X10X11X12X13X14X15X16X17X18X19CATTGGCTCTTCTTACTagggtttg
ttaagtttccct (SEQ ID NO: 174)

Where:
- ***X (bold and italics)*** is a DNA base of the amiRNA sequence, and the subscript number is the base position in the amiRNA 21-mer
- *X (regular and italics)* is a DNA base of the amiRNA* sequence, and the subscript number is the base position in the amiRNA* 21-mer
- **X (bold)** is a DNA base of the BS region of the AtMIR390a precursor
- X (regular) is a DNA base of the OsMIR390 precursor included in the oligonucleotides required to clone the amiRNA insert in B/c vectors
- X (regular underlined) is a DNA base of the OsMIR390a precursor that may be modified to preserve the authentic AtMIR390a duplex structure
- x (lower cases) is a DNA base of the PVX sequence, required for Gibson-based assembly

In the sequence above, the amiRNA sequence and the base-pairing rule that has to verify the amiRNA* sequence areas are those indicated above for the design of the amiRNAs downstream the BS region in amiRNA in BS-AtMIR390a (see section B. 1).

### Design of the dsDNA amiRNA insert to generate TRV-based amiRNA constructs (shc precursor).

A dsDNA (129 bp, eg. ultramer duplex in IDT) is designed, including the sequences of the amiRNA/amiRNA* inserted into the shc (MIR390-based) precursor, as follows:

cacttacccgagttaacgccAGTAGAGAAGAATCTGTAX1X2X3X4X5X6X7X8X9X10X11X12X13X14X15X16X17X18X19X20X21
CGAAATCAAACTX1X2X1X2X3X4X5X6X7X8X9X10X11X12X13X14X15X16X17X18X19CATTGGCTCTTCTTACTatgtcccg
aagacattaaac (SEQ ID NO: 175)

Where:
- ***X (bold and itallics*)** is a DNA base of the amiRNA sequence, and the subscript number is the base position in the amiRNA 21-mer
- *X (regular and itallics)* is a DNA base of the amiRNA* sequence, and the subscript number is the base position in the amiRNA* 21-mer
- **X (bold)** is a DNA base of the BS region of the *AtMIR390a* precursor
- X (regular) is a DNA base of the *OsMIR390* precursor included in the oligonucleotides required to clone the amiRNA insert in B/c vectors
- X (regular underlined) is a DNA base of the OsMIR390a precursor that may be modified to preserve the authentic AtMIR390a duplex structure
- x (lower cases) is a DNA base of the TRV sequence, required for Gibson-based assembly

In the sequence above, the amiRNA sequence and the base-pairing rule that has to verify the amiRNA* sequence areas are those indicated above for the design of the amiRNAs downstream the BS region in amiRNA in BS-AtMIR390a (see section B.1).

### DNA constructs

Oligonucleotides AC-627 and AC-628 were annealed and ligated in to *pENTR-D-TOPO* to generate *pENTR-BS-AtMIR390a-BB* including the *BS* sequence interrupted by two inverted *Bsa*I restriction sites. The *B*/*c* cassette was excised from *Bsa*I-digested *pENTR-AtMIR390a-B*/*c* (Addgene plasmid #51778), (Carbonell,A., et al., (2014) New generation of artificial MicroRNA and synthetic trans-acting small interfering RNA vectors for efficient gene silencing in Arabidopsis. Plant Physiol, 165, 15-29) and ligated into *Bsa*I-digested *pENTR-BS-AtMIR390a-BB* to generated *pENTR-BS-AtMIR390a-B*/*c.* The *BS-AtMIR390a-BB* cassette from *pENTR-AtMIR390a-BB* was transferred by LR recombination into *pMDC32B* (Carbonell, A., et al., (2014) supra), a version of *pMDC32* (Curtis,M.D. and Grossniklaus,U. (2003) A gateway cloning vector set for high-throughput functional analysis of genes in planta. Plant Physiol, 133, 462-9) with mutated *Bsa*I site, to generate *pMDC32B-BS-AtMIR390a-BB.* Finally, the *B*/*c* cassette was excised from *Bsa*I-digested *pENTR-AtMIR390a-B*/*c* and ligated into *Bsa*l-digested *pMDC32B-BS-AtMIR390a-BB* to generate *pMDC32B-BS-AtMIR390a-B*/*c.* AmiRNA *pENTR-BS-AtMIR390a-B*/*c* (Addgene plasmid 199559) and *pMDC32B-BS-AtMIR390a-B*/*c* vectors (Addgene plasmid 199560) are available from Addgene at http://www.addqene.orq/.

Constructs *35S:AtDSL-D6-amiR-NbSu, 35S:AtDSL-D13-amiR-NbSu, 35S:AtDSL-D21-amiR-NbSu, 35S:AtDSL-D25-amiR-NbSu, 35S:pri-amiR-NbDXS, 35S:AtDSL-D6-amiR-NbDXS, 35S:AtDSL-D13-amiR-NbDXS, 35S:AtDSL-D21-amiR-NbDXS, 35S:AtDSL-D25-amiR-NbDXS, 35S:OsDSL-amiR-NbSu, 35S:OsDSL-D2-amiR-NbSu, 35S:OsDSL-D4-amiR-NbSu, 35S:OsDSL-D6-amiR-NbSu, 35S:OsDS-AtL-amiR-NbSu, 35S:OsDSL-amiR-NbDXS, 35S:OsDSL-D2-amiR-NbDXS, 35S:OsDSL-D4-amiR-NbDXS, 35S:OsDSL-D6-amiR-NbDXS, 35S:OsDS-AtL-amiR-NbDXS* were obtained by ligating annealed oligonucleotide pairs listed in Table 1 into *pMDC32B-AtMIR390a-B*/*c* (Addgene plasmid 51776) as described (Carbonell,A., et al., (2014) supra). BS cassettes from *35S:pri-amiR-NbSu* and *35S:pri-amiR-NbDXS* were amplified with oligo pairs AC-335/AC336, inserted into *pENTR-D-TOPO* and transferred by LR recombination into *pMDC32B* to generate *35S:BS-amiR-NbSu* and *35S:BS-amiR-NbDXS,* respectively.

**Table 1 Name, sequence and use of DNA oligonucleotides used in this study.**

| **Oligonucleotide** | **Sequence** | **Construct/Aim** | **SEQ ID NO.** |
|---|---|---|---|
| AC-55 | AGGGGCCATGCTAATCTTCTC | DNA probe for U6 detection | 67 |
| AC-157 | GGCCTCTTCCTTTATAACCAA | DNA probe for amiR-AtFT detection | 68 |
| AC-158 | AGGGATTTCCGTGACACTTAA | DNA probe for amiR-AtCH42 detection | 69 |
| AC-159 | AAAAATGGCTGAGGCTGATGA | qPCR amplification of *AtACT2* mRNA | 70 |
| AC-160 | GAAAAACAGCCCTGGGAGC | | 71 |
| AC-163 | CATGCACAAGTAGGGACGGTT | qPCR amplification of *AtCH42* mRNA | 72 |
| AC-164 | GTCACGGAAATCCTTTGGGTT | | 73 |
| AC-169 | TGGAACAACCTTTGGCAATG | qPCR amplification of *AtFT* mRNA | 74 |
| AC-170 | CGACACGATGAATTCCTGCA | | 75 |
| AC-251 | TGTATAAACCGCGGGTTCCTAACAGAT GATGATCACATTCGTTATCTATTTTTTC TGTTAGGAAACCGCGGTTTA | *35S:pri-amiR-NbDXS-1* (*35S:pri-amiR-NbDXS*) | 76 |
| AC-252 | AATGTAAACCGCGGTTTCCTAACAGAA AAAATAGATAACGAATGTGATCATCATC TGTTAGGAACCCGCGGTTTA | | 77 |
| AC-253 | TGTATCATAACCTCTAGAGCTTCTGATG ATGATCACATTCGTTATCTATTTTTTCA GAAGCTCTCGAGGTTATGA | *35S:pri-amiR-NbDXS-2* | 78 |
| AC-254 | AATGTCATAACCTCGAGAGCTTCTGAA AAAATAGATAACGAATGTGATCATCATC AGAAGCTCTAGAGGTTATGA | | 79 |
| AC-255 | TGTATTCTGCAATTAAAGCCTCCGGAT GATGATCACATTCGTTATCTATTTTTTC CGGAGGCTTGAATTGCAGAA | *35S:pri-amiR-NbDXS-3* | *80* |
| AC-256 | AATGTTCTGCAATTCAAGCCTCCGGAA AAAATAGATAACGAATGTGATCATCATC CGGAGGCTTTAATTGCAGAA | | 81 |
| AC-270 | CTGTTAGGAACCCGCGGTTTA | DNA probe to detect amiR-NbDXS-1 | 82 |
| AC-271 | CAGAAGCTCTAGAGGTTATGA | DNA probe to detect amiR-NbDXS-2 | 83 |
| AC-272 | CCGGAGGCTTTAATTGCAGAA | DNA probe to detect amiR-NbDXS-3 | 84 |
| AC-335 | CACCAGTAGAGAAGAATCTGTA | *pENTR-BS-amiR-NbSu*/*pMDC32B-BS-amiR-NbSu*/ | 85 |
| AC-336 | AGTAAGAAGAGCCAATGT | | 86 |
| | | *pENTR-BS-amiR-NbDXS*/*pMDC32B-BS-amiR-NbDXS* | |
| AC-355 | GACCCTGATGTTGATGTTCGCT | qPCR amplification of *NbSu* mRNA | 87 |
| AC-356 | GAGGGATTTGAAGAGAGATTTC | | 88 |
| AC-359 | GGTGGTGGGACTGGTATGAA | qPCR amplification of *NbDXS* mRNA | 89 |
| AC-360 | GCAAATCTCACTGGCAGCTT | | 90 |
| AC-365 | GACCCTGATGTTGATGTTCGCT | PCR&qPCR amplification of *NbPP2A* mRNA | 91 |
| AC-366 | GAGGGATTTGAAGAGAGATTTC | | 92 |
| AC-416 | A+GGA+CAC+AAT+CAC+GTC+TTA+CA | LNA probe for amiR-TSWV detection | 93 |
| AC-417 | G+CGG+GAA+GTC+CAC+CAC+GGT+TA | LNA probe for amiR-NbSu detection | 94 |
| AC-418 | C+TGT+TAG+GAA+CCC+GCG+GTT+TA | LNA probe for amiR-NbDXS detection | 95 |
| AC-484 | TGTATAACCGTGGTGGACTTCCCGCTC GAAATCAAACTAGCGGGAAGTCAACCA CGGTTA | *35S:OsDSL-amiR-NbSu* | *96* |
| AC-485 | AATGTAACCGTGGTTGACTTCCCGCTA GTTTGATTTCGAGCGGGAAGTCCACCA CGGTTA | | *97* |
| AC-486 | TGTATAACCGTGGTGGACTTCCCGCCG AAATCAAACTGCGGGAAGTCAACCACG GTTA | *35S:OsDSL-D2-amiR-NbSul 35S:shc-amiR-NbSu* | *98* |
| AC-487 | AATGTAACCGTGGTTGACTTCCCGCAG TTTGATTTCGGCGGGAAGTCCACCACG GTTA | | *99* |
| AC-488 | TGTATAACCGTGGTGGACTTCCCGCGA AATCAAACGCGGGAAGTCAACCACGGT TA | *35S:OsDSL-D4-amiR-NbSu* | *100* |
| AC-489 | AATGTAACCGTGGTTGACTTCCCGCGT TTGATTTCGCGGGAAGTCCACCACGGT TA | | *101* |
| AC-490 | TGTATAACCGTGGTGGACTTCCCGCAA ATCAAAGCGGGAAGTCAACCACGGTTA | *35S:OsDSL-D6-amiR-NbSu* | *102* |
| AC-491 | AATGTAACCGTGGTTGACTTCCCGCTT TGATTTGCGGGAAGTCCACCACGGTTA | | *103* |
| AC-492 | TGTATAACCGTGGTGGACTTCCCGCTC | *35S:OsDS-AtL-amiR-NbSu* | *104* |
| | GATTCCTAGCGGGAAGTCAACCACGGT TA | | |
| AC-493 | AATGTAACCGTGGTTGACTTCCCGCTA GGAATCGAGCGGGAAGTCCACCACGG TTA | | *105* |
| AC-494 | TGTATAACCGTGGTGGACTTCCCGCGA TGATCACATTCGTTATCTATTGCGGGAA GTCAACCACGGTTA | *35S:AtDSL-D6-amiR-NbSu* | *106* |
| AC-495 | AATGTAACCGTGGTTGACTTCCCGCAA TAGATAACGAATGTGATCATCGCGGGA AGTCCACCACGGTTA | | 107 |
| AC-496 | TGTATAACCGTGGTGGACTTCCCGCGA TCACATTCGTTATCGCGGGAAGTCAAC CACGGTTA | *35S:AtDSL-D13-amiR-NbSu* | *108* |
| AC-497 | AATGTAACCGTGGTTGACTTCCCGCGA TAACGAATGTGATCGCGGGAAGTCCAC CACGGTTA | | *109* |
| AC-498 | TGTATAACCGTGGTGGACTTCCCGCAC ATTCGTGCGGGAAGTCAACCACGGTTA | *35S:AtDSL-D21-amiR-NbSu* | 110 |
| AC-499 | AATGTAACCGTGGTTGACTTCCCGCAC GAATGTGCGGGAAGTCCACCACGGTTA | | 111 |
| AC-500 | TGTATAACCGTGGTGGACTTCCCGCAT TCGCGGGAAGTCAACCACGGTTA | *35S:AtDSL-D25-amiR-NbSu* | 112 |
| AC-501 | AATGTAACCGTGGTTGACTTCCCGCGA ATGCGGGAAGTCCACCACGGTTA | | *113* |
| AC-558 | CACCGAGAAGAATCTGTATAACCGTGG TGGACTTCCCGCATGATGATCACATTC GTTATCTATTTTTTGCGGGAAGTCAACC ACGGTTACATTGGCTCTTCTT | *pENTR-BS-D7-amiR-NbSul 35S:BS-D7-amiR-NbSu* | 114 |
| | AAGAAGAGCCAATGTAACCGTGGTTGA CTTCCCGCAAAAAATAGATAACGAATG TGATCATCATGCGGGAAGTCCACCACG GTTATACAGATTCTTCTCGGTG | | *115* |
| AC-559 | CACCGAATCTGTATAACCGTGGTGGAC TTCCCGCATGATGATCACATTCGTTATC TATTTTTTGCGGGAAGTCAACCACGGT TACATTGGCTC | *pENTR-BS-D17-amiR-NbSul 35S:BS-D17-amiR-NbSu* | *116* |
| | GAGCCAATGTAACCGTGGTTGACTTCC | | 117 |
| | CGCAAAAAATAGATAACGAATGTGATC ATCATGCGGGAAGTCCACCACGGTTAT ACAGATTCGGTG | | |
| AC-560 | CACCTCTGTATAACCGTGGTGGACTTC CCGCATGATGATCACATTCGTTATCTAT TTTTTGCGGGAAGTCAACCACGGTTAC ATTGG | *pENTR-BS-D23-amiR-NbSul 35S:BS-D23-amiR-NbSu* | *118* |
| | CCAATGTAACCGTGGTTGACTTCCCGC AAAAAATAGATAACGAATGTGATCATCA TGCGGGAAGTCCACCACGGTTATACAG AGGTG | | *119* |
| AC-561 | CACCTATAACCGTGGTGGACTTCCCGC ATGATGATCACATTCGTTATCTATTTTTT GCGGGAAGTCAACCACGGTTACA | *pENTR-BS-D31-amiR-NbSul 35S:BS-D31-amiR-NbSu* | 120 |
| | TGTAACCGTGGTTGACTTCCCGCAAAA AATAGATAACGAATGTGATCATCATGC GGGAAGTCCACCACGGTTATAGGTG | | 121 |
| AC-593 | TGTATAAACCGCGGGTTCCTAACAGGA TGATCACATTCGTTATCTATTCTGTTAG GAAACCGCGGTTTA | *35S:AtDSL-D6-amiR-NbDXS* | 122 |
| AC-594 | AATGTAAACCGCGGTTTCCTAACAGAA TAGATAACGAATGTGATCATCCTGTTAG GAACCCGCGGTTTA | | *123* |
| AC-595 | TGTATAAACCGCGGGTTCCTAACAGGA TCACATTCGTTATCCTGTTAGGAAACC GCGGTTTA | *35S:AfDSL-D13-amiR-NbDXS* | *124* |
| AC-596 | AATGTAAACCGCGGTTTCCTAACAGGA TAACGAATGTGATCCTGTTAGGAACCC GCGGTTTA | | *125* |
| AC-597 | TGTATAAACCGCGGGTTCCTAACAGAC ATTCGTCTGTTAGGAAACCGCGGTTTA | *35S: AfDSL-D21-amiR-NbDXS* | *126* |
| AC-598 | AATGTAAACCGCGGTTTCCTAACAGAC GAATGTCTGTTAGGAACCCGCGGTTTA | | *127* |
| AC-599 | TGTATAAACCGCGGGTTCCTAACAGAT TCCTGTTAGGAAACCGCGGTTTA | *35S:AtDSL-D25-amiR-NbDXS* | *128* |
| AC-600 | AATGTAAACCGCGGTTTCCTAACAGGA ATCTGTTAGGAACCCGCGGTTTA | | *129* |
| AC-601 | TGTATAAACCGCGGGTTCCTAACAGTC | *35S:OsDSL-amiR-NbDXS* | *130* |
| | GAAATCAAACTACTGTTAGGAAACCGC GGTTTA | | |
| AC-602 | AATGTAAACCGCGGTTTCCTAACAGTA GTTTGATTTCGACTGTTAGGAACCCGC GGTTTA | | *131* |
| AC-603 | TGTATAAACCGCGGGTTCCTAACAGCG AAATCAAACTCTGTTAGGAAACCGCGG TTTA | *35S:OsDSL-D2-amiR-NbDXSI 35S:shc-amiR-NbDXS* | *132* |
| AC-604 | AATGTAAACCGCGGTTTCCTAACAGAG TTTGATTTCGCTGTTAGGAACCCGCGG TTTA | | *133* |
| AC-605 | TGTATAAACCGCGGGTTCCTAACAGGA AATCAAACCTGTTAGGAAACCGCGGTT TA | *35S:OsDSL-D4-amiR-NbDXS* | *134* |
| AC-606 | AATGTAAACCGCGGTTTCCTAACAGGT TTGATTTCCTGTTAGGAACCCGCGGTT TA | | *135* |
| AC-607 | TGTATAAACCGCGGGTTCCTAACAGAA ATCAAACTGTTAGGAAACCGCGGTTTA | *35S:OsDSL-D6-amiR-NbDXS* | *136* |
| AC-608 | AATGTAAACCGCGGTTTCCTAACAGTTT GATTTCTGTTAGGAACCCGCGGTTTA | | *137* |
| AC-609 | TGTATAAACCGCGGGTTCCTAACAGTC GATTCCTACTGTTAGGAAACCGCGGTT TA | *35S: OsDS-AtL-amiR-NbDXS* | *138* |
| AC-610 | AATGTAAACCGCGGTTTCCTAACAGTA GGAATCGACTGTTAGGAACCCGCGGTT TA | | *139* |
| AC-611 | CACCGAGAAGAATCTGTATAAACCGCG GGTTCCTAACAGATGATGATCACATTC GTTATCTATTTTTTCTGTTAGGAAACCG CGGTTTACATTGGCTCTTCTT | *pENTR-BS-D7-amiR-NbDXSI 35S:BS-D7-amiR-NbDXS* | *140* |
| | AAGAAGAGCCAATGTAAACCGCGGTTT CCTAACAGAAAAAATAGATAACGAATGT GATCATCATCTGTTAGGAACCCGCGGT TTATACAGATTCTTCTCGGTG | | *141* |
| AC-612 | CACCGAATCTGTATAAACCGCGGGTTC CTAACAGATGATGATCACATTCGTTATC TATTTTTTCTGTTAGGAAACCGCGGTTT | *pENTR-BS-D17-amiR-NbDXSI 35S:BS-D17-amiR-NbDXS* | *142* |
| | ACATTGGCTC | | |
| | GAGCCAATGTAAACCGCGGTTTCCTAA CAGAAAAAATAGATAACGAATGTGATC ATCATCTGTTAGGAACCCGCGGTTTAT ACAGATTCGGTG | | *143* |
| AC-613 | CACCTCTGTATAAACCGCGGGTTCCTA ACAGATGATGATCACATTCGTTATCTAT TTTTTCTGTTAGGAAACCGCGGTTTACA TTGG | *pENTR-BS-D23-amiR-NbDXSI 35S:BS-D23-amiR-NbDXS* | *144* |
| | CCAATGTAAACCGCGGTTTCCTAACAG AAAAAATAGATAACGAATGTGATCATCA TCTGTTAGGAACCCGCGGTTTATACAG AGGTG | | *145* |
| AC-614 | CACCTATAAACCGCGGGTTCCTAACAG ATGATGATCACATTCGTTATCTATTTTTT CTGTTAGGAAACCGCGGTTTACA | *pENTR-BS-D31-amiR-NbDXSI 35S:BS-D31-amiR-NbDXS* | *146* |
| | TGTAAACCGCGGTTTCCTAACAGAAAA AATAGATAACGAATGTGATCATCATCTG TTAGGAACCCGCGGTTTATAGGTG | | *147* |
| AC-621 | TGTATTGGTTATAAAGGAAGAGGCCCG AAATCAAACTGGCCTCTTCCGTTATAAC CAA | *35S:shc-amiR-AtFT* | *148* |
| AC-622 | AATGTTGGTTATAACGGAAGAGGCCAG TTTGATTTCGGGCCTCTTCCTTTATAAC CAA | | *149* |
| AC-623 | TGTATTAAGTGTCACGGAAATCCCTCG AAATCAAACTAGGGATTTCCTTGACACT TAA | *35S:shc-amiR-AtCH42* | *150* |
| AC-624 | AATGTTAAGTGTCAAGGAAATCCCTAG TTTGATTTCGAGGGATTTCCGTGACAC TTAA | | *151* |
| AC-627 | agtaagaagagccaatgTgagaccGGTCTCTTA CAGATTCTTCTCTACTGGTG | *pENTR-BS-AtMIR390a-BB* | *152* |
| AC-628 | CACCAGTAGAGAAGAATCTGTAAGAGA CCggtctcAcattggctcttcttact | | *153* |
| AC-648 | gaggtcagcaccagctagcaTATAGGGGGAA AAAAAGGTAG | *PVX-pri-amiR-GUS_{Nb}*/ *PVX-pri-amiR-NbSu* | *154* |
| AC-650 | gaggtcagcaccagctagcaGTAGAGAAGAAT | *PVX-shc-amiR-NbSu* | *155* |
| | CTGTA | | |
| AC-654 | GGGAATCAATCACAGTGTTGGC | *amiRNA precursors detection* | *156* |
| AC-655 | GCTACTATGGCACGGGCTGTAC | | *157* |
| AC-657 | ATGTCAGGCCTGTTCACTATCC | *PVX diagnostic* | *158* |
| AC-658 | TGGTGGTGGTAGAGTGACAAC | | *159* |
| AC-662 | gggaaacttaacaaaccctaGAGACTAAAGATG AGATCTAATCTG | *PVX-pri-amiR-GUS_{Nb}*/ *PVX-pri-amiR-NbSu* | *160* |
| AC-663 | gggaaacttaacaaaccctaGTAAGAAGAGCC AA | *PVX-shc-amiR-NbSu* | *161* |
| AC-672 | TGTATGTAAGACGTGATTGTGTCCTCG AAATCAAACTAGGACACAATAACGTCTT ACA | *35S: shc-amiR-TSWV* | *162* |
| AC-673 | AATGTGTAAGACGTTATTGTGTCCTAGT TTGATTTCGAGGACACAATCACGTCTTA CA | | *163* |
| D2065 | TGTATAACCGTGGTGGACTTCCCGCAT GATGATCACATTCGTTATCTATTTTTTG CGGGAAGTCAACCACGGTTA | *35S:BS-amiR-NbSu* | *164* |
| D2066 | AATGTAACCGTGGTTGACTTCCCGCAA AAAATAGATAACGAATGTGATCATCATG CGGGAAGTCCACCACGGTTA | | 165 |
| AC-617 | cacttacccgagttaacgccAGTAGAGAAG AATCTGTA | TRV-based amiRNA constructs (shc precursor) | 166 |
| AC-618 | gtttaatgtcttcgggacatAGTAAGAAGA GCCAA | TRV-based amiRNA constructs (shc precursor) | 167 |

DsDNA oligonucleotides AC-558, AC-559, AC-560, AC-561, AC-611, AC-612, AC-613 and AC-14 were ligated into *pENTR-D-TOPO* to generate *pENTR-BS-D7-amiR-NbSu, pENTR-BS-D17-amíR-NbSu, pENTR-BS-D23-amiR-NbSu, pENTR-BS-D31-amiR-NbSu, pENTR-BS-D7-amiR-NbDXS, pENTR-BS-D17-amiR-NbDXS, pENTR-BS-D23-amiR-NbDXS,* and *pENTR-BS-D31-amiR-NbDXS,* respectively. *BS* cassettes were transferred by LR recombination to *pMDC32B* to generate *35S:BS-D7-amiR-NbSu, 35S:BS-D17-amiR-NbSu, 35S:BS-D23-amiR-NbSu, 35S:BS-D31-amiR-NbSu, 35S:BS-D7-amiR-NbDXS, 35S:BS-D17-amiR-NbDXS, 35S:BS-D23-amiR-NbDXS, 35S:BS-D31-amiR-NbDXS.*

Constructs *35S:shc-amiR-NbSu, 35S:shc-amiR-NbDXS, 35S:shc-amiR-TSWV, 35S:shc-amiR-AtCH42, 35S:shc-amiR-AtFT* were obtained by ligating with T4 DNA ligase annealed oligonucleotide pairs AC-486/AC-487, AC-603/AC-604, AC-672/AC-673, AC-623/AC-624 and AC-621/AC-622, respectively into *pMDC32B-BS-AtMIR390a-B*/*c* previously digested with Bsal restriction enzyme..

The PVX vector consisted of a modified version of PVX-MT799816.1 (Uranga,M., et al., (2021) Efficient Cas9 multiplex editing using unspaced sgRNA arrays engineering in a Potato virus X vector. Plant J, 106, 555-565). The PVX vector was cloned into *pLX-B2* (Pasin,F., et al., (2017) Multiple T-DNA Delivery to Plants Using Novel Mini Binary Vectors with Compatible Replication Origins. ACS Synth. Biol., 6, 1962-1968.) flanked by the 35S CaMV promoter and terminator and a delta ribozyme (Schürer,H., et al., (2002) A universal method to produce in vitro transcripts with homogeneous 3' ends. Nucleic Acids Res, 30, e56.). For PVX-based amiRNA constructs, *pri-* and *shc*-based cassettes were amplified from *35S:pri-amiR-GUS_{Nb}*/*35S:pri-amiR-NbSu* or *35S:shc-amiR-NbSu* constructs with oligonucleotides AC-648/AC-662 and AC-650/AC-663, respectively, gel purified and assembled into *Mlu*I-digested and gel-purified PVX plasmid in the presence of GeneArt Gibson Assembly HiFi Master Mix (Invitrogen) to generate *35S:PVX-pri-amiR-GUS_{Nb}, 35S:PVX-pri-amiR-NbSu* and *35S:PVX-shc-amiR-NbSu..* All precursors were expressed from endogenous PVX CP promoter. An heterologous promoter derived from BaMV drives the expression of PVX CP, which contains the deletion of the 29 initial codons to enhance the stability of the recombinant clones (Dickmeis,C., et al., (2014) Potato virus X-based expression vectors are stabilized for long-term production of proteins and larger inserts. Biotechnol J, 9, 1369-1379.). 35S:PVXwas obtained by digesting the PVX plasmid with *Mlu*I*,* gel purifying and re-ligating the backbone band.

AmiRNA constructs *35S:pri-amiR-GUS_{Nb}* and *35S:pri-amiR-NbSu* were reported previously (Cisneros,A.E., et al., (2022) supra). All DNA oligonucleotides used for generating the constructs described above are listed in Table 1 above. The sequence of the *BS-AtMIR390a-based* B/c amiRNA vectors corresponds to SEQ ID NO. 168.

### Transient expression of constructs and virus infection assays

DNA constructs were agroinfiltrated in *N. benthamiana* leaves as described (Cuperus,J.T., et al., (2010) Unique functionality of 22-nt miRNAs in triggering RDR6-dependent siRNA biogenesis from target transcripts in Arabidopsis. Nat Struct Mol Biol, 17, 997-1003; Llave,C., et al., (2002) Cleavage of Scarecrow-like mRNA Targets Directed by a Class of Arabidopsis miRNA. Science, 297, 2053-2056), using A. *tumefaciens* GV3101 strain. Infection assays with TSWV LL-N.05 isolate were done as described (Carbonell,A., Lopez,C. et al., (2019) Fast-Forward Identification of Highly Effective Artificial Small RNAs Against Different Tomato spotted wilt virus Isolates. Mol Plant Microbe Interact, 32, 142-156;). Mechanical inoculation with crude extracts from PVX infected plants was done as described for TSWV. For spray inoculations, 5 ml of crude extract were obtained from each plant after grinding 1g of apical tissue to fine powder and homogenizing in inoculation buffer (50 mM potassium phosphate pH 8.0, 1% polyvinylpyrrolidone 10 and 1% polyethylene glycol 6000, 10 mM mercaptoethanol). All 5 ml were sprayed to two different leaves of a 3-weeks old *N. benthamiana* plant with a high-density polyethylene vaporizer.

### Chlorophyll extraction and analysis

Pigments from *N. benthamiana* infiltrated leaf tissues were extracted as described (Carbonell,A., et al., (2015) Highly specific gene silencing in a monocot species by artificial microRNAs derived from chimeric miRNA precursors. Plant J, 82, 1061-75.). Absorbance measurements and content in chlorophyll a were calculated as described (Lopez-DoIz,L., et al., supra).

### RNA preparation

Total RNA from *N. benthamiana* leaves or from *A*. *thaliana* seedlings was isolated as follows. Tissue was ground in liquid nitrogen to fine powder and homogenized in extraction buffer (1 M guanidinium thiocyanate, 1 M ammonium thiocyanate, 0.1 M sodium acetate, 5 % glycerol, 38 % water-saturated phenol). Next, RNA was extracted by chloroform extraction and precipitated after adding 0.5 volumes of isopropanol for 20 min. Triplicate samples from pools of two *N. benthamiana* leaves or 9-12 Arabidopsis seedlings were analyzed.

### Real-time RT-qPCR

cDNA was obtained from 500 ng of DNasel-treated total RNA from apical leaves at 14 dpa, using the PrimeScript RT Reagent Kit (Perfect Real Time) (Takara) according to the manufacturer's instructions.

Real time RT-qPCR was done as described in a QuantStudio 3 Real-Time PCR system (Thermo Fisher Scientific). *NbSuINbDXS* and *AtCH42* target RNA expression levels were calculated relative to *N*. *benthamiana PROTEIN PHOSPHATASE 2A (NbPP2A)* and Arabidopsis *ACTIN 2* (*AtACT2*) reference genes, respectively, using the delta delta cycle threshold comparative method of the QuantStudio Design and Analysis Software (version 1.5.1.; Thermo Fisher Scientific). Oligonucleotides used for RT-qPCR are listed in Table 1 above.

### Small RNA blot assays

Small RNA blot assays and band quantification from radioactive membranes were done as described (Cisneros,A.E., et al., (2022) supra. Oligonucleotides used as probes for sRNA blots are listed in Table 1 above.

### Small RNA sequencing and data analysis

Total RNA quantity, purity and integrity was analyzed with a 2100 Bioanalyzer (RNA 6000 Nano kit, Agilent) and submitted to BGI (Hong Kong, China) for sRNA library preparation and SE50 sequencing in a DNBSEQ-G-400 sequencer (BGI). After reception of quality-trimmed, adaptor removed clean reads from BGI, fastx_collapser was used to collapse identical reads into a single sequence, while maintaining read counts. A custom Python script was then used to map each clean, unique read against the forward strand of the amiRNA precursor overexpressed in each sample not allowing mismatches or gaps, and also to calculate the counts and RPMs (reads per million mapped reads) for each mapping position. Processing accuracy of amiRNA foldbacks was assessed by quantifying the proportion of 19-24 nt sRNA (+) reads that mapped within 4 nt of the 5' end of the amiRNA guide as reported before (Cuperus,J.T., et al., (2010) , supra; Carbonell,A, et al., (2015), supra).

### Stability and sequence analyses of amiRNA precursors during viral infections

Total RNA from apical leaves of each of the three biological replicates was pooled before cDNA synthesis, which was performed as explained above. PCR to detect amiRNA precursors, PVX and *NPP2A* was performed using oligonucleotide pairs AC-654/AC-655, AC-657/AC-658 and AC-365/AC-366 (Table 1), respectively, and Phusion DNA polymerase (ThermoFisher Scientific). PCR products were analyzed by agarose gel electrophoresis, and products of the expected sized were excised from the gel and sequenced.

### Preparation of crude extracts from virus infected plants

For each plant, 1g from 1-2 apical leaves was ground to fine powder, homogenized in 5 ml of buffer (50 mM potassium phosphate pH 8.0, 1% polyvinylpyrrolidone 10 and 1% polyethylene glycol 6000) and filtered with sterile Miracloth filter paper.

### Gene and virus identifiers

Arabidopsis and *N. benthamiana* gene identifiers are *AtACT2* (AT3G18780), *AtCH42* (AT4G18480), *AtFT* (AT1G65480), *NbSu* (Nbv5.1tr6204879), *NbDXS* (Nbv5.1tr6224823) and *NbPP2A* (Nbv5.1tr6224808). TSWV LL-N.05 segment L, M and S genome identifiers are KP008128, FM163373 and KP008129, respectively. PVX sequence variant MT799816.1 was cloned into a derivative of *pLX-B2* (KY825137.1).

### Protocol to generate TRV-based amiRNA constructs (she precursor)

TRV2-based constructs were obtained by Gibson cloning, by ligating annealed oligonucleotide pair AC-617/AC-618 (Table 1) into the *pLB-TRV-Z* plasmid previousely digested with *Bsa*I.

### Example 2 - Results

### A silencing sensor system in N. benthamiana for easily visualizing and quantifying amiRNA-induced silencing of endogenous genes

First, it was tested if a visible bleaching could be triggered with amiRNAs directed against the *1-DEOXY-D-XYLULOSE-5-PHOSPHATE SYNTHASE (NbDXS)* gene in the system described in Cisneros,A.E., et al., (2022). Briefly, three different constructs expressing amiRNAs targeting *NbDXS* at unique sites and with no predicted off-targets were independently agroinfiltrated in two areas of two leaves from three different *N*. *benthamiana* plants agroinfiltrated, together with a negative control construct expressing an amiRNA against *Escherichia coli* b-glucuronidase GUS. At 7 days post-agroinfiltratino (dpa), areas agroinfiltrated with anti-*NbDXS* amiRNA constructs displayed a visually obvious bleaching predicted from *NbDXS* knockdown while areas expressing the control construct did not. The chlorophyll analysis of agroinfiltrated areas showed a similar decrease in chlorophyll content in bleached areas expressing anti-NbDXS amiRNAs compared to areas expressing the control construct. Next, two leaves of three different plants were independently agroinfiltrated in the whole leaf surface with each of the amiRNA constructs described above. sRNA blot assays of RNA preparations from agroinfiltrated leaves showed that all anti-NbDXS amiRNAs accumulated as single-size sRNA species, with amiR-NbDXS-1 accumulating to significantly higher levels than the other two amiRNAs. RT-qPCR analysis revealed that all samples expressing anti-NbDXS amiRNAs accumulated significantly lower levels of *NbDXS* mRNA than control samples. These results indicate that anti-NbDXS amiRNAs are highly active and induce a strong bleaching phenotype in the tissue where they are expressed. Thus, the methodology described in (Cisneros,A.E., et al., (2022) supra) and outlined in Figure 1 was used in this work to test the effect on *NbSu* and *NbDXS* silencing efficacy of amiR-NbSu-1 (hereafter amiR-NbSu) and amiR-NbDXS-1 (hereafter amiR-NbDXS), respectively (Figure 2A), produced from *pri-AtMIR390a* precursors with reduced BS or DSL regions (see next four sections).

### Functional analysis of pri-AtMIR390a-based amiRNA precursors with shortened DSL regions

First, it was analyzed the silencing activity of amiR-NbSu- and amiR-NbDXS-expressing constructs including *pri-AtMIR390a*-based precursors with progressively shorter DSL regions ranging from 31 nt of the full-length *pri-AtMIR390a* (or simply *"pri"*) precursor to only 6 nt (Figure 2B). As expected, at 7 dpa areas expressing amiR-NbSu or amiR-NbDXS from the *pri* precursor displayed the reduced pigmentation characteristic of *NbSu* or *NbDXS* knock-down, while areas expressing amiR-GUS_{Nb} did not (Figure 2C). Interestingly, a similar bleaching was also observed in areas expressing amiR-NbSu or amiR-NbDXS from *AtDSL-D6* and *AtDSL-D13* precursors, while a less intense bleaching was noticed in areas expressing amiR-NbSu (but not amiR-NbDXS) from the *AtDSL-D21* precursor (Figure 2C). No bleaching was observed in areas expressing amiRNAs from the *AtDSL-D25* precursor (Figure 2C). Visual silencing phenotypes were confirmed by the chlorophyll analysis of agroinfiltrated areas, with bleached areas displaying lower chlorophyll a content than darker green areas (Figure 2D). RNA blot assays of RNA preparations from agroinfiltrated leaves showed that amiR-NbSu and amiR-NbDXS accumulated to high levels when expressed from *pri* or from shorter *AtDSL-D6* and *AtDSL-D13* precursors (Figure 2E). Both amiRNAs were also detected although to low levels when expressed from *AtDSL-D21* and also from *AtDSL-D25* in the case of amiR-NbDXS (Figure 2E). Finally, RT-qPCR analysis revealed that target mRNA expression was considerably reduced in samples expressing amiRNAs from *pri* or from shortened precursors compared to control samples, except for samples expressing amiR-NbSu or amiR-NbDXS from *AtDSL-D25* (Figure 2F). Notably, samples expressing amiRNAs from *pri* or from *AtDSL-D6* accumulated similar low target mRNA levels, while the expression of shorter *AtDSL-D13* and *AtDSL-D21* precursors generally induced a more moderate reduction in target mRNA levels (Figure 2F). In contrast, no significant reduction in target mRNA levels was observed when amiRNAs were expressed from *AtDSL-D25* (Figure 2F). These results indicate that the DSL region of *pri-AtMIR390a* can be shortened to some extent without significantly affecting amiRNA accumulation and target mRNA silencing.

### AmiRNAs produced from pri-AtMIR390a-based precursors including shortened OsM/R390-derived DSL regions trigger gene silencing with high efficacy

The OsMIR390 precursor has a DSL region (OsDSL) consisting of only 16 nt, which represents one of the shortest DSL regions from all conserved plant *MIRNA* precursors. Here, we reasoned that the OsDSL region might also tolerate nucleotide deletions while preserving efficient amiRNA production and target mRNA silencing. To test this, several constructs were generated for expressing amiR-NbSu and amiR-NbDXS from chimeric constructs including the complete (16-nt long) *OsDSL* region or progressively shortened versions of it (Figure 3A). In addition, a construct in which the 8-nt terminal loop of *OsMIR390* was replaced by the shorter 4-nt long terminal loop of *AtMIR390a* was also included in the analysis (Figure 3A). Areas agroinfiltrated with constructs expressing amiRNAs from *pri* or from chimeric *OsDSL* and *OsDSL-D2* precursors showed intense bleaching and similar reduction in the accumulation of chlorophyll a compared to controls (Figure 3B and 2C). In contrast, other agroinfiltrated areas displayed weaker bleaching and lower chlorophyll a content except those from controls and from areas expressing amiR-NbSu from *OsDSL-D6* which did not display any sign of silencing (Figure 3B and 3C). RNA blot analysis of RNA preparations from agroinfiltrated leaves revealed that amiRNAs accumulated to high levels in samples expressing the *pri* or chimeric OsDSL and *OsDSL-D2* precursors, while amiRNA levels dropped significantly in the rest of samples except for those expressing the *35S:OsDSL-D6-amiR-NbSu* construct in which amiR-NbSu was not detected (Figure 3D). RT-qPCR analysis revealed that target mRNA accumulation significantly decreased in all samples expressing amiRNAs from *pri* or from chimeric precursors compared to controls, except in samples expressing amiR-NbSu from *OsDSL-D4* and *OsDSL-D6* (Figure 3E). Importantly, target mRNA levels were similar in samples expressing amiRNAs from *pri* or from chimeric OsDSL and *OsDSL-D2* precursors (Figure 3E). Taken together, these results indicate that the OsDSL region can also be shortened -in this case down to 14 nt- in *AtMIR390a*-based precursors without compromising silencing efficacy.

### Functional analysis of AtMIR390a-based amiRNA precursors with shortened BS

Next, it was analyzed if shortening the BS region of *pri-AtMIR390a* could affect amiRNA production and silencing efficacy. Several constructs for expressing amiR-NbSu and amiR-NbDXS were generated, in which the 448-nt BS region of *pri-AtMIR390a* was progressively shortened down to 2 nt (Figure 4A). Similar intense bleaching and reduced chlorophyll a level was observed in areas expressing amiR-NbSu or amiR-NbDXS from the *pri* or *BS* precursors (Figure 4B and 3C). In contrast, weaker bleaching was observed in areas expressing any of the two amiRNAs from *BS-D7,* or amiR-NbSu from *BS-D17, BS-D23* or *BS-31* (Figure 4B), corresponding with slight decreases in chlorophyll a content compared to controls (Figure 4C). No bleaching or changes in chlorophyll a content were observed in leaves expressing amiR-NbDXS from *BS-D17, BS-D23* or *BS-31* (Figure 4B and 4C). Both amiR-NbSu and amiR-NbDXS amiRNAs expressed from the *pri* or BS precursors accumulated to similarly high levels and induced comparable target mRNA silencing (Figure 4D and 4E). In contrast, their levels dropped or were undetectable when expressed from *BS-D7*/*BS-D17* or BS-*D23*/*BS-D31,* respectively (Figure 4D) and induced lower target mRNA downregulation (Figure 4E). All together these results indicate that a complete BS region is enough for accurate precursor processing to produce high levels of amiRNAs, while further shortening this BS region reduces amiRNA accumulation.

### New high-throughput vectors for expressing amiRNAs from AtMIR390a-based precursors only including the BS

Previous high-throughput *AtMIR390a*-based amiRNA "B/c" vectors included the complete 521 bp sequence of the *pri-AtMIR390a* precursor (Carbonell,A., et al., (2014), supra). Here, two additional "B/c" vectors were developed for the efficient cloning and expression of amiRNAs from shortened precursors only including *AtMIR390a* BS (Figure 12). *pENTR-BS-AfMIR390a-B*/*c* Gateway-compatible entry vector was generated for direct cloning of amiRNA inserts and subsequent recombination into the preferred Gateway expression vector including the regulatory features of choice. *pMDC328-BS-AfMIR390a-B*/*c* vector was generated for the direct cloning of amiRNA inserts into a binary expression vector. Both vectors contain a truncated *AtMIR390a* BS sequence including a 1461-bp DNA cassette with the control of cell death (*ccd*B) gene flanked by two inverted *Bsa*I sites downstream the BS region. The generation of an amiRNA construct with these two new B/c vectors is similar to that described for previous AtMIR390a-BIe-based vectors (see Materials and Methods section, Carbonell,A., et al., (2014), supra,).

### Transient expression of amiRNAs from shortened, MIR390-based chimeric precursors triggers effective silencing of Nicotiana benthamiana endogenous genes and of pathogenic viral RNAs

The new "B/c" vectors were used to systemically test the effect of combining both regions (BS and OsMIR390 D2) in a single precursor. The resulting shortened chimeric *MIR390*-based precursor of only 89 nt was named *shc-MIR390* precursor (or simply "*shc*") (Figure 5A). First, AtMIRthe sequence corresponding to amiR-NbSu or amiR-NbDXS including the *OsMIR390-D2* region was introduced into *pMDC32B-BS-AtMIR390a-B*/*c* to generate the *35S:shc-amiR-NbSu* or *35S:shc-amiR-NbDXS* constructs, respectively. Both constructs were independently agroinfiltrated into *N. benthamiana* leaves together with control constructs, and induced similar strong bleaching, amiRNA accumulation and target mRNA downregulation in agroinfiltrated areas than their respective controls expressing these same amiRNAs from the full-length *pri* precursor (Figure 5B-D). To confirm the correct processing of *shc* precursors and compare it to that of the *pri,* sRNA libraries from leaves expressing *35S:pri-amiR-NbSu, 35S:pri-amiR-NbDXS, 35S:shc-amiR-NbSu* and *35S:shc-amiR-NbDXS* were prepared and sequenced. Interestingly, in samples expressing *35S:shc-amiR-NbSu* or *35S:shc-amiR-NbDXS,* the majority (95% and 90% respectively) of 19-24-nt (+) reads corresponded to authentic 21-nt amiR-NbSu or amiR-NbDXS (Figure 5E). These results indicate a high accuracy in the processing of shc precursors which was similar to that observed for the *pri* precursors (Figure 5E).

Next, it was tested if amiRNAs produced from the shc precursor could also be applied to silence exogenous transcripts, such as viral RNAs. Here, the sequence corresponding to the most efficient anti-tomato spotted wilt virus (TSWV) amiRNA (amiR-TSWV) was expressed from the shc precursor in a *N. benthamiana* leaf inoculated two days later with TSWV(Figure 10 A). At 7 days post-inoculation (dpi), all leaves inoculated with TSWV and expressing amiR-TSWV either from the *pri* or shc precursors did not display virus-induced symptoms, while leaves inoculated with TSWV and expressing the control amiR-GUS_{Nb} displayed multiple local lesions typical of TSWV infection (Fig. 10B). AmiRNA accumulation analyzed by sRNA northern blot showed that amiR-TSWV accumulated to similar levels in tissues expressing *35S:shc-amiR-TSWV* or *35S:pri-amiR-TSWV*(Fig. 10C). At 20 dpi, TSWV RNA was not detected in upper non-agroinfiltrated leaves from plants inoculated with TSWV and expressing *35S:shc-amiR-TSWV* or *35S:pri-amiR-TSWV,* while plants expressing *35S:pri-amiR-GUS_{Nb}* and inoculated with TSWV accumulated high levels of TSWV RNA (Fig. 10C). Thus, these results indicate that the shc precursor can be used to express high levels of amiRNAs to induce antiviral resistance in plants.

### Stable expression of amiRNAs from shc-MIR390 precursors leads to effective silencing of Arabidopsis thaliana endogenous genes

Next, the functionality of shc precursors in a different plant species and when stably expressed in transgenic plants was tested. For that purpose it was used the amiR-AtFT/*AtFT* and amiR-AtCH42/*AtCH42* silencing sensor systems in Arabidopsis in which the transgenic expression of amiR-AtFT or amiR-AtCH42 from *pri-AtMIR390a* leads to a significant delay in flowering time or to an intense bleaching due to the targeting of endogenous *FLOWERING LOCUS T* (*AtFT*) or *CHLORINA 42 (AtCH42)* endogenous genes, respectively (Carbonell,A., et al., (2021), supra). Here, amiR-AtFT and amiR-CH42 were introduced into *pMDC32B-BS-AtMIR390a-Blc* to generate the *35S:shc-amiR-AtFT* and *35S:shc-amiR-AtCH42* constructs, respectively (Figure 6A). These constructs were transformed independently into Arabidopsis Col-0 plants together with control constructs *35S:pri-amiR-GUS_{Ath}, 35S:pri-amiR-AtFT* and *35S:pri-amiR-AtCH42* expressing an amiRNA against GUS (with no predicted off-targets in *A*. *thaliana), AtFT* and *AtCH42*, respectively, from *pri-AfMIR390a.* To systemically compare the processing and induced silencing of amiR-AtFT or amiR-AtCH42 produced from shc and *pri* precursors, plant phenotypes, amiRNA and target mRNA accumulation and processing efficiency were measured in Arabidopsis T1 transgenic lines.

All *35S:pri-amiR-AfFT* (n=40) and *35S:shc-amiR-AtFT* (n=34) transformants flowered later than the average flowering time of the *35S:pri-amiR-GUS_{Ath}* control lines (n=48) (Figure 6B,), and their average flowering time (54.7 ± 6 and 54.4 ± 3.2, respectively) was not significantly different between each other (Figure 6C). RNA-blot and RT-qPCR assays revealed that both amiR-AtFT and *AtFT* mRNA accumulation was similar in lines expressing amiR-AtFT from each of the two precursors (Figure 6D), and high-throughput sequencing of sRNAs showed that both precursors were efficiently processed, as in both cases 99% of the reads corresponded to authentic amiR-AtFT (Figure 6E). Similarly, all *35S:pri-amiR-AtCH42* (n=54) and *35S:shc-amiR-AtCH42* (n=38) transformants displayed bleaching of comparable degrees (Figure 6B and 6C), accumulated similar levels of amiR-CH42 and AtCH42 mRNA (Figure 6D), and displayed effective precursor processing (Figure 6E). Taken together these results indicate that the *shc* precursor is functionally equivalent to the *pri* precursor in inducing highly effective silencing of endogenous genes when stably expressed in Arabidopsis.

### Unique functionality of the shc precursor in triggering widespread gene silencing in N. benthamiana when expressed from a viral vector

Because cargo capacity of viral vectors is limited, and large inserts are typically eliminated during viral replication in VIGS vectors (Rössner,C., et al., (2022) VIGS Goes Viral: How VIGS Transforms Our Understanding of Plant Science. Annu Rev Plant Biol, 73, 703-728), it was hypothesized that the short (89-nt long) shc precursor fragment could be a more stable insert when included into a viral vector than the longer 521-nt *pri* precursor. To test this, *pri-amiR-NbSu* and *shc-amiR-NbSu* sequences were inserted into a potato virus X (PVX) infectious clone to generate the *35S:PVX-pri-amiR-NbSu* and *35S:PVX-shc-amiR-NbSu* constructs, respectively (Figure 7A). These constructs were expected to express amiR-NbSu from *pri* and *shc* precursors, respectively, during PVX infection and induce the photo-bleaching of PVX-infected tissues. In addition, the *35S:PVX-pri-amiR-GUS_{Nb}* negative control construct was also generated. These three constructs together with the insert-free *35S:PVX* construct (Figure 7A) were independently agroinoculated in one leaf of three *N. benthamiana* plants. A "mock" set of plants was agroinfiltrated with the agroinfiltration solution.

Mild leaf curling typical of PVX-induced symptoms was observed in upper non-agroinoculated leaves between 4 and 5 dpa in all plants agroinoculated with the *35S:PVX* and *35S:PVX-shc-amiR-NbSu* constructs (Figure 7B). However, curling in apical leaves from plants agroinoculated with the *35S:PVX-pri-amiR-GUS_{Nb}* and *35S:PVX-pri-amiR-NbSu* constructs appeared 3-4 days later, between 7 and 9 dpa (Figure 7B). Interestingly, bleaching of apical leaves appeared at 8-9 dpa only in plants agroinoculated with the *35S:PVX-shc-amiR-*NbSu construct, and at 14 dpa extended to most of the apical tissues of infected plants (Figure 7C). Additional analyses confirmed the decrease in chlorophyll a level in bleached tissues (Figure 7D). Plants were monitored until 28 dpa, and only those expressing the *35S:PVX-shc-amiR-NbSu* construct displayed the characteristic yellowing derived from NbSu silencing. RNA-blot and RT-qPCR analyses showed that only tissues from plants expressing *35S:PVX-shc-amiR-NbSu* accumulated high levels of amiR-NbSu (Figure 7E) and low levels of *NbSu* mRNA (Figure 7F). Interestingly, sRNA sequencing from apical leaves of plants agroinoculated with *35S:PVX-shc-amiR-NbSu* revealed that the shc-amiR-NbSu precursor inserted into PVX was accurately processed (Figure 7G). Additional analyses showed that the percentages of sRNAs of (+) or (-) polarity derived from the PVX sequence are similar (48 % and 52 %, respectively), suggesting that these sRNAs originate from double-stranded RNA (dsRNA) intermediates produced by RNA-dependent RNA polymerases during PVX replication . In contrast, the majority (89 %) of sRNAs derived from the shc-amiR-NbSu sequence included in PVX are of (+) polarity, similarly to when the same precursor is expressed without PVX. These results most likely indicate that amiR-NbSu originates from the shc precursor rather than from dsRNA intermediates of replication.

Finally, the presence of both *pri* and shc precursors in apical leaves was analyzed by RT-PCR at 7 and 14 dpa with oligonucleotides flanking the precursor insertion site. At 7 dpa, the shc precursor was clearly amplified in plants expressing *35S:PVX-shc-amiR-NbSu,* while only the *pri* precursor of the *35S:PVX-pri-amiR-NbSu* samples was barely detected (Figure 7H). Indeed, at 14 dpa only the shc precursor was detected (Figure 7H). Importantly, PVX was detected in all plants expressing PVX-based constructs at the two timepoints analyzed (Figure 7H) thus excluding the possibility that the absence of *pri* precursors was due to a lack of infection in these samples. Sequencing analysis of RT-PCR products from each of the three *PVX-shc-amiR-NbSu* infected samples showed that no mutations accumulated in the *shc-amiR-NbSu* insert. These results indicate that the *pri* fragments are eliminated from the PVX vector during viral infection, while the shorter shc fragments are stable both structurally and at a sequence level.

### A PVX-based, DNA-free system for inducing widespread silencing in plants with amiRNAs

Next, it was explored the possibility of triggering widespread silencing in a DNA-free, non-transgenic manner, by using alternative methods to agroinfiltration for inoculating shc-containing PVX sequences. For this purpose, a crude extract from bleached apical leaves of each of the three individual plants agroinfiltrated with *35S:PVX-shc-amiR-NbSu* was prepared. Each crude extract was used as inoculum to mechanically inoculate one young *N. benthamiana* plant (Figure 8A). As controls, similar crude extracts were prepared from mock and empty PVX-agroinoculated plants and analyzed in parallel. Interestingly, all three plants mechanically inoculated with extracts derived from *35S:PVX-shc-amiR-NbSu* agroinfiltrated plants displayed prominent bleaching and accumulated authentic PVX-derived *shc-amiR-NbSu* precursors (Figure 8B) with no sequence alterations as confirmed by sequencing of RT-PCR products. Plants inoculated with extracts from *35S:PVX-*agroinfiltrated plants displayed mild PVX characteristic symptoms and accumulate PVX RNAs, while plants inoculated with extracts derived from mock-inoculated plants remained symptomless and virus-free (Figure 8B).

Finally, it was tested if widespread silencing could also be triggered by applying PVX-shc-amiR-NbSu-containing raw extracts through spraying (Figure 8A). Remarkably, all three plants sprayed with crude extracts derived from *35S:PVX-shc-amiR-NbSu* agroinoculated plants displayed bleached apical leaves which accumulated authentic *shc-amiR-NbSu* precursors with no sequence alterations as confirmed by sequencing of the RT-PCR products (Figure 8C). Importantly, plants sprayed with crude extracts from *35S:PVX-*agroinoculated plants or mock-inoculated plants displayed only mild PVX-derived symptoms or no symptoms at all, respectively, and no bleaching (Figure 8C). These results indicate that crude extracts can be directly sprayed to leaves to trigger amiR-VIGS in *N. benthamiana* in a non-transgenic, DNA-free manner.

### Highly efficient amiR-VIGS in Nicotiana benthamiana using an RNA virus

To further expand the amiR-VIGS toolbox, it was developed an amiR-VIGS system based on the positive, single-stranded RNA virus *Tobacco rattle virus (TRV).* TRV is one of the preferred viruses for VIGS as it has a wide host range and induces very mild symptoms. Its genome comprises 2 components: RNA1 and RNA2. The *JoinTRV* vectors(Aragonés V. et al., (2022), Simplifying plant gene silencing and genome editing logistics by a one-Agrobacterium system for simultaneous delivery of multipartite virus vectors. Biotechnology Journal, 17:2100504) were used to express from RNA2 (TRV2) an amiRNA against the *SULPHUR (Su)* gene, coding for the magnesium chelatase subunit CHLI whose silencing leads to tissue photo-bleaching. TRV-based constructs were agroinfiltrated in *N. benthamiana* leaves and induced barely detectable TRV symptoms as early as 4 days post-agroinfiltration (dpa). At 10 dpa, upper (non-agroinfiltrated) leaves from plants agroinfiltrated with *TRV1* and *TRV2-amiR-Su* constructs displayed bleached areas characteristic of *Su* silencing, while similar leaves from control plants agroinfiltrated with *TRV1* and *TRV2-amiR-GUS* constructs remained green (Fig. 9). Indeed, yellowing of upper tissues was more intense than that induced by amiR-Su expressed from *CalCuV* (data not shown).

### CONCLUSIONS

Plant monocistronic miRNA precursors commonly used to express amiRNAs in plants vary in structure and length, with an average length significantly higher than that of their corresponding miRNA foldback (Figure 11), as they generally correspond to full-length pri-miRNA precursors. The shc-MIR390 precursor has the minimal length of all amiRNA precursors tested in plants so far, being considerably shorter than its parental pri-AtMIR390a precursor. Minimal amiRNA precursors may have several advantages. First, they should be more stable and retained in the viral genome for longer periods. This advantage may be of particular interest in amiR-VIGS experiments involving viruses with limited cargo capacity. Second, using shorter amiRNA precursor sequences should decrease the cost of producing amiRNA constructs, which is particularly convenient in high-throughput applications where large amounts of amiRNA constructs are generated for plant transformation. Here, shc precursors are inserted into BS-AtMIR390a-B/c-based vectors after annealing two oligos of 58 bases, while cloning of amiRNA inserts in previous AtMIR390a-B/c-based vectors required the annealing of 75 bases-long oligonucleotides. Importantly, the reduction of the oligonucleotide length from 75 to 58 bases should allow for a more cost-effective oligo synthesis and at the lowest production scale (25 nmole) offered by the main oligo-manufacturing companies. And third, minimal amiRNA precursors may be more efficiently synthesized in vitro or in bacteria for topical application to plants, among other possible advantages. In any case, the use of minimal amiRNA precursors in a non-transgenic, DNA-free manner should definitely accelerate the development of next-generation crops in line with international regulations affecting genetically modified plants.

## Claims

1. A viral vector comprising a heterologous nucleotide sequence that encodes, or corresponds to the RNA sequence S1 that is of a length of 56-350 ribonucleotides and that comprises regions R1, R2, R3, R4 and R5 directly linked to each other as indicated in formula (i):
(I) 5'-R1-R2-R3-R4-R5-3',
wherein:
- R1 consists of the RNA sequence of a strand of a basal stem (BS) structure,
- R2 consists of the RNA sequence of a miRNA,
- R3 comprises an RNA sequence that has a length of at least 8 nucleotides,
- R4 consists of an RNA sequence that base pairs with the RNA sequence of R2 to form a miRNA/miRNA* duplex,
- R5 consists of the RNA sequence complementary to R1 in the BS comprising R1,
and wherein the RNA sequence S1 forms a stem-loop structure where the stem comprises regions R1, R2, R4 and R5 wherein region R1 base pairs with region R5 and region R2 base pairs with region R4, and the loop comprises a sequence of at least 4 contiguous nucleotides of region R3.

2. The viral vector according to claim 1, wherein the sum of the lengths of all the heterologous sequences comprised in the viral vector is equal to less than 400 nucleotides.

3. The viral vector according to any one of claims 1-2 wherein:
- Region R1 comprises or consists of a sequence selected from the list consisting of SEQ ID NO. 1 (UCUGUA), SEQ ID NO. 18 (GAAUCUGUA), SEQ ID No. 19 (GAGAAGAAUCUGUA), SEQ ID NO. 20 (AGUAGAGAAGAAUCUGUA), or a variant thereof at least 80% identical to SEQ ID NO.1, SEQ ID NO. 18, SEQ ID NO. 19, or SEQ ID NO. 20, particularly comprises or consists of SEQ ID NO. 20 (AGUAGAGAAGAAUCUGUA), or a variant thereof at least 80% identical to SEQ ID NO. 20 and/or
- Region R3 comprises a sequence selected from the list consisting of SEQ ID NO. 3 (AAAUCAAA), SEQ ID NO. 4 (GAAAUCAAA), SEQ ID NO. 5 (CGAAAUCAAACU), SEQ ID NO. 6 (UCGAAAUCAAACUA), SEQ ID NO. 7 (AUUC), SEQ ID NO. 8 (CAUUCG), SEQ ID NO. 9 (ACAUUCGU), SEQ ID NO. 10 (UCACAUUCGUUA), SEQ ID NO. 11 (AUCACAUUCGUUAU), SEQ ID NO. 12 (GAUCACAUUCGUUAUC), SEQ ID NO. 13 (UGAUCACAUUCGUUAUCUA), SEQ ID NO. 14 (AUGAUCACAUUCGUUAUCUAU), SEQ ID NO. 15 (GAUGAUCACAUUCGUUAUCUAUU), SEQ ID NO. 16 (AUGAUGAUCACAUUCGUUAUCUAUUUUUU), SEQ ID NO. 17 (UCGAUUCCUA), particularly wherein region R3 comprises SEQ ID NO. 5 (CGAAAUCAAACU), and/or
- Region R5 comprises or consists of SEQ ID NO. 2 (UUGG), SEQ ID NO. 21 (UUGGCUC), SEQ ID NO. 22 (UUGGCUCUUCUU), SEQ ID NO. 23 (UUGGCUCUUCUUACU), or a variant thereof at least 80% identical to SEQ ID NO. 2, SEQ ID NO. 21, SEQ ID NO. 22, or SEQ ID NO. 23, particularly comprises or consists of SEQ ID NO. 23 (UUGGCUCUUCUUACU) or a variant thereof at least 80% identical to SEQ ID NO. 23.

4. The viral vector according to any one of claims 1-3 wherein region R3 further comprises at the 3'end, two nucleotides X₁ and X₂ that form a sequence 5'-X₁X₂-3' that is the reverse complement of the sequence 5'-Y₁Y₂-3' formed by the last two nucleotides Y₁ and Y₂ at the 3' end of region R2 sequence.

5. The viral vector according to any one of claims 1-4 wherein:
- Region R1 comprises or consists of SEQ ID NO. 20 (AGUAGAGAAGAAUCUGUA), or a variant thereof at least 80% identical to SEQ ID NO. 20,
- Region R3 comprises or consists of SEQ ID NO. 24 (CGAAAUCAAACUX₁X₂) wherein nucleotides X1 and X₂ at the 3'end of SEQ ID NO. 24 form the sequence 5'-X₁X₂-3' that is the reverse complement of the sequence 5'-Y₁Y₂-3' formed by the last two nucleotides Y₁ and Y₂ at the 3' end of region R2 sequence,
- Region R5 comprises or consists of SEQ ID NO. 23 (UUGGCUCUUCUUACU), or a variant thereof at least 80% identical to SEQ ID NO. 23.

6. The viral vector according to any one of claims 1-5 wherein the RNA sequence S1 is operatively linked to an expression promoter.

7. The viral vector according to any one of claims 1-6 wherein the miRNA targets a gene in the genome of a plant pathogen or a gene in the genome of a plant, particularly wherein the gene in the genome of the plant pathogen is a gene encoding a protein selected from the list consisting of the viral coat protein (CP), the viral movement protein (MP), RNA polymerases, the viral RNA-dependent RNA polymerase (RdRp), and the viral suppressors of RNA silencing (VSRs) and/or the gene in the genome of the plant is selected from the list consisting of SFT (single flower truss), TFL1 (terminal flower 1), DET1 (de-etiolated 1), ALS (acetolactate synthase), ACCase (acetyl-CoA carboxylase) and EPSPS (5-enolpyruvylshikimate-3-phosphate).

8. The viral vector according to claim 7 wherein the plant pathogen is selected from the group consisting of viruses, viroids, bacteria, fungi, oomycetes, insects, and nematodes; wherein the viruses are particularly selected from the group consisting of Tomato Spotted wilt virus (TSWV), Tomato brown rugose fruit virus (ToBRFV), Tomato leaf curl new delhi virus (ToLCNDV), and the viroids consist of the Potato spindle tuber viroid (PSTVd).

9. A virus encoded by or comprising the viral vector according to any one of claims 1-8.

10. An RNA polynucleotide comprising the RNA sequence S1 as defined in any one of claims 1-8 provided that the RNA polynucleotide does not comprise:
(i) in the region upstream of region R1, in a 5'- to 3'-end direction, sequence SEQ ID NO. 25, and/or
(ii) in the region downstream of region R5, in a 5'- to 3'-end direction, sequence SEQ ID NO. 26.

11. A plasmid comprising a sequence that encodes or corresponds to the sequence of the viral vector as defined in any one of claims 1-8, or the sequence coding the polynucleotide as defined in claim 10, particularly wherein the sequence that encodes or corresponds to the sequence of the viral vector, or the sequence encoding the polynucleotide, is operatively linked to an expression promoter.

12. A bacterium that comprises the plasmid as defined in claim 11, particularly wherein the bacterium is from the species *Agrobacterium thumefaciens.*

13. A plant that comprises the viral vector as defined in any one of claims 1-8, the virus as defined in claim 9, the polynucleotide as defined in claim 10, the plasmid as defined in claim 11 and/or the bacterium a as defined in claim 12, particularly wherein the plant is a non-transgenic plant.

14. A plant extract from the plant as defined in claim 13, particularly wherein the plant extract comprises the virus as defined in claim 9.

15. A method for obtaining the plant as defined in claim 13, the method comprising the step of:
(i) transfecting a plant with the plasmid as defined in claim 11, particularly by agroinfiltration with the bacterium as defined in claim 12, or alternatively,
(ii) contacting the surface of at least one organ of a plant with the virus as defined in claim 9, particularly
wherein contacting the surface of at least one organ of the plant with the virus comprises spraying the surface of said organ with the plant extract as defined in claim 14.
